# EUROPEAN PATENT APPLICATION

(11) **EP 4 389 122 A1**
(43) Date of publication of application: **26.06.2024**
(21) Application number: 22215382.7
(22) Date of filing: 21.12.2022
(51) Int. Cl.: A61K 31/37, A61K 38/22, A61P 1/00, A61P 35/00

(54) **ADIPONECTIN ALONE OR IN COMBINATION WITH EXTRACORPOREAL PHOTOPHERESIS (ECP) FOR IMMUNE RELATED ADVERSE EVENTS OF IMMUNE CHECKPOINT INHIBITORS**

(71) Applicant: Mallinckrodt Pharmaceuticals Ireland Limited, Dublin 15 (IE)
(72) Inventor: ZEISER, Robert, 79100 Freiburg (DE)
(74) Representative: Carpmaels & Ransford LLP

(57) **Abstract**

The invention relates to a substance adiponectin and/or adiponectin receptor agonist (ARA) for use in the treatment of immune checkpoint blockade (ICB)-induced immune related adverse events (irAEs) in a subject, wherein the subject is preferably receiving checkpoint blockade therapy as a cancer therapy. As well as to a combination medication comprising adiponectin and/or adiponectin receptor agonist (ARA) and a photosensitizing agent for combined use in the treatment of immune checkpoint blockade (ICB)-induced immune related adverse events (irAEs) in a subject, wherein the treatment further comprises the extracorporeal irradiation of a blood sample of the subject with ultraviolet A (UVA), preferably extracorporeal photopheresis (ECP) therapy.

## Description

The invention relates to a substance adiponectin and/or adiponectin receptor agonist (ARA) for use in the treatment of immune checkpoint blockade (ICB)-induced immune related adverse events (irAEs) in a subject, wherein the subject is preferably receiving checkpoint blockade therapy as a cancer therapy. The invention further relates to a combination medication comprising adiponectin and/or adiponectin receptor agonist (ARA) and a photosensitizing agent for combined use in the treatment of immune checkpoint blockade (ICB)-induced immune related adverse events (irAEs) in a subject, wherein the treatment further comprises the extracorporeal irradiation of a blood sample of the subject with ultraviolet A (UVA), preferably extracorporeal photopheresis (ECP) therapy.

### BACKGROUND OF THE INVENTION

Immune checkpoint blockade (ICB) has proven clinical benefit in the treatment of multiple cancers. Immune checkpoint blockade increases antitumor immunity by blocking negative regulators of immunity, such as cytotoxic T-lymphocyte antigen 4 (CTLA-4), programmed cell death-1 (PD-1) or programmed cell death ligand-1 (PD-L1). Immune-mediated tissue damage of an organ resulting from ICB-therapy is classified as immune-related adverse event (irAE). irAEs can affect any organ system and most commonly involves the gastrointestinal tract, lungs, endocrine glands, skin, and liver (Weber et al., 2017).

Treatment of irAEs includes cessation of ICB-therapy and/or administration of glucocorticosteroids, adapted to the severity of the irAEs (Haanen et al., 2017). These measures incur the risk that lack of ICB-therapy and immunosuppression reduce anti-tumor immune responses. So far, no randomized trial has analyzed the impact of glucocorticosteroids on the anti-tumor immune effect in ICB treated patients and retrospective data are controversial (Luo et al., 2017; Horvat et al., 2015; Marschner et al., 2020).

Additionally, glucocorticosteroids cause major side-effects including hyperglycemia, fluid retention, psychological disorders, iatrogenic adrenal insufficiency if the glucocorticoids are tapered too fast and infectious complication (Postow et al., 2018).

Also, second line therapies such as TNF-antagonists may interfere with anti-tumor effects as TNF was shown to be required for anti-tumor effects in hematological malignancies (Schmaltz et al., 2003) and TNF-blockade for autoimmunity was connected to a numerically increased risk for lymphoproliferative cancers (Askling et al., 2009; Setoguchi et al., 2006) and non-melanoma skin cancers (Chakravarty e al., 2005; Wolfe et al., 2007). A retrospective study that analyzed 790 patients with advanced melanoma being treated with ICB, reported a rate of serious infections of 13.5% in the subgroup of patients who received either glucocorticoids or infliximab (TNF antagonist; Del Castillo et al., 2016). A recent clinical study showed that TNF-blockade along with immunotherapy was connected to a non-response to ICB in 50% of the patients (Montfort at al., 2020).

In summary, patients developing severe irAEs (grade 3 and 4) after ICB are currently treated with a complete and durable interruption of the immunotherapy and the administration of glucocorticosteroids (Haanen et al., 2017). Both interventions may have negative effects on the anti-tumor immune response. Currently no prospective randomized trial supports the use of glucocorticosteroids or second line therapies such as the frequently used approaches with mycophenolate mofetile (MMF), TNF antagonists or cyclosporine A. Novel approaches that are active against irAE are lacking.

Therefore, novel therapeutic approaches that interfere with irAEs without diminishing anti-tumor effects and which induce little systemic side effects are an unmet medical need.

### SUMMARY OF THE INVENTION

In light of the prior art the technical problem underlying the present invention is to provide improved therapeutic strategies for the treatment of irAEs, especially during ICB therapy, which do not interfere with ICB mediated anti-tumor effects and cause less side effects than state of the art glucocorticoid treatment.

This problem is solved by the features of the independent claims. Preferred embodiments of the present invention are provided by the dependent claims.

The inventors surprisingly found that both, the administration of adiponectin alone or the combined administration of adiponectin and/or adiponectin receptor agonist (ARA), and a photosensitizing agent in the context of extracorporeal photopheresis (ECP) therapy could successfully be used in the treatment of immune checkpoint blockade (ICB)-induced immune related adverse events (irAEs) in a subject.

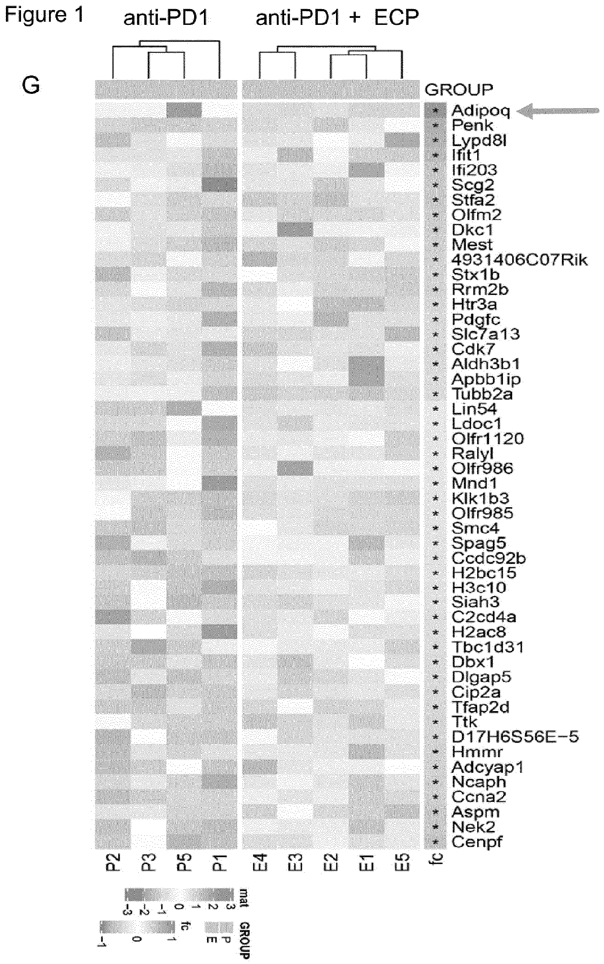

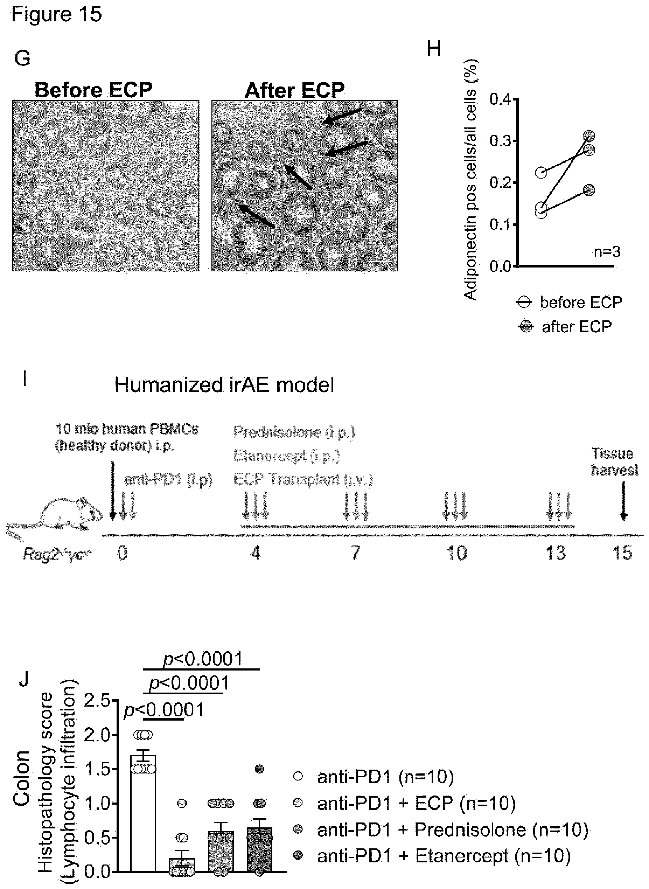

The inventors have successfully applied extracorporeal photopheresis (ECP) in the treatment of ICB-induced colitis in a patient with metastatic melanoma before (Apostolova et al., 2020). Presently, when analyzing the mechanisms by which ECP reduced colitis after ICB, the inventors found that ECP surprisingly caused adiponectin expression in the intestinal tract (Fig. 1G-K) leading to arginase-1 (Arg-1) release by myeloid cells as well as reduced T cell activation and expansion of regulatory KLRG1⁺BTLA⁺CD47⁺TOX^{low} T cells. Adiponectin production was induced by apoptotic cells that were phagocytosed by macrophages in the intestinal tract. The inventors found that ECP reduced clinical and histological signs of ICB-induced colitis in mice as well as *in vivo* expansion of pathogenic luciferase-transgenic T cells infiltrating irAE target organs. Surprisingly the anti-tumor immunity in melanoma-bearing mice treated with anti-PD1 remained intact during ECP-treatment, while corticosteroids reduced anti-melanoma immune effects. Consistent with the findings in mice ECP induced adiponectin in blood and intestinal tissues of irAE patients (Fig. 15G, H).

The inventors identified ECP as a novel immunomodulatory therapy that controls ICB-induced irAE, such as colitis, by activating the adiponectin/Arg-1 axis and by direct effects on T cells and macrophages. Using genetic loss-of-function approaches (Adiponectin KO, Arginase-1 KO mice) and pharmacological interventions the inventors could show a functional role of the adiponectin/Arg-1 axis for the protective effects of ECP.

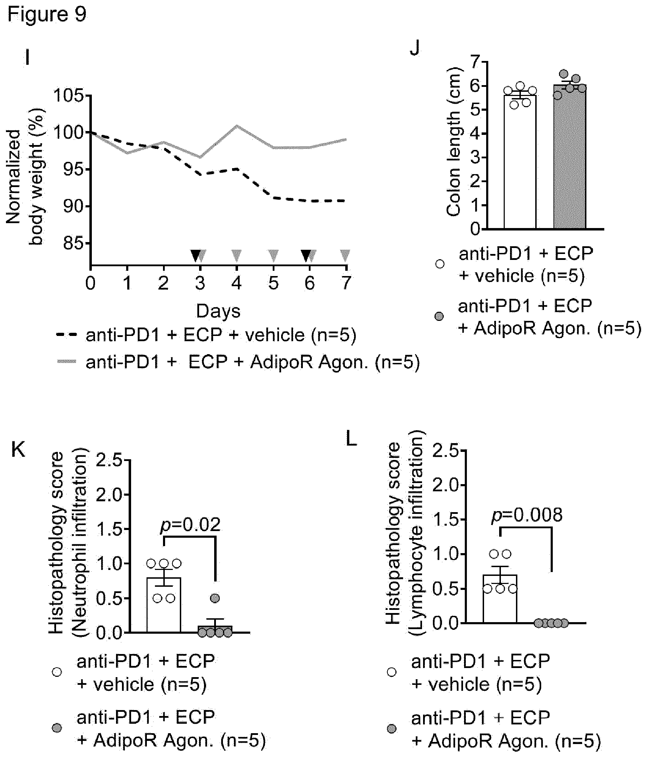

The inventors surprisingly found that adiponectin and/or Adiponectin Receptor Agonist (ARA) administration enhances the activity of ECP in the treatment of ICB-induced irAE, which could be observed, e.g., as reduced intestinal inflammation and less weight loss (see Fig. 9L-P) during/after ICB therapy.

The invention therefore relates in a first aspect to a combination medication comprising a substance adiponectin and/or adiponectin receptor agonist (ARA) and a photosensitizing agent for combined use in the treatment of immune checkpoint blockade (ICB)-induced immune related adverse events (irAEs) in a subject, wherein the treatment (further) comprises the extracorporeal irradiation of a blood sample of the subject with ultraviolet A (UVA), preferably extracorporeal photopheresis (ECP) therapy.

In preferred embodiments the photosensitizing agent is administered ex *vivo* to the blood sample of the subject before the extracorporeal UVA-irradiation of the blood sample of the subject. In preferred embodiments the subject is or has been receiving checkpoint blockade therapy, for example, as a cancer therapy.

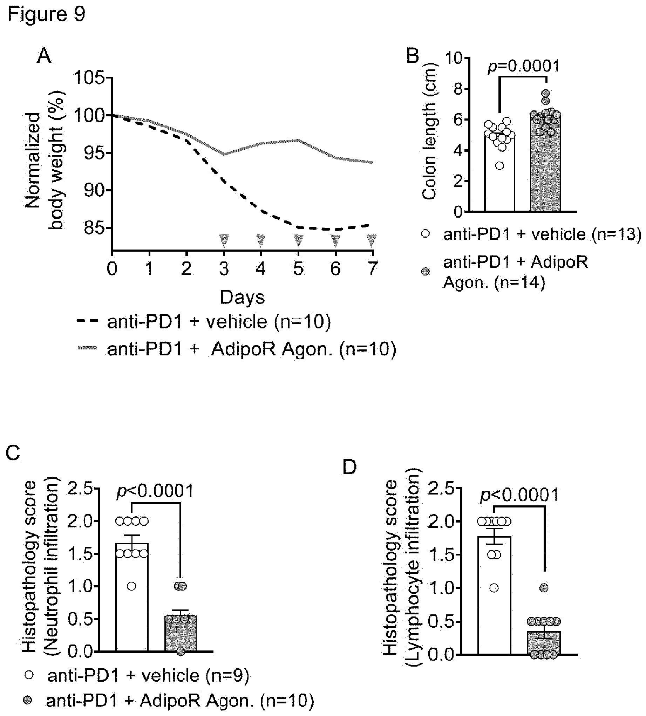

Unexpectedly, the inventors further found that also adiponectin and/or Adiponectin Receptor Agonist (ARA) administration alone also reduces and prevents ICB-induced irAEs (e.g., see Fig. 9A-H).

The invention therefore relates in another aspect to adiponectin and/or Adiponectin Receptor Agonist (ARA) for use in the treatment or prevention of immune checkpoint blockade (ICB)-induced immune related adverse events (irAEs) in a subject. In preferred embodiments the subject is or has been receiving checkpoint blockade therapy, for example, as a cancer therapy.

ICB checkpoint blockade therapy commonly comprises administration of at least one antibody, preferably at least two antibodies, that are preferably selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

In embodiments of adiponectin and/or ARA for use according to the invention, Adiponectin and/or ARA is/are administered at least one hour before, during and/or at least one hour after administration of checkpoint blockade therapy.

In embodiments of adiponectin and/or ARA for use according to the invention, Adiponectin and/or ARA is/are administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks after administration of checkpoint blockade therapy.

In embodiments of adiponectin and/or ARA for use according to the invention, Adiponectin and/or ARA is/are administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks before administration of checkpoint blockade therapy.

In embodiments of adiponectin and/or ARA for use according to the invention, Adiponectin and/or ARA is/are administered at least once daily for at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks during administration of checkpoint blockade therapy.

Preferably Adiponectin and/or ARA is/are administered at least once daily for at least 2 to 5 weeks during administration of checkpoint blockade therapy, more preferably for at least 3 weeks during administration of checkpoint blockade therapy.

In embodiments of adiponectin and/or ARA for use according to the invention, the subject suffers from cancer.

In embodiments of adiponectin and/or ARA for use according to the invention, the cancer is selected from the group comprising malignant melanoma, breast cancer or another cancer treatable by checkpoint blockade therapy, e.g., such as any cancer disclosed herein.

In embodiments of adiponectin and/or ARA for use according to the invention, the checkpoint blockade therapy comprises administration of at least one antibody, preferably at least two antibodies.

In embodiments of adiponectin and/or ARA for use according to the invention, the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

In embodiments of adiponectin and/or ARA for use according to the invention, irAEs can involve any organ, preferably the gastrointestinal tract, lungs, endocrine glands, skin, and/or liver.

In embodiments of adiponectin and/or ARA for use according to the invention, the irAE comprises an ICB-induced colitis.

In embodiments of adiponectin and/or ARA for use according to the invention, the administration of adiponectin and/or ARA reduces ICB-induced colitis.

In embodiments of adiponectin and/or ARA for use according to the invention, the administration of adiponectin and/or ARA does not interfere with the anti-tumor response induced by the checkpoint blockade therapy, which is preferably an anti-PD1 treatment, (at least partially) due to the tissue specific adiponectin induction.

In embodiments of adiponectin and/or ARA for use according to the invention, an anti-PD-1 induced anti-tumor immune response mediated by T cells in the tumor microenvironment is not impaired by the administration of Adiponectin and/or ARA, wherein the intact anti-tumor immune response is preferably proven by recall immunity experiments.

In another aspect the present invention relates to a combination medication for use in the treatment of immune checkpoint blockade (ICB)-induced immune related adverse events (irAEs) in a subject, comprising at least two of the following components:
a. adiponectin and/or Adiponectin Receptor Agonist (ARA), and
b. a photosensitizing agent,
and wherein the treatment further comprises the treatment of a blood sample of said subject with extracorporeal ultraviolet A (UVA) irradiation, preferably extracorporeal photopheresis (ECP).

In preferred embodiments the photosensitizing agent is administered ex *vivo* to the blood sample before the treatment of the blood sample with extracorporeal ultraviolet A (UVA) irradiation, preferably extracorporeal photopheresis (ECP).

In embodiments the combination medication for use in the treatment of immune checkpoint blockade (ICB)-induced immune related adverse events (irAEs) in a subject, comprises the following components:
a. adiponectin and/or Adiponectin Receptor Agonist (ARA),
b. a photosensitizing agent, and
c. a blood sample of the subject and/or lymphocytes, preferably T cells, extracted therefrom,
wherein the blood sample and/or lymphocytes extracted therefrom, has/have been subjected ex *vivo* to a treatment with the photosensitizing agent, and subsequent extracorporeal ultraviolet A (UVA) irradiation, preferably extracorporeal photopheresis (ECP).

In other words, in embodiments the combination medication for use in the treatment of immune checkpoint blockade (ICB)-induced immune related adverse events (irAEs) in a subject, comprises a substance adiponectin and/or adiponectin receptor agonist (ARA), a photosensitizing agent and a blood sample and/or lymphocytes, preferably T cells, comprised therein or extracted therefrom, wherein the blood sample and/or lymphocytes have been subjected to an extracorporeal treatment comprising (i) administration of the photosensitizing agent to the blood sample and/or lymphocytes, and subsequently (ii) an extracorporeal irradiation with ultraviolet A (UVA), preferably extracorporeal photopheresis (ECP).

In embodiments the combination medication for use in the treatment of immune checkpoint blockade (ICB)-induced immune related adverse events (irAEs) in a subject, comprises
a. adiponectin and/or adiponectin receptor agonist (ARA),
b. a photosensitizing agent, and
c. leukocytes, preferably (immunoregulatory) T cells, obtained from a method comprising the steps of
   (i) providing of a sample derived from an isolated blood sample of the subject,
   (ii) adding the photosensitizing agent to the sample, and
   (iii) subjecting the sample to UVA irradiation, preferably ECP treatment.

As shown in the examples, the positive effect and the efficacy of the administration of a sample that underwent ECP treatment according to the invention may be at least partially due to a modulation of the function of the leukocytes, specifically T cells, comprised in the blood sample, wherein said modulation is a result of the irradiation in presence of a photosensitizing agent. Therefore, the present invention also encompasses leukocytes, preferably lymphocytes, more preferably T cells, even more preferably immunomodulatory/ immunoregulatory T cells, as combination medication with adiponectin and/or adiponectin receptor agonist (ARA) for the combined use in the treatment of and/or prevention of immune-related adverse events (irAE) in a subject that is receiving a checkpoint-inhibitor therapy. Preferably, the immunomodulatory T cells were generated by subjecting blood, the buffy coat, lymphocytes and/or MNCs cells from a person, preferably a subject that has received a checkpoint-inhibitor therapy and is suspected of developing or has developed irAE, to the ECP method described herein, and are subsequently used for treating a subject that has received a checkpoint-inhibitor therapy and is suspected of developing or has developed irAE, which is preferably the blood and/or cell donor.

In preferred embodiments the blood sample of the subject is subjected extracorporeal to centrifugation before the treatment with the photosensitizing agent and UVA irradiation, such that only the buffy coat, which preferably comprises lymphocytes and optionally other leukocytes and platelets, is subjected to extracorporeal treatment with the photosensitizing agent and UVA irradiation, and is preferably afterwards administered back to the subject.

In preferred embodiments the subject is or has been receiving checkpoint blockade therapy.

The invention further relates to a combination medication for use in the treatment of immune checkpoint blockade (ICB)-induced immune related adverse events (irAEs) in a subject, wherein the combination medication comprises
(i) adiponectin and/or adiponectin receptor agonist (ARA),
(ii) a photosensitizing agent, and
(iii) a closed extracorporeal photopheresis (ECP) system,
wherein the closed ECP system forms a closed circuit with the vascular system, preferably the venous system, of the subject.

In preferred embodiments in the closed ECP system a blood sample is received from a subject's vein through an intravenous line, whereinafter the buffy coat is separated from the residual blood by centrifugation within the ECP system, subsequently the residual blood is reintroduced into a subject's vein, while the buffy coat is mixed or combined within the ECP system with the photosensitizing agent and subjected to UVA irradiation, and finally the treated and irradiated buffy coat and/or lymphocytes derived therefrom is reinfused into a vein of the subject through an intravenous line.

In preferred embodiments the buffy coat comprises T cells. In preferred embodiments the reinfused buffy coat comprises immunoregulatory T cells.

In embodiments of the invention, the photosensitizing agent is a psoralen reagent, preferably 8-methoxypsoralen. Preferably herein the 'ECP method' or `ECP treatment' of a blood sample, a fraction thereof or cells derived therefrom comprises the treatment or mixing of said blood sample, a fraction thereof or cells derived therefrom with a photosensitizing agent prior to its UVA irradiation. In preferred embodiments of the invention the photosensitizing agent is administered ex vivo/extracorporeal to a blood sample, or a fraction thereof, of the subject.

Aspects of the present invention are based on the entirely surprising finding that patients that have received a checkpoint-inhibitor therapy, for example, as a cancer treatment, which caused irAEs, can be effectively treated by administering adiponectin and/or adiponectin receptor agonist (ARA) and a photosensitizing agent and subsequently applying extracorporeal irradiation of a blood sample of the subject with ultraviolet A (UVA), preferably applying ECP. In preferred embodiments ECP basically refers to a method comprising adding extracorporeally a photosensitizing agent to a blood sample or blood-derived sample of an irAE patient, and subjecting the sample to extracorporeal UVA irradiation. Surprisingly, it was found that preforming this combined treatment approach reduces both the occurrence of and the severity of irAEs in a subject, without impairing the anti-cancer effect of the ICB therapy.

In embodiments of a substance adiponectin and/or adiponectin receptor agonist (ARA) and a photosensitizing agent for combined use according to the invention, the subject is or has been receiving checkpoint blockade therapy.

In a further aspect, the invention relates to a combination medication for the use in the treatment and/or prevention of immune checkpoint blockade (ICB)-induced immune related adverse events (irAEs) in a subject,
comprising adiponectin and/or adiponectin receptor agonist (ARA) and lymphocytes obtained from a method comprising the steps of
   - provision of a sample derived from an isolated blood sample of the subject,
   - adding a photosensitizing agent to the sample, and
   - subjecting the sample to UVA irradiation,
wherein preferably the subject is or has been receiving checkpoint blockade therapy, for example, as a cancer therapy.

In preferred embodiments the lymphocytes comprise (immunoregulatory) KLRG1⁺BTLA⁺CD47⁺TOX^{low} T cells.

Hence, in preferred embodiments the combination medication for the use in the treatment and/or prevention of immune checkpoint blockade (ICB)-induced immune related adverse events (irAEs) in a subject, comprises adiponectin and/or adiponectin receptor agonist (ARA) and (immunoregulatory) T cells obtained from a method comprising the steps of
- provision of a sample derived from an isolated blood sample of a subject,
- adding a photosensitizing agent to the sample, and
- subjecting the sample to UVA irradiation,
wherein preferably the subject is or has been receiving checkpoint blockade therapy, for example, as a cancer therapy.

This aspect of the invention is based on the observation that T cells comprised in a blood-derived sample that underwent irradiation after adding a photosensitizing adopt an immunoregulatory phenotype that is particularly advantageous when using the resulting cells in the treatment and/or prevention of irAE in a subject in combination with the administration of adiponectin and/or adiponectin receptor agonist (ARA).

In preferred embodiments, the blood sample used for generating the immunoregulatory T cells of the invention is from a subject that has received a checkpoint-inhibitor therapy and is suspected of developing or has developed symptoms of immune-related adverse events (irAE). This embodiment is particularly advantageous, since the cells are autologous to said patient and there will be no adverse events that can occur upon transplantation of heterologous cells.

Preferably, the immunoregulatory T cells for use in such a treatment or prevention have been generated by adding a photosensitizing agent to a sample derived from a blood sample of a human subject and subjecting the sample to subsequent UVA irradiation. Adding a photosensitizing agent to the blood-derived sample and subjecting the sample to UVA irradiation preferably generates or induces (the formation of) immunoregulatory T cells in said sample.

In preferred embodiments the photosensitizing agent is 8-methoxypsoralen and/or the irradiation is UVA irradiation. In the context of the invention, irradiation is preferably performed by means of an extracorporeal photopheresis (ECP) system. Irradiation of the blood sample can be performed using any suitable system or irradiation device known to the skilled person.

The ECP treatment or ECP method according to the invention relates to a method comprising the steps of
- provision of a sample derived from a blood sample of a subject that has received a checkpoint-inhibitor therapy and is suspected of developing or has developed symptoms of immune-related adverse events (irAE),
- adding a photosensitizing agent to the sample, and
- subjecting the sample to UVA irradiation, preferably extracorporeal photopheresis (ECP) therapy.

In preferred embodiments of the combination medication according to the invention, the adiponectin and/or ARA is administered before, during and/or after the ECP treatment of a sample of the subject, wherein the ECP treatment comprises extracorporeally *(ex vivo)* treating a blood sample of a patient with a photosensitizing agent and subjecting it to extracorporeal (ex *vivo)* UVA irradiation, and wherein the combination medication preferably further comprises administering said ECP-treated blood sample, a fraction thereof and/or lymphoid cells derived therefrom back to the subject.

In preferred embodiments the blood sample of the subject is subjected extracorporeally to centrifugation before the ECP treatment, such that only the buffy coat, which preferably comprises lymphocytes and optionally other leukocytes and platelets, is subjected to extracorporeal treatment with the photosensitizing agent and UVA irradiation, and is afterwards preferably administered (back) to the subject.

The combined ECP treatment of blood samples from the subject, such as in particular a blood sample comprising mononuclear cells (MNCs), leads to the generation of immunoregulatory T cells in said sample. In this context, "generation of" immunoregulatory T cells is understood as inducing or inducing the formation of such cells in the sample. In other words, cells that are comprised in the sample differentiate into or adopt a phenotype of immunoregulatory T cells.

It was found that the sample resulting from the ECP treatment according to the invention and in particular the induced immunoregulatory T cells comprised by the sample are useful for the treatment of irAE patients. It surprisingly could be shown that administration of such a sample or cells comprised in such a sample, which underwent the method of the invention, to a subject that has received a checkpoint-inhibitor therapy in combination with adiponectin/ARA is effective in preventing the occurrence of irAE and even in the treatment of irAE that is already established in the subject. The combination medication comprising adiponectin and/or ARA and an ECP-treated blood sample of the subject, or lymphocytes extracted therefrom, surprisingly showed even synergistic effects in preventing or treating ICB-induced irAE, when compared to the treatment of a subject with only one of the medications.

It was completely unexpected, that the therapy comprising administering adiponectin and/or ARA and using a sample or cells resulting from the ECP-method of the invention is even effective for irAE patients that are refractory to other immunosuppressive therapies, such as steroids or anti-TNF antibodies, and which continue to show symptoms of or still suffer from irAE after checkpoint-inhibitor treatment was discontinued.

Importantly, the ECP treatment according to the invention can be performed on a sample of the same subject that has received the checkpoint inhibitor therapy. Therefore, the cells or the sample resulting from the method of the invention can be administered to the same subject that served as a blood donor and therefore the resulting sample can represent an autologous cell therapy. In other embodiments the ECP treatment according to the invention can be performed on a sample of another subject, which is a compatible donor for the subject receiving ICB treatment, therefore the resulting sample can represent a heterologous cell therapy.

As used herein, the term "subject that is or has been receiving checkpoint-inhibitor therapy" includes subjects that are currently under ongoing checkpoint-inhibitor therapy, or subjects that have received a checkpoint-inhibitor therapy that was either discontinued, for example after irAE symptoms occurred, or completed.

In embodiments, the ECP treatment according to the invention is performed *in vitro* or ex *vivo.* As used herein, the terms *in vivo* and ex *vivo* are used synonymously. In the context of the present invention, the terms relate to a method that is performed on a blood-derived sample that has been removed from the human body, wherein the method of the invention is performed on the blood-derived sample out-side the human body. To this end, blood of a donor subject is taken out of the body, meaning out of the physiological circulatory system. As used herein, an "isolated blood sample" is a sample of blood (meaning a certain volume of blood) that is removed from the circulatory system of the donor to a location outside the body of the person. Such an isolated blood sample can be subjected or introduced into an extracorporeal photopheresis (ECP) system or an apheresis system for performing at least certain steps of the method of the invention. Therein, such a system can be an online system that is in a fluid connection with the blood circulatory system of the subject. In alternative embodiments, the method can be performed offline, wherein the blood-derived sample that is subjected to irradiation is disconnected from the circulatory system of the donor subject.

Accordingly, in embodiments, the ECP-treatment of the invention is an *in vitro* method. In embodiments of the method of the invention, the blood sample is an isolated blood sample. In embodiments, the method is an *in vitro* method and the blood sample is an isolated blood sample.

As used herein the term "blood sample" comprises all kinds of blood-derived samples, including blood-cell samples such as MNC samples that are generated from blood.

In embodiments of the invention, the subject (the blood donor and/or recipient of cells) shows symptoms of or suffers from irAE. In further embodiments, the subject has received immune checkpoint-inhibitor therapy (ICB), but the checkpoint-inhibitor therapy was either completed, or discontinued after symptoms and/or manifestation of irAE occurred in said subject. In embodiments, symptoms and/or manifestation of irAE occurred in the subject after the checkpoint-inhibitor therapy was discontinued. In embodiments, symptoms and/or manifestation of irAE were maintained after the checkpoint-inhibitor therapy was discontinued.

In embodiments of the invention, where the subject that has received a immune checkpoint-inhibitor therapy (ICB) and is suspected of developing or has developed symptoms of irAE serves as a blood donor, provision and/or isolation of the blood sample can occur at any of the time points described in the embodiments above. For example, sample isolation can occur prior to or after development of irAE symptoms. Furthermore, sample isolation can occur during checkpoint-inhibitor therapy or after discontinuation of said therapy.

In preferred embodiments of the invention, the sample resulting from the ECP-treatment according to the invention or the T cells for use according to the present invention are administered to the subject while check-point-inhibitor therapy is ongoing.

In preferred embodiments, sample isolation occurs during ongoing checkpoint-inhibitor therapy, either before or after occurrence of irAE symptoms. The sample or cells resulting from the method of the examples can be used preventively or therapeutically by administering the sample/cells to the subject during ongoing checkpoint-inhibitor therapy. Accordingly, in preferred embodiments of the invention, the subject that has received checkpoint-inhibitor therapy can receive immunoregulatory T cells, which are preferably autologous and have been generated by irradiating a blood-derived sample according to a method described herein, while checkpoint-inhibitor therapy is ongoing. Such embodiments are particularly advantageous, since checkpoint-inhibitor therapy, such as an anti-cancer checkpoint inhibitor therapy, can be maintained, while the patient receives cells of the invention and/or cells resulting from the method of the invention.

Accordingly, in such embodiments a checkpoint-inhibitor therapy can be administered to the subject for a longer period, since the administration of the irradiated cells either prevents the occurrence of irAE or ameliorates irAE so that the checkpoint-inhibitor therapy can be continued. It could surprisingly be shown that administration of such cells resulting from a method of the invention, in particular immunomodulatory T cells of the invention, does not interfere with the anti-cancer response of the subject to the checkpoint-inhibitor therapy. This represents an important advantage in comparison to known treatments/preventive measures of irAE, in particular administration of immunosuppressive drugs, such as corticosteroids. It surprisingly turned out that performing ECP did not notably alter the anti-cancer effect of checkpoint-inhibitor therapy, while parallel administration of glucocorticoids (specific class of corticosteroids), in particular prednisolone, resulted in a worse outcome, indicating a reduced effectivity of the checkpoint-inhibitor treatment.

Furthermore, in embodiments where a (human) blood-derived sample that underwent a method of adding a photosensitizing agent to the sample and subjecting the sample to irradiation is used in the treatment and/or prevention of irAE in a subject that has received a checkpoint-inhibitor therapy, irrespective of the source of the blood-derived sample (autologous or heterologous), the administration of the sample or cells resulting from such a method can occur, for example, prior to or after development of irAE symptoms, and/or during the checkpoint-inhibitor therapy or after discontinuation of the checkpoint-inhibitor therapy.

In embodiments, the subject (blood-donor and/or of recipient of cells) suffers from cancer, such as malignant melanoma or another cancer treatable by checkpoint-inhibitor therapy.

In embodiments, the subject is receiving immunosuppressive drugs, such as steroids, corticosteroid, cyclosporine and/or anti-TNF antibodies (for example infliximab) and/or is refractory to immunosuppressive drugs.

In embodiments of the invention, the irAE (of a blood-donor and/or of recipient of cells) comprise symptoms of an autoimmune disease and/or is caused by an autoimmune reaction. In preferred embodiments, the irAE comprise or are autoimmune colitis.

In embodiments, the irAE comprise at least one irAE selected from the group comprising autoimmune colitis, autoimmune hepatitis, autoimmune thyroiditis and autoimmune dermatitis.

In embodiments, the irAE comprise at least one irAE selected from the group comprising immune checkpoint inhibitor-related colitis, immune checkpoint inhibitor-related hepatitis, immune checkpoint inhibitor-related thyroiditis and immune checkpoint inhibitor-related dermatitis.

It was entirely surprising, that administration of cells that resulted from the methods described herein, such as immunoregulatory T cells, have a therapeutic effect on irAE patients that are refractory to immunosuppressive drugs that have been administered for treating the irAE symptoms. For such patients, there is no effective treatment available for ameliorating irAE symptoms and the present invention therefore represents a completely unexpected possibility of treating irAE in these patients.

In embodiments, the checkpoint-inhibitor therapy comprises administration of at least one of anti-CTLA4 antibodies and anti-PD-1 antibodies.

In embodiments, the immunoregulatory T cells for use in the treatment and/or prevention of irAE in a subject that has received a checkpoint-inhibitor therapy are autologous with respect to said subject. In alternative embodiment, the T cells may be heterologous with respect to said subject.

In embodiments, the combination medication comprising adiponectin and/or adiponectin receptor agonist (ARA) and lymphocytes, preferably (immunoregulatory) T cells, for use according to the present invention is administered upon occurrence of irAE symptoms. In embodiments, the cells are administered 1, 2, 3, 4, 5, 6 or 7 days or 1, 2, 3, 4, 5, 6, 7, 8, 9, 10, 12, 14, 16, 18, 20, 22, 24, 26, 28, 30, 35, 40, 45 or 50 weeks, preferably for at least 1-3 weeks, more preferably for at least 2 weeks after occurrence of irAE symptoms, and adiponectin and/or adiponectin receptor agonist (ARA) is administered for 1, 2, 3, 4, 5, 6 or 7 days or for 1, 2, 3, 4, 5, 6, 7, 8, 9, 10, 12, 14, 16, 18, 20, 22, 24, 26, 28, 30, 35, 40, 45 or 50 weeks, preferably for 1-5 weeks, more preferably for at least 3 weeks after occurrence of irAE symptoms. In embodiments, the combination medication comprising adiponectin and/or adiponectin receptor agonist (ARA) and immunoregulatory T cells are administered during checkpoint-inhibitor therapy is ongoing or after the checkpoint-inhibitor therapy was discontinued or completed.

In embodiments of the combination medication, the lymphocytes, preferably (immunoregulatory) T cells, are administered at least once to the subject, preferably at least twice, preferably on consecutive days. Accordingly, the subject may receive more than one dose of the lymphocytes /T cells, wherein preferably not more than one dose per day is administered. In further embodiments, the subject receives at least 2, 3, 4 or 5 dosages of the lymphocytes/T cells of the invention. In embodiments, the lymphocytes/the immunoregulatory T cells of the invention are administered to the subject at least every 8 weeks, preferably every 2-4 weeks. In embodiments the adiponectin and/or adiponectin receptor agonist (ARA) is administered before, during and/or after the administration of the lymphocytes/immunoregulatory T cells. In embodiments adiponectin is administered at least once, more preferably on two days, and at least once per day.

In embodiments of the combination medication, or a substance adiponectin and/or adiponectin receptor agonist (ARA) and a photosensitizing agent for combined use according to the invention, adiponectin and/or ARA and the photosensitizing agent are administered at least 30 minutes after administration of checkpoint blockade therapy.

In embodiments of the combination medication, or a substance adiponectin and/or adiponectin receptor agonist (ARA) and a photosensitizing agent for combined use according to the invention, the subject has been diagnosed with and/or is suffering from cancer.

In embodiments of the combination medication, or a substance adiponectin and/or adiponectin receptor agonist (ARA) and a photosensitizing agent for combined use according to the invention, the photosensitizing agent is a psoralen reagent, preferably 8-methoxypsoralen.

In embodiments of the combination medication, or a substance adiponectin and/or adiponectin receptor agonist (ARA) and a photosensitizing agent for combined use according to the invention, the wavelength of the UVA irradiation is from wavelength of 3200 to 4000 angstroms.

In embodiments of the combination medication, or a substance adiponectin and/or adiponectin receptor agonist (ARA) and a photosensitizing agent for combined use according to the invention, the subject suffers from cancer.

In embodiments of the combination medication, or a substance adiponectin and/or adiponectin receptor agonist (ARA) and a photosensitizing agent for (combined) use according to the invention, the cancer is selected from the group comprising malignant melanoma, breast cancer or another cancer treatable by checkpoint blockade therapy.

In embodiments of the combination medication, or a substance adiponectin and/or adiponectin receptor agonist (ARA) and a photosensitizing agent for (combined) use according to the invention, the checkpoint blockade therapy comprises administration of at least one antibody, preferably at least two antibodies.

In embodiments of the combination medication, or a substance adiponectin and/or adiponectin receptor agonist (ARA) and a photosensitizing agent for (combined) use according to the invention, the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

In embodiments of the combination medication, or a substance adiponectin and/or adiponectin receptor agonist (ARA) and a photosensitizing agent, for (combined) use according to the invention, irAEs can involve any organ, preferably the gastrointestinal tract, lungs, endocrine glands, skin, and liver.

In embodiments of the combination medication, or a substance adiponectin and/or adiponectin receptor agonist (ARA) and a photosensitizing agent, for (combined) use according to the invention, the irAE comprises an ICB induced colitis.

In embodiments of the combination medication, or a substance adiponectin and/or adiponectin receptor agonist (ARA) and a photosensitizing agent, for (combined) use according to the invention, said combination medication reduces ICB-induced colitis.

In embodiments of the combination medication, or a substance adiponectin and/or adiponectin receptor agonist (ARA) and a photosensitizing agent, for (combined) use according to the invention, said combination medication induces apoptosis in leukocytes, which are phagocytosed by intestinal macrophages thereby causing adiponectin production in the inflamed intestinal tract.

In embodiments of the combination medication, or a substance adiponectin and/or adiponectin receptor agonist (ARA) and a photosensitizing agent, for (combined) use according to the invention, said combination medication induces an increase of adiponectin, arginase-1 (Arg1) and tolerogenic KLRG1⁺BTLA⁺CD47⁺TOX^{low} T cells in inflamed intestines, but not in cancer tissue in said subject.

In embodiments of the combination medication, or a substance adiponectin and/or adiponectin receptor agonist (ARA) and a photosensitizing agent, for (combined) use according to the invention, said combination medication does not interfere with the anti-tumor response induced by anti-PD1 treatment due to the tissue specific adiponectin induction.

In embodiments of the combination medication, or a substance adiponectin and/or adiponectin receptor agonist (ARA) and a photosensitizing agent, for (combined) use according to the invention, an anti-PD-1 induced anti-tumor immune response mediated by T cells in the tumor microenvironment is not impaired by the said combination medication, wherein the intact anti-tumor immune response is proven by recall immunity experiments.

In another aspect the present invention relates to adiponectin and/or Adiponectin Receptor Agonist (ARA) for use in the treatment of a tumor patient, or a subject suffering from cancer, wherein said patient/subject has developed immune checkpoint blockade (ICB) induced immune related adverse events (irAEs), wherein said treatment comprises the combined administration of Adiponectin and/or ARA with an anti-tumor immunotherapy, wherein said immunotherapy comprises the administration of immunoregulatory T cells, and/or of blood samples.

In embodiments of adiponectin and/or ARA for use in the treatment of a tumor patient according to the invention, the immunoregulatory T cells and/or of blood samples are obtained from a method comprising the steps of
- providing a sample derived from an isolated blood sample of said patient,
- adding a photosensitizing agent to the sample, and subjecting the sample to irradiation.

In embodiments of adiponectin and/or ARA for use in the treatment of a tumor patient according to the invention, the photosensitizing agent is 8-methoxypsoralen and/or the irradiation is UVA irradiation.

In another aspect the present invention relates to a method of treating a subject that has received a checkpoint-inhibitor therapy and is suspected of developing or has developed symptoms of immune-related adverse events (irAE), the method comprising administering adiponectin and/or Adiponectin Receptor Agonist (ARA) at least once to the subject before, during or after subjecting said subject to an extracorporeal photopheresis (ECP) therapy, such as blood irradiation therapy by means of an ECP system.

In embodiments the extracorporeal photopheresis (ECP) therapy comprises the administration of a photosensitizing agent to a blood sample, or fragments thereof, of the subject before UVA irradiation of said blood sample, or fragments thereof.

Each optional or preferred feature of the invention that is disclosed or described in the context of one aspect of the invention is herewith also disclosed in the context of the other aspects of the invention described herein.

All features disclosed in the context of the ECP method for use in the treatment and/or prevention of immune-related adverse events (irAE) in a subject that has received a checkpoint-inhibitor therapy of the invention also relate to and are herewith disclosed also in the context of the *in vitro* method of the invention, the method of treatment of the invention and the lymphocytes and/or T cells for use in the treatment and/or prevention of immune-related adverse events (irAE) in a subject that has received a checkpoint-inhibitor therapy of the invention, and vice versa.

### DETAILED DESCRIPTION OF THE INVENTION

All cited documents of the patent and non-patent literature are hereby incorporated by reference in their entirety.

The present invention is directed to adiponectin and/or adiponectin receptor agonist (ARA) or a combination medication comprising adiponectin and/or adiponectin receptor agonist (ARA), a photosensitizing agent and lymphocytes obtained from a method comprising the steps of (i) providing of a sample derived from an isolated blood sample of the subject, (ii) adding the photosensitizing agent to the sample, and (iii) subjecting the sample to UVA irradiation, preferably ECP treatment, for use in the treatment of immune checkpoint blockade (ICB)-induced immune related adverse events (irAEs) in a subject.

In preferred embodiments the photosensitizing agent is administered ex *vivo* to the blood sample of the subject before the extracorporeal UVA-irradiation of the blood sample of the subject. In preferred embodiments the subject is receiving checkpoint blockade therapy, for example, as a cancer therapy.

As used herein, the term "subject' means a human or non-human animal selected for treatment or therapy. The subject or patient, such as the subject in need of treatment or prevention, may be an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e. g. gorilla, chimpanzee, orangutan, gibbon), or a human. The meaning of the terms "animal", "mammal", etc. is well known in the art and can, for example, be deduced from Wehner und Gehring (1995; Thieme Verlag). The term subject may also refer to a "patient" and may be used herein interchangeably therewith. In the context of this invention, it is particularly envisaged that animals are to be treated which are economically, agronomically or scientifically important. Preferably, the subject/patient is a mammal. More preferably, the subject/patient is a human.

In embodiments of the present invention, the subject that has received a checkpoint-inhibitor therapy suffers from cancer, such as malignant melanoma or another cancer treatable by checkpoint-inhibitor therapy.

"Adiponectin" (also named Acrp30, GBP28, apM1, orAdipoQ) is a protein hormone and adipokine (cytokine secreted by adipose tissue), which is involved in regulating glucose levels as well as fatty acid breakdown. Adiponectin is known to bind to at least receptors, wherein two receptors comprise homology to G protein-coupled receptors, namely Adiponectin receptor 1 (AdipoR1), and Adiponectin receptor 2 (AdipoR2), and one receptor comprises similarity with the cadherin family, namely T-cadherin CDH13. Adiponectin receptors affect the downstream target AMP kinase, an important cellular metabolic rate control point. Expression of the receptors is correlated with insulin levels. Adiponectin commonly regulates a number of metabolic processes, such as glucose regulation and fatty acid oxidation, comprising glucose flux, decreased gluconeogenesis, increased glucose uptake, lipid catabolism, β-oxidation, triglyceride clearance, protection from endothelial dysfunction (important facet of atherosclerotic formation), insulin sensitivity, weight loss, control of energy metabolism, upregulation of uncoupling proteins, reduction of TNF-alpha, promotion of reverse cholesterol transport. Adiponectin is known to be able to automatically self-associate into larger structures, starting from three adiponectin molecules forming a homotrimer, followed by further self-association into hexamers or dodecamers. Monomeric (30-kDa) adiponectin is commonly not observed in the circulation and appears to be confined to adipocytes.

### Irradiation and Extracorporeal photopheresis (ECP)

Photopheresis, or extracorporeal photopheresis or ECP, is a form of apheresis and photodynamic therapy in which blood is treated with a photosensitizing agent and subsequently irradiated with specified wavelengths of light to achieve an effect. For example, buffy coat (WBC + platelets) can be separated from whole blood, chemically treated with 8-methoxypsoralen (instilled into collection bag or given per os in advance), exposed to ultraviolet light (UVA), and returned to the patient. Activated 8-methoxypsoralen crosslinks DNA in exposed cells, ultimately resulting apoptosis of nucleated cells. The photochemically damaged T-cells returned to the patient appear to induce cytotoxic effects on T-cell formation.

Photopheresis involving 8-methoxypsoralen was first described in a 1987 New England Journal of Medicine publication (Edelson, R, et al. (1987). "Treatment of cutaneous T-cell lymphoma by extracorporeal photochemotherapy. Preliminary results". New England Journal of Medicine. 316 (6): 297-303.). Photopheresis is currently standard therapy approved by the U.S. Food and Drug Administration (FDA) for cutaneous T-cell lymphoma. Evidence suggests that this treatment might be effective in the treatment of graft-versus-host disease. Photopheresis has also been used successfully in the treatment of epidermolysis bullosa acquisita when all other treatments have been ineffective.

ECP as used herein comprises blood irradiation therapy. In embodiments, ECP and ECP systems of the invention relates to blood irradiation therapy and systems for blood irradiation therapy. In embodiments of the invention, ECP relates to ECP with exception of blood irradiation therapy.

Blood irradiation therapy is a procedure in which the blood is exposed to low level red light (often laser light) for therapeutic reasons. Blood irradiation therapy can be administered in three ways.

Extracorporeally, drawing blood out and irradiating it in a special cuvette. This method is used for the ultraviolet (UV) blood irradiation (UVBI) by UV lamps. The laser light is monochromatic, i.e., it has such a wavelength that allows you to bring light into the optical fiber and carry out irradiation intravenously through a catheter in a vein. This method is more simple and effective. Blood irradiation therapy is also administered externally through the skin on the projection of large blood vessels.

"Intravenous or intravascular laser blood irradiation (ILBI)" involves the *in-vivo* illumination of the blood by feeding low level laser light generated by a 1-3 mW helium-neon laser at a wavelength of 632.8 nm into a vascular channel, usually a vein in the forearm, under the assumption that any therapeutic effect will be circulated through the circulatory system. Most often wavelengths of 365, 405, 525 and 635 nm and power of 2.3 mW are used. The technique is widely used at present in Russia, less in Asia, and not extensively in other parts of the world. It is shown that ILBI improves blood flow and its transport activities, therefore, tissue trophism, has a positive effect on the immune system and cell metabolism. This issue is subject to skepticism. There have been some calls to increase research on this topic. Transcutaneous therapy applies laser light on unbroken skin in areas with large numbers of blood vessels (such as the forearm). Because of the skin acting as a barrier to the blood, absorbing low level laser energy, the power of the laser is often boosted to compensate. The problem can be solved by using pulsed matrix laser light sources. Extracorporeal irradiation is used only for ultraviolet blood irradiation, that involves drawing blood out through a vein and irradiating it outside of the body. Though promoted as a treatment for cancer, a 1952 review in the Journal of the American Medical Association and another review by the American Cancer Society in 1970 concluded the treatment was ineffective.

"Extracorporeal photopheresis (ECP)", also known as "extracorporeal photoimmunotherapy" or "photochemotherapy", is a leukapheresis-based therapy which was initially used in patients with cutaneous T-cell lymphoma (CTCL). Specifically, for the treatment of therapy refractory CTCL patients suffering from the leukemic variant, the Sézary Syndrome, ECP received FDA (United States Food and Drug Administration) approval in 1988. During ECP, whole blood of the patient is collected via a cubital vein, or a permanently implanted catheter, for separation of leucocytes from plasma and non-nucleated cells. With a specifically constructed device for this procedure, collected leukocytes, the so-called buffy coat, are then exposed to ultraviolet-A (UVA) irradiation in the presence of a photosensitizing agent, 8-methoxypsoralen prior to reinfusion to the patient.

Two basically different methods for performing ECP procedure have been described and comprised by the present invention. They differ in the device used for leukocyte collection and UVA irradiation: the "closed system" and the so called "open system." The closed system is based on the original design by Edelson and coworkers and is the only FDA-approved system.

In a closed ECP system the buffy coat, comprising lymphocytes and platelets, is separated from the blood by centrifugation. While red blood cells and plasma are redirected into the vein of the subject (through an intravenous line), the separated buffy coat is mixed with a photosensitizing substance, preferably 8-methoxypsoralen, and is then subjected to ultraviolet A (UVA) irradiation (320-400 nm). The UVA radiation induces the incorporation of the photosensitizing substance into the DNA of the lymphocytes. Subsequently the treated buffy coat is reinfused into the patient through an intravenous line.

The open system is a system incorporating different separation instruments, mostly used outside the United States. Although ECP is a valid treatment method since 30 years and over 2 million of treatments have been performed, there are no reports about negative cytogenetic effects.

Indications for initiating ECP were continuously extended since its introduction. ECP treatments are generally well-tolerated by patients and there are almost no significant unwanted side effects. Taken together, ECP combines an excellent safety profile with efficacy.

Extracorporeal photopheresis (also sometimes referred to as extracorporeal photochemotherapy) is a process that includes: (1) collection of mononuclear cells (MNC) from a patient, (2) photoactivation treatment of the collected MNC cells; and (3) reinfusion of the treated cells (MNC) back to the patient. More specifically, ECP involves the extracorporeal exposure of peripheral blood mononuclear cells combined with a photoactive compound, such as 8-methoxypsoralen or "8-MOP" which is then photoactivated by ultraviolet light, followed by the reinfusion of the treated mononuclear cells. It is believed that the combination of 8-MOP and UV radiation causes apoptosis or programmed cell death of ECP-treated T-cells.

Although the precise mechanism of action in ECP treatment (in the different disease states) is not fully known, according to early theories, it was believed that photoactivation causes 8-MOP to irreversibly covalently bind to the DNA strands contained in the T-cell nucleus. When the photochemically damaged T-cells are reinfused, cytotoxic effects are induced. For example, a cytotoxic T-cell or "CD8+ cell" releases cytotoxins when exposed to infected or damaged cells or otherwise attacks cells carrying certain foreign or abnormal molecules on their surfaces. The cytotoxins target the damaged cell's membrane and enter the target cell, which eventually leads to apoptosis or programmed cell death of the targeted cell. In other words, after the treated mononuclear cells are returned to the body, the immune system recognizes the dying abnormal cells and begins to produce healthy lymphocytes (T-cells) to fight against those cells.

In addition to the above, it has also been theorized that extracorporeal photopheresis also induces monocytes (a type of mononuclear cell) to differentiate into dendritic cells capable of phagocytosing and processing the apoptotic T-cell antigens. When these activated dendritic cells are re-infused into systemic circulation, they may cause a systemic cytotoxic CD8+ T-lymphocyte-mediated immune response to the processed apoptotic T-cell antigens like that described above. It will be appreciated that other possible mechanisms of action may be involved in achieving the benefits that have been observed from the ECP treatment of mononuclear cells and the subsequent benefits to patients undergoing ECP based therapies.

More recently, it has been postulated that ECP may result in an immune tolerant response in the patient. For example, in the case of graft versus-host disease, the infusion of apoptotic cells may stimulate regulatory T-cell generation, inhibit inflammatory cytokine production, cause the deletion of effective T-cells and result in other responses. See Peritt, "Potential Mechanisms of Photopheresis in Hematopoietic Stem Cell Transplantation," Biology of Blood and Marrow Transplantation 12:7-12 (2006). While presently the theory of an immune tolerant response appears to be among the leading explanations, there exist other theories as to the mechanism of action of ECP relative to graft-versus-host disease, as well as other disease states.

Systems for performing ECP include, for example, the UVAR XTS Photopheresis System and the CellEx Photopheresis System available from Therakos, Inc., of Exton, Pa. Further details of performing ECP on the Therakos system can be found, for example, in U.S. Pat. No. 5,984,887.

There are currently two commonly used methods for performing photopheresis-online and offline systems and methods.

In online methods, a dedicated photopheresis device, such as the Therakos device mentioned above, is used to perform the entire therapy including reinfusion of treated MNCs. Such devices are "dedicated" photopheresis devices, designed only for performing photopheresis and cannot perform other collection protocols needed in a hospital or blood processing setting including, for example, multifunctional apheresis protocols for collection of platelets, plasma, RBCs, ganulocytes and/or perform plasma/RBC exchange protocols.

In offline photopheresis methods, a multifunctional apheresis device may be used to collect mononuclear cells. The collected MNCs, typically contained in one or more collection containers, are severed or otherwise separated from the tubing set used during collection, where they are later treated in a separate irradiation or UVA light device followed by manual reinfusion of the treated cells to a patient. However, during such offline methods, when the cells are transferred from the apheresis device to the irradiation device (which device may be located in another room or laboratory) communication with the donor must be severed and accordingly, the cells detached from the donor. Thus, additional traceability procedures are required to ensure that the treated MNC product is ultimately reinfused into the correct donor.

In embodiments ECP employs (a) apheresis with ex *vivo* collection of peripheral mononuclear cells, (b) photoactivation with exposure of leukocyte-enriched plasma to the photosensitizing agent 8-methoxypsoralen and ultraviolet A light, (c) reinfusion of such modified ECP-treated cells to the patient.

According to the invention, the expression "extracorporeal blood purification" refers preferably to the process of removing substances from body fluids through their clearance from flowing blood in a diverted circuit outside the patient's body (extracorporeal). Said substances may include endogenous toxins (i.e., uremic toxins), exogenous poisons (i.e., ethylene glycol or fungal toxin), administered drugs, viruses, bacteria, antibodies, metabolites and proteins (i.e., IMHA, myasthenia gravis), abnormal cells (i.e., leukemia), and excessive water. Therapeutic procedures include hemodialysis, including intermittent hemodialysis (HD, HDF, HF) and continuous renal replacement therapy (CRRT); hemoperfusion; plasma exchange and therapeutic apheresis. Such methods are known to a skilled person and the device of the invention can be incorporated accordingly.

The expression "blood" as used herein refers to whole blood which contains all components of the blood of an organism, including red cells, white cells, and platelets suspended in plasma. The expression "blood plasma" refers to the fluid, composed of about 92% water, 7% proteins such as albumin, gamma globulin, fibrinogen, complement factors, clotting factors, and 1% mineral salts, sugars, fats, electrolytes, hormones and vitamins which forms part of whole blood but no longer contains red and white cells and platelets. In the context of the present invention, the expression "blood plasma" or "plasma" refers to specific fractions of the above defined blood plasma in its standard meaning, such as, for example, blood serum.

In therapeutic apheresis whole blood can be treated or blood is separated into its component fractions, for example by centrifugation or by means of a plasma membrane or filter, and the fraction containing the solute which shall be removed, is specifically treated prior to return to the patient.

The present invention provides for an apheresis treatment in which whole blood or plasma (containing the target proteins) is removed from the patient's flowing blood and, after having been contacted with an apheresis device or matrix is returned to the patient.

### ICB treatment

"Immune checkpoints" are an important part of the immune system, as they prevent an excessive immune response, which can lead to autoimmune reactions or generally damage healthy cells of the body. Immune checkpoints are effective when proteins on the surface of T cells (e.g., PD-1) recognize and bind to checkpoint proteins (e.g., PD-L1), on other cells, e.g., tumor cells. The binding of the checkpoint proteins to the partner proteins on T cells can result in T cell-inhibition and prevention of T cells killing the other cells. This mechanism can prevent auto-immune actions of T cells, but also the killing of tumor cells.

"Immune checkpoint blockade" (ICB) therapy, "checkpoint-inhibitor therapy" or "immune checkpoint inhibitors", block (immune) checkpoint proteins from binding to their partner proteins on T cells, thereby preventing the inhibition of T cell-mediated killing of, e.g., cancer cells. Immune checkpoint blockade therapy can comprise a treatment with anticytotoxic T lymphocyte-associated antigen 4 (CTLA-4) (ipilimumab and tremelimumab), anti-programmed cell death receptor (PD-1) (nivolumab and pembrolizumab), or anti-PD-ligand (PD-L1) (durvalumab, atezolizumab, and avelumab) monoclonal antibodies. ICB uses the infiltration of immune cells into a tumor or tumor environment to initiate and/or revive an effective anti-tumor immune response. The PD-1/PD-L1 signaling pathway is considered crucial for the interactions between immune, stromal and tumor cells. Herein the terms "Immune checkpoint blockade" (ICB) therapy, "checkpoint-inhibitor therapy" or "immune checkpoint inhibitor" therapy may be used interchangeably.

"Immune checkpoint inhibitors" or "ICB" therapy can cause various side effects, which can depend on the patient health at treatment initiation, the type of cancer that is treated, its stage, the drug(s) administered and its/their dose. A group of side effects can be summarized as immune-related adverse events (irAEs).

### Immune-related adverse events (irAE)

As used herein, the term "Immune-related adverse events" (irAE) relates any side effect specific that specifically occur in the context of an immune checkpoint inhibitor treatment, for example in the context of a cancer therapy. IrAEs are unique and are different to adverse events occurring in the context of traditional cancer therapies, and typically have a delayed onset and prolonged duration. IrAEs can involve any organ or system. These effects are frequently low grade and are treatable and reversible; however, some adverse effects can be severe and lead to permanent disorders. Management is primarily based on corticosteroids and other immunomodulatory agents, which should be prescribed carefully to reduce the potential of short-term and long-term complications.

Frequent immune-related adverse events (irAEs) comprise gastrointestinal, endocrine, and dermatologic toxicities. Rare but more severe and potential fatal irAEs can comprise neurotoxicity, cardiotoxicity, and pulmonary toxicity. The observed cytotoxicity is commonly graded according to 4 grades of severity (presently according to the Common Terminology Criteria for Adverse Events, European Society for Medical Oncology guideline and American Society of Clinical Oncology guideline; see e.g., Brahmer et al., 2018, Haanen et al., 2018). Higher grades of irAE cytotoxicity are commonly treated with discontinuation of ICB treatment and/or initiation of glucocorticoid-treatment, e.g., comprising administration of prednisone.

In particular comprised by the term irAEs are symptoms of an autoimmune disease and autoimmune disease, such as (autoimmune) colitis, (autoimmune)hepatitis, (autoimmune)thyroiditis and (autoimmune)dermatitis.

The irAE due to administration of a checkpoint-inhibitor therapy in the present invention is not particularly limited. An irAE is to be understood as an adverse event that is presumed to be immune-related: irAE (see, for example, Drug Interview Form of OPDIVO^{®} Intravenous Infusion 20 mg-100 mg, revised in April 2016 (version 9); Properties and Handling of Adverse Events of an Anti-CTLA-4 Antibody, Ipilimumab (YERVOY^{®}), dated Aug. 24, 2015, issued by the Committee on Safety of New Drugs for Malignant Melanoma of the Japanese Dermatological Association).

Specific embodiments of the irAE of the present invention include interstitial lung disease, myasthenia gravis, myositis, colitis, type 1 diabetes mellitus, hepatic dysfunction (hepatic disorder), pulmonary disorder such as hepatitis (e.g., autoimmune pneumonia), pituitarism such as hypopituitarism or hypophysitis, thyroid dysfunction such as hypothyroidism, neuropathy, nephropathy, encephalitis, adrenal disorder such as adrenal insufficiency, severe skin disorder, venous thromboembolism, infusion reaction, psoriasis, psoriasiform rash, diarrhea (e.g., severe diarrhea), rheumatoid arthritis, uveitis, episcleritis, bursitis, exacerbation of radiodermatitis, chronic inflammatory demyelinating polyneuropathy (hereinafter also referred to as demyelinating polyneuropathy), biliary tract disorder, or nephritis, and pituitarism is preferable.

The immune-related adverse event (IrAE) has been commonly known to occur, for example, after 8 to 12 weeks after administration of ICB. The immune-related adverse event can be evaluated by "grade" or "irAE evaluation". Here, "irAE evaluation" is an index representing the seriousness of a disease, and is represented by 1 to 3. In the irAE evaluation, 1 represents a condition that "does not require additional therapeutic intervention due to irAE", 2 represents a condition that "requires drug intervention, etc., due to irAE, but does not require hospitalization treatment or does not require interruption of treatment", and 3 represents a condition that "requires drug intervention, etc., accompanied by hospitalization due to irAE and requires interruption of treatment". The correspondence between "irAE evaluation" and "grade" varies depending on each disease.

### Immune checkpoint molecules and checkpoint modulators

In the context of the present invention, an immune checkpoint inhibitor is a drug that activates immune cells by modulating immune checkpoint molecules.

Immune checkpoint molecules are molecules in the immune system that either turn up a signal (co-stimulatory molecules) or turn down a signal provided to immune effector cells. Thus, immune checkpoint molecules can be subdivided into co-stimulatory checkpoint molecules or co-inhibitory checkpoint molecules. Co-stimulatory checkpoint molecules include co-stimulatory lymphocyte receptors, which are lymphocyte surface-receptors that can lead to an activation or stimulation of lymphocyte effector functions. Co-inhibitory checkpoint molecules include co-inhibitory lymphocyte receptors, which are lymphocyte surface-receptors that can lead to an inhibition of lymphocyte effector functions.

Co-stimulatory checkpoint molecules comprise, without limitation, HVEM, CD27, CD40, OX40, GITR, CD137, CD28 and ICOS.

In preferred embodiments of the present invention, the term co-stimulatory lymphocyte receptor does not refer to CD122.

HVEM (Herpesvirus entry mediator, CD270) is also known as tumor necrosis factor receptor superfamily member 14 (TNFRSF14) and is a receptor of the TNF-receptor superfamily, which can bind to BTLA.

CD27 supports antigen-specific expansion of naive T cells and is vital for the generation of T cell memory and is also a memory marker of B cells. CD27's activity is governed by the transient availability of its ligand, CD70, on lymphocytes and dendritic cells. CD27 co-stimulation is known to suppress Th17 effector cell function. The agonistic monoclonal antibody CDX-1127/ Varlilumab against CD27 has been shown to be effective in the context of T cell receptor stimulation in animal models.

CD28 is constitutively expressed on almost all human CD4+ T cells and on around half of all CD8 T cells. Binding of one of its two ligands CD80 and CD86, expressed for example on dendritic cells, prompts T cell expansion.

CD40 is expressed on a variety of immune system cells including antigen-presenting cells. The ligand of CD40 is called CD40L, also known as CD154, and is transiently expressed on the surface of activated CD4+ T cells, as its ligand. CD40 signaling is known to 'license' dendritic cells to mature and thereby trigger T-cell activation and differentiation.

4-1BB (CD137) is bound by CD137 ligand resulting in T-cell proliferation. CD137-mediated signaling is also known to protect T cells, and in particular, CD8+ T cells from activation-induced cell death. The fully human IgG2 agonistic monoclonal antibody Utomilumab (PF-05082566) targets 4-1BB to stimulate a more intense immune system attack on cancers.

OX40 (CD134) has OX40L (CD252) as its ligand. OX40 promotes the expansion of effector and memory T cells and is also known for its ability to suppress the differentiation and activity of T-regulatory cells. OX40 is being transiently expressed after T-cell receptor engagement, which is why it is only upregulated on the most recently antigen-activated T cells within inflammatory lesions, which is why OX40 is a valuable drug target. Agonistic anti-OX40 monoclonal antibodies have been shown to have clinical utility in advanced cancer. The pharma company AstraZeneca has three drugs in development targeting OX40: MEDI0562 is a humanized OX40 agonist; MEDI6469, murine OX40 agonist; and MEDI6383, an OX40 agonist.

GITR (Glucocorticoid-Induced TNFR family Related gene) prompts T cell expansion. The ligand for GITR (GITRL) is mainly expressed on antigen presenting cells. Antibodies to GITR have been shown to promote an anti-tumor response through loss of Treg lineage stability.

ICOS (Inducible T-cell costimulator, also called CD278) is expressed on activated T cells. Its ligand is ICOSL, expressed mainly on B cells and dendritic cells. The molecule seems is important in T cell effector function.

Co-inhibitory checkpoint molecules comprise, without limitation, A2AR, B7-H3, B7-H4, BTLA, CTLA-4, IDO, KIR, LAG3, PD-1, TIM-3, TIGIT and VISTA.

A2AR (Adenosine A2A receptor) is regarded as an important checkpoint in cancer therapy because adenosine in the immune microenvironment, leading to the activation of the A2a receptor, is negative immune feedback loop and the tumor microenvironment has relatively high concentrations of adenosine.

B7-H3, also called CD276, was originally understood to be a co-stimulatory molecule but is now regarded as co-inhibitory. MacroGenics is working on MGA271 (Enoblituzumab), which is an Fc-optimized monoclonal antibody that targets B7-H3.

B7-H4 (or VTCN1) is expressed by tumor cells and tumor-associated macrophages and plays a role in tumor evasion.

BTLA (B and T Lymphocyte Attenuator, also called CD272) is a co-inhibitory receptor, which has HVEM (Herpesvirus Entry Mediator) as its ligand. Surface expression of BTLA is gradually downregulated during differentiation of human CD8+ T cells from the naive to effector cell phenotype, however tumor-specific human CD8+ T cells express high levels of BTLA.

IDO (Indoleamine 2,3-dioxygenase) is a tryptophan catabolic enzyme with immune-inhibitory properties. Another important molecule is TDO, tryptophan 2,3-dioxygenase. IDO is known to suppress T and NK cells, generate and activate Tregs and myeloid-derived suppressor cells, and promote tumor angiogenesis.

KIR (Killer-cell Immunoglobulin-like Receptor) is a receptor for MHC Class I molecules on Natural Killer cells. Lirilumab is a monoclonal antibody to KIR.

LAG-3 (Lymphocyte Activation Gene-3) works to suppress an immune response by action to Tregs as well as direct effects on CD8+ T cells.

"Cytotoxic T-lymphocyte-associated protein 4" (CTLA-4, or CTLA4, or CD152 - cluster of differentiation 152), is a protein receptor constitutively expressed in regulatory T cells, which functions as an immune checkpoint that can downregulate a T cell-mediated immune response. CTLA-4 can inactivate T cell effector function when bound to CD80 or CD86 on the surface of antigen-presenting cells. CTLA-4 is a regulatory molecule that represses T cell effector function after initial activation by costimulatory signals. Human monoclonal antibodies targeting CTLA-4 are used in ICB treatment and can increase T-cell function and anti-tumor immune response. Immune checkpoint blockade (ICB) therapy can comprise a treatment with anticytotoxic T lymphocyte-associated antigen 4 (CTLA-4) (ipilimumab and tremelimumab) monoclonal antibodies.

"Programmed cell death protein 1" (PD-1, or CD279 -cluster of differentiation 279), is a cell surface protein/receptor of B and T cells, which plays a role in the regulation of auto-immune responses by promoting self-tolerance through suppression of T cell inflammatory activity. PD-1 has two ligands, PD-L1 and PD-L2. PD-1 is an immune checkpoint, which guards against autoimmunity through two mechanisms: by promoting apoptosis of antigen-specific T cells in lymph nodes, and by reducing apoptosis in regulatory T cells. An advantage of targeting PD-1 is that it can restore immune function in the tumor microenvironment. Immune checkpoint blockade (ICB) therapy can comprise a treatment with anti-programmed cell death receptor (PD-1) monoclonal antibodies, such as nivolumab (Opdivo - Bristol Myers Squibb), Pembrolizumab (Keytruda, MK-3475, Merck), Pidilizumab (CT-011, Cure Tech) and BMS-936559 (Bristol Myers Squibb). Both Atezolizumab (MPDL3280A, Roche) and Avelumab (Merck KGaA, Darmstadt, Germany & Pfizer) are monoclonal antibodies directed against PD-L1, the ligand of PD-1.

The transmembrane protein "programmed death-ligand 1" (PD-L1 or, CD274 - cluster of differentiation 274, or B7-H1 - B7 homolog 1) is encoded in human by the CD274 gene. IFN-γ stimulation induces PD-L1 expression on T cells, NK cells, macrophages, myeloid dendritic cells, B cells, epithelial cells, and vascular endothelial cells. Binding of PD-L1 to its receptor PD-1, which is present on activated T cells, B cells and myeloid cells, modulates activation or inhibition of the immune cells. The binding of PD-L1 to its receptor PD-1 on T cells initiates a signal that inhibits TCR-mediated activation of T cell proliferation und IL-2 production. Interaction of PD-1 with its ligand PD-L1 can prevent autoimmunity. PD-L1 can be highly expressed in a variety of cancers, particularly lung cancer. Immune checkpoint blockade (ICB) therapy can comprise a treatment with anti-PD-ligand (PD-L1) (durvalumab, atezolizumab, and avelumab) monoclonal antibodies.

TIM-3 (T-cell Immunoglobulin domain and Mucin domain 3) expresses on activated human CD4+ T cells and regulates Th1 and Th17 cytokines. TIM-3 acts as a negative regulator of Th1/Th17 function by triggering cell death upon interaction with its ligand, galectin-9.

VISTA (V-domain Ig suppressor of T cell activation) is a protein that is primarily expressed on hematopoietic cells so that consistent expression of VISTA on leukocytes within tumors may allow VISTA blockade to be effective across a broad range of solid tumors.

TIGIT (T cell immunoreceptor with Ig and ITIM domains, also called WUCAM and Vstm3) is an immune receptor present on some T cells and Natural Killer Cells and regulates T cell mediated immunity. TIGIT could bind to CD155 on DCs and macrophages with high affinity and to CD112 with lower affinity.

Co-inhibitory lymphocyte receptors of the present invention comprise PD-1, CTLA-4, TIM-3, LAG-3, TIGIT, BTLA or VISTA. Co-stimulatory lymphocyte receptors of the present invention comprise OX40, 4-1BB, GITR, CD27, HVEM, CD28 or CD40.

An inhibitor of a receptor prevents the generation of a signal by the respective receptor. Accordingly, an inhibitor of a co-inhibitory lymphocyte receptor is a molecule that prevents the activation of the respective receptor and thereby prevents the generation of an inhibitory signal. Conversely, an activator of a receptor induces the generation of a signal by the respective receptor and an activator of a co-stimulatory lymphocyte receptor leads to the generation of a stimulatory signal.

Checkpoint modulators are molecules that interfere with the activity of immune checkpoint molecules, either by stimulating or inhibiting the activity of immune checkpoint molecules.

Soluble checkpoint modulators are molecules that may be able to freely diffuse and, for example, are not bound to a cell membrane or do not remain intracellular.

Checkpoint inhibitors in the sense of the invention comprise lymphocyte-stimulating checkpoint modulators, which are molecules that lead to an activation of lymphocytes, preferably effector T cells, either through activation of a co-stimulatory checkpoint molecule, or through inhibition of a co-inhibitory checkpoint molecules. Furthermore, soluble lymphocyte-stimulating checkpoint modulators include molecules that interfere with the activation of membrane bound immune checkpoint molecules, such as a soluble form of the respective immune checkpoint molecule.

Checkpoint modulators can be naturally occurring molecules or engineered molecules with the respective function interfering with or modulating the activity of an immune checkpoint molecule. Checkpoint modulators include, for example, antibodies or antibody-fragments activity directed against immune checkpoint molecule with agonistic or antagonistic, and ligands or modified ligands of immune checkpoint molecules.

The present invention encompasses both treatment and prophylactic treatment of a subject. A "prophylactic" treatment is a treatment administered to a subject who does not exhibit signs of a disease or exhibits only early signs for the purpose of decreasing the risk of developing pathology. Prophylactic treatment is administered to prevent the progression, acceleration, escalation and/or occurrence of a disease.

"Glucocorticosteroids", which are further called "glucocorticoids", "corticosteroids" or simply "steroids", are the presently most effective anti-inflammatory substances used in the treatment of chronic inflammatory, auto-immune and immune diseases. Glucocorticoids induce immunosuppression, which majorly comprises the decreases in the function and numbers of lymphocytes, of both B cells and T cells. Glucocorticoids are lipophilic hormones, which can be subclassified into glucocorticoids produced in the zona fasciculata of the adrenal cortex, mineralocorticoids produced in the zona glomerulosa, and sex hormones produced in the zona reticularis and to a great extent in the gonads. A variety of synthetic glucocorticoids are available for therapeutic use. Examples of glucocorticoids are cortisol (hydrocortisone), cortisone, prednisone, prednisolone, methylprednisolone and dexamethasone.

### Immune cells

Immune cells as described herein relate to biological cells involved in the immune response in a subject. Immune cells are preferably selected from T Cells, B Cells, Dendritic Cells, Granulocytes, Innate Lymphoid Cells (ILCs), Megakaryocytes, Monocytes/Macrophages, Natural Killer (NK) Cells, Platelets, Red Blood Cells (RBCs) and/or Thymocytes.

The term "immune cells" comprises the MNCs comprised in blood, which may also be called peripheral blood mononuclear cell (PBMC). PBMCs comprise any peripheral blood cell having a round nucleus and consist mainly of lymphocytes (T cells, B cells, NK cells) and monocytes, whereas erythrocytes and platelets have no nuclei, and granulocytes (neutrophils, basophils, and eosinophils) have multi-lobed nuclei. In humans, lymphocytes (lymphoid cells) make up the majority of the PBMC population, followed by monocytes, and only a small percentage of dendritic cells. These cells can be extracted from whole blood using ficoll, a hydrophilic polysaccharide that separates layers of blood, and "density gradient centrifugation", which will separate the blood into a top layer of plasma, followed by a layer of PBMCs and a bottom fraction of polymorphonuclear cells (such as neutrophils and eosinophils) and erythrocytes. The polymorphonuclear cells can be further isolated by lysing the red blood cells. Basophils are sometimes found in both the denser and the PBMC fractions.

In preferred embodiments of the present invention leukocytes may particularly refer to lymphocytes, wherein leukocytes or lymphocytes may preferably comprise or refer to T cells.

A "buffy coat" can be isolated from blood plasma and erythrocytes by centrifugation. The buffy coat can also be referred to as the fraction of an anticoagulated blood sample that contains most of the white blood cells (leucocytes) and platelets after centrifugation. It is rich in a number of immune cells including platelets and leukocytes (white blood cells), such as lymphocytes, granulocytes, monocytes, and macrophages. Buffy coats can be used for enrichment of high numbers of leukocyte subset from peripheral blood mononuclear cells (PBMCs). PBMCs are individual lymphocytes and monocytes that can be separated from the residual whole blood sample through a process called density gradient centrifugation (see above).

"T cells" or T lymphocytes are a type of lymphocyte (a subtype of white blood cell) that plays a central role in cell-mediated immunity. They can be distinguished from other lymphocytes, such as B cells and natural killer cells, by the presence of a T-cell receptor on the cell surface. The several subsets of T cells each have a distinct function. T cell subtypes include, without limitation, T helper type 1 (Th1) cells, T helper type 2 (Th2) cells, T helper type 9 (Th9) cells, T helper type 17 (Th17) cells, T helper type 22 (Th22) cells, Follicular helper T (Tfh) cells, Regulatory T (Treg) cells, Natural killer T (NKT) cells, Gamma delta T cells, CD8+ cytotoxic T lymphocytes (CTLs). Further non-limiting embodiments of T cells include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)-restricted, natural killer (NK)-like T lymphocytes. The T cell can be a CD4+ T cell, a cytotoxic T cell (CTL; CD8+ T cell), CD4+CD8+ T cell, CD4 CD8 T cell, or any other subset of T cells.

"Natural killer (NK) cells" are cytotoxic innate effector cells analogous to the cytotoxic T cells of the adaptive immune system. NK cells are a group of innate immune cells that show spontaneous cytolytic activity against stressed cells, e.g., cancer cells or virus-infected cells. Upon activation, NK cells can secrete cytokines such as interferon-γ (IFN-γ), tumor necrosis factor-a (TNF-α), granulocyte macrophage colony-stimulating factor (GM-CSF), and chemokines (CCL1, CCL2, CCL3, CCL4, CCL5, and CXCL8) that can modulate the function of other innate and adaptive immune cells. NK cells in human are commonly identified as CD3⁻CD56⁺ cells. They are distributed throughout the blood, organs, and lymphoid tissue and make up around 15% of the peripheral blood lymphocytes. NK cells play a role in tumor surveillance and the rapid elimination of virus-infected cells. They do not require the missing "self" signal of MHC Class I and can recognize stressed cells in the absence of antibodies, allowing them to react much more quickly than the adaptive immune system. Natural killer (NK) cells play critical roles in host immunity against cancer. In response, cancers develop mechanisms to escape NK cell attack or induce defective NK cells (Cheng M et al. Cell Mol Immunol. 2013 May;10(3):230-52. doi: 10.1038/cmi.2013.10. Epub 2013 Apr 22.).

The present invention in particular relates to immunoregulatory T cells (which can also be referred to as immunomodulatory T cells) for use in the treatment and/or prevention of immune-related adverse events (irAE) in a subject that has received a checkpoint-inhibitor therapy, preferably as a cellular therapy.

In embodiments, generation or induction of immunoregulatory T cells in a sample can be measured by comparing the monocyte and T cell phenotype and population distribution in a sample before and after performing the method of the invention, for example by flow cytometry, gene expression and/or mass spectrometry analysis of protein abundance. For example, a shift from Arginase-1^{low} to Arginase-1^{high} monocytes after irradiation is indicative in of the induction of an immunoregulatory phenotype. Furthermore, a decrease in the expression of GM-CSF, IFN-y, TNF and/or IL-2 in the T cell population would also indicate induction of immunoregulatory T cells.

A skilled person is aware of suitable methods and protocols of identifying different monocyte and T cell populations within a sample. For example, in a blood-derived sample, the total monocytes and T cell population may be defined and identified, preferably by flow cytometry, as tolerogenic KLRG1⁺BTLA⁺CD47⁺TOX^{low} T cell population.

Further characteristics and definitions of immunoregulatory T cells are well established in the art and are subject of multiple research and review articles that are known or can be identified by a skilled person.

The term "immunomodulatory functions" relates to functions or properties of molecules or cells that induce a changes or modulation in the function, action or status of any component of the immune system.

Cellular therapies typically involve the administration of immune cells isolated from the blood of the patient. Cell types that can be used in this way are, without limitation, natural killer cells, lymphokine-activated killer cells, cytotoxic T cells, monocytes, macrophages, granulocytes and dendritic cells. Dendritic cell therapy provokes anti-tumor responses by causing dendritic cells to present tumor antigens. Dendritic cells present antigens to lymphocytes, which activates them, priming them to kill other cells that present the antigen.

### Cancer

In the context of the present invention, the term "cancer" relates to the treatment of all kinds of cancer, independent of whether the cancer is associated with the formation of a solid tumor or whether the cancer cells do not form a solid tumor, as it is the case for certain leukemias.

Cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

### Therapeutic application of the invention

### Pharmaceutical combination or combination medicine

According to the present invention, a "pharmaceutical combination" or "combination medicine" is the combined presence of adiponectin and/or adiponectin receptor agonist (ARA), and a photosensitizing agent and/or lymphoid cells obtained by ECP-treatment according to the invention, in proximity to one another. In one embodiment, the combination is suitable for combined administration.

In one embodiment, the pharmaceutical combination or combination medication as described herein is characterized in that adiponectin and/or adiponectin receptor agonist (ARA) is in a pharmaceutical composition in admixture with a pharmaceutically acceptable carrier, and the photosensitizing agent is in a separate pharmaceutical composition in admixture with a pharmaceutically acceptable carrier, and the blood sample or lymphoid cells are in a separate pharmaceutical composition in admixture with a pharmaceutically acceptable carrier. The combination medication or pharmaceutical combination of the present invention can therefore in some embodiments relate to the presence of two or three or even more separate compositions or dosage forms in proximity to each other. The agents in combination are not required to be present in a single composition.

### Combined administration

According to the present invention, the term "combined administration", otherwise known as coadministration or joint treatment, encompasses in some embodiments the administration of separate formulations of the compounds described herein, whereby treatment may occur within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g., orally by ingesting separate tablets simultaneously. A combination medicament, such as a single formulation comprising multiple agents disclosed herein, and optionally additional anti-cancer and/or immuno-suppressing (e.g., steroids) or immuno-modulating medicaments, may also be used in order to co-administer the various components in a single administration or dosage.

In the context of the present invention combined administration or combined use may comprise in preferred embodiments the administration of lymphocytes to the subject and/or the administration of adiponectin and/or adiponectin receptor agonist (ARA) to the subject and the ex vivo/extracorporeal administration of a photosensitizing agent to a sample/cells of the subject.

A combined therapy or combined administration of one agent may precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-96 hours of each other and, in embodiments preferably, within about 6-48 hours of each other. In some situations, it may be desirable to extend the time period for treatment significantly, however, where several days (2, 3, 4, 5, 6 or 7) to several weeks (1, 2, 3, 4, 5, 6, 7, 8, 9, 10, 11 or 12) lapse between the respective administrations.

In embodiments, the therapeutic agent or combination medication is administered from day 1, 2, 3, 4, 5, 6 or day 7 of the occurrence of irAE symptoms, or for 1, 2, 3, 4, 5, 6, 7, 8, 9, 10, 12, 14, 16, 18, 20, 22, 24, 26, 28, 30, 35, 40, 45 or 50 weeks, or from day 1 to at least 3 weeks, after occurrence of irAE symptoms. In embodiments, the therapeutic agent or combination medication is administered between 1 - 5 days before the administration of the ICB therapy, or for 1, 2, 3, 4, 5, 6 or 7 days or 1, 2, 3, 4, 5, 6, 7, 8, 9, 10, 12, 14, 16, 18, 20, 22, 24, 26, 28, 30, 35, 40, 45 or 50 weeks before the administration of the ICB therapy. In preferred embodiments, the therapeutic agent or combination medication is administered between 1-14 days, more preferable for 1-5 days before the administration of the ICB therapy.

In the meaning of the invention, any form of administration of the multiple agents described herein is encompassed by combined administration, such that a beneficial additional therapeutic effect, preferably a synergistic effect, is achieved through the combined administration of the two medications, and preferably two agents and ECP.

It is understood that substituents and substitution patterns of the compounds described herein can be selected by one of ordinary skill in the art to provide compounds that are chemically stable and that can be readily synthesized by techniques known in the art and further by the methods set forth in this disclosure.

Another aspect of the disclosure includes "pharmaceutical compositions" prepared for administration to a subject and which include a "therapeutically effective amount" of one or more of the compounds disclosed herein. In certain embodiments, the pharmaceutical compositions are useful for treating immune checkpoint blockade (ICB)-induced immune related adverse events (irAEs) in a subject. The therapeutically effective amount of a disclosed compound (e.g., agent, substance, blood sample or cells) will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The pharmaceutical compositions can be administered by intramuscular, intraocular, subcutaneous, intravenous, intra-arterial, intra-articular, intraperitoneal, intrathecal, intracerebroventricular, or parenteral routes. Optionally, compositions can be administered to subjects by a variety of mucosal administration modes, including by oral, rectal, intraocular, intranasal, intrapulmonary, or transdermal delivery, or by topical delivery to other surfaces.

The compositions according to the invention can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In accordance with the treatment methods according to the invention, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of and "administering a" compound should be understood to mean providing a compound, a prodrug of a compound, a combination of at least two compounds or a pharmaceutical composition as described herein. The compound(s) or composition(s) can be administered by another person to the subject (e.g., intravenously) or it can be self-administered by the subject (e.g., tablets).

Dosage can be varied by the attending clinician to maintain a desired concentration at a target site (for example, the lungs or systemic circulation). Higher or lower concentrations can be selected based on the mode of delivery, for example, intravenous or subcutaneous delivery, trans-epidermal, rectal, oral, pulmonary, or intranasal delivery. Dosage can also be adjusted based on the release rate of the administered formulation, for example, of an intrapulmonary spray versus powder, sustained release oral versus injected particulate or transdermal delivery formulations, and so forth.

The present invention also relates to a method of treatment of subjects suffering from the medical conditions disclosed herein. The method of treatment comprises preferably the administration of a therapeutically effective amount of a compound disclosed herein to a subject in need thereof.

A "therapeutically effective amount" refers to a quantity of a specified compound sufficient to achieve a desired effect in a subject being treated with said compound. For example, this may be the amount of a compound disclosed herein useful in treating a condition of immune checkpoint blockade (ICB)-induced immune related adverse events (irAEs) in a subject. The therapeutically effective amount or diagnostically effective amount of a compound will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects.

A non-limiting range for a therapeutically effective amount of a compound adiponectin or ARA and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight, or about 1*10^4 cells/kg body weight.

A non-limiting range for a therapeutically effective amount of tolerogenic T cells about 1*10^3 cells/kg body weight, about 5*10^3 cells/kg body weight, about 1*10^4 cells/kg body weight, about 5*10^4 cells/kg body weight, about 1*10^5 cells/kg body weight, about 5*10^5 cells/kg body weight, about 1*10^6 cells/kg body weight, about 5*10^6 cells/kg body weight, about 1*10^7 cells/kg body weight, about 5*10^7 cells/kg body weight, or about 1*10^8 cells/kg body weight or more.

A non-limiting range for a therapeutically effective amount of the photosensitizing agent, preferably 8-Methoxyproralen, is between 1-10 mg/ml sample, preferably about 2 mg/ml sample.

As used herein, the term "approximately" or "about" is used to describe and account for small variations. For example, the term may refer to less than or equal to 10, such as less than or equal down to 1, when appropriate, also the term may refer to more than or equal to 10, such as more than or equal up to 100 or more, when appropriate. It is to be understood that range format is used for the sake of simplicity and brevity and is to be flexibly understood to include numeric values expressly stated as boundaries of a range, encompassing each numeric value and sub-ranges.

The instant disclosure also includes kits, packages and multi-container units containing the herein described pharmaceutical compositions, active ingredients, and/or means for administering the same for use in the prevention and treatment of diseases and other conditions in mammalian subjects.

With the above context, the following consecutively numbered embodiments provide further specific aspects of the invention:
1. Adiponectin and/or Adiponectin Receptor Agonist (ARA) for use in the treatment or prevention of immune checkpoint blockade (ICB)-induced immune related adverse events (irAEs) in a subject.
2. Adiponectin and/or ARA for use according to embodiment 1, wherein the subject is receiving checkpoint blockade therapy.
3. Adiponectin and/or ARA for use according to any one of the preceding embodiments, wherein Adiponectin and/or ARA is administered at least 1 hour before, during and/or at least one hour after administration of checkpoint blockade therapy.
4. Adiponectin and/or ARA for use according to any one of the preceding embodiments,
   wherein the checkpoint blockade therapy comprises administration of at least one antibody, preferably at least two antibodies.
5. Adiponectin and/or ARA for use according to any one of the preceding embodiments,
   wherein the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).
6. Adiponectin and/or ARA for use according to any one of the preceding embodiments,
   wherein irAEs can involve any organ, preferably the gastrointestinal tract, lungs, endocrine glands, skin, and liver.
7. Adiponectin and/or ARA for use according to any one of the preceding embodiments,
   wherein the irAE comprise an ICB-induced colitis.
8. Adiponectin and/or ARA for use according to any one of the preceding embodiments,
   wherein the administration of Adiponectin and/or ARA reduces ICB-induced colitis.
9. Adiponectin and/or ARA for use according to any one of the preceding embodiments, wherein the administration of Adiponectin and/or ARA does not interfere with the anti-tumor response induced by the checkpoint blockade therapy, which is preferably an anti-PD1 treatment, due to the tissue specific adiponectin induction.
10. Combination medication for use in the treatment of immune checkpoint blockade (ICB)-induced immune related adverse events (irAEs) in a subject, comprising at least two of the following components:
   a. a photosensitizing agent, and
   b. Adiponectin and/or Adiponectin Receptor Agonist (ARA),
   and wherein a blood sample of said subject is subjected to ultraviolet A (UVA) irradiation extracorporeally, preferably to extracorporeal photopheresis (ECP) therapy
11. The combination medication for use according to embodiment 10, wherein the photosensitizing agent is a psoralen reagent, preferably 8-methoxypsoralen.
12. The combination medication for use according to embodiments 10 to 11, wherein the wavelength of the UVA irradiation is from wavelength of 3200 to 4000 angstroms.
13. The combination medication for use according to embodiments 11 to 12, wherein the subject suffers from cancer.
14. The combination medication for use according to embodiments 10 to 13, wherein the checkpoint blockade therapy comprises administration of at least one antibody, preferably at least two antibodies.
15. The combination medication for use according to embodiments 10 to 14, wherein the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).
16. The combination medication for use according to embodiments 10 to 15, wherein irAEs can involve any organ, preferably the gastrointestinal tract, lungs, endocrine glands, skin, and liver.
17. The combination medication for use according to embodiments 10 to 16, wherein the irAE comprises an ICB induced colitis.
18. The combination medication for use according to embodiments 10 to 17, wherein the said combination medication induces an increase of adiponectin, arginase-1 (Arg1) and tolerogenic KLRG1⁺BTLA⁺CD47⁺TOX^{low} T cells in inflamed intestines, but not in cancer tissue in said subject.
19. Adiponectin and/or Adiponectin Receptor Agonist (ARA) for use in the treatment of a tumor patient (or: subject suffering from cancer), wherein said patient has developed immune checkpoint blockade (ICB) induced immune related adverse events (irAEs), wherein said treatment comprises the combined administration of Adiponectin and/or ARA with an anti-tumor immunotherapy, wherein said immunotherapy comprises the administration of immunoregulatory T cells (or more general: blood samples).
20. Adiponectin and/or ARA for use in the treatment of a tumor patient according to the preceding embodiment, wherein the immunoregulatory T cells (or: blood samples)
   are obtained from a method comprising the steps of
   - providing a sample derived from an isolated blood sample of said patient,
   - adding a photosensitizing agent to the sample, and subjecting the sample to irradiation.
21. Adiponectin and/or ARA for use in the treatment of a tumor patient according to the embodiments 19 and 20 wherein the photosensitizing agent is 8-methoxypsoralen and/or the irradiation is UVA irradiation.
22. A method of treating or preventing immune checkpoint blockade (ICB)-induced immune related adverse events (irAEs) comprising administering Adiponectin and/or Adiponectin Receptor Agonist (ARA) in a subject, wherein the subject is receiving checkpoint blockade therapy.
23. The method of embodiment 22, wherein Adiponectin and/or ARA is administered at least one hour before, concurrently with, and/or at least one hour after administration of the checkpoint blockade therapy.
24. The method of embodiment 22 or 23, wherein the checkpoint blockade therapy comprises administration of at least one antibody.
25. The method of embodiment 24, wherein the at least one antibody is selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
26. The method of any one of embodiments 22 or 23, wherein the checkpoint blockade therapy comprises administration of two or more antibodies.
27. The method of embodiment 26, wherein at least one of the two or more antibodies is selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
28. The method of embodiment 26 or 27, wherein the two or more antibodies are selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
29. The method of any one of embodiments 22-28, wherein the irAEs involve in any organ.
30. The method of embodiment 29, wherein the organ is selected from the group consisting of gastrointestinal tract, lungs, endocrine glands, skin, and liver.
31. The method of any one of embodiments 22-30, wherein the irAEs comprise an ICB-induced colitis.
32. The method of any one of embodiments 22-31, wherein the administration of Adiponectin and/or ARA reduces ICB-induced colitis.
33. The method of any one of embodiments 22-32, wherein the administration of Adiponectin and/or ARA does not interfere with an anti-tumor response induced by the checkpoint blockade therapy due to the tissue specific adiponectin induction.
34. The method of any one of embodiments 22-33, wherein the checkpoint blockade therapy is an anti-PD1 treatment.
35. A method of treating immune checkpoint blockade (ICB)-induced immune related adverse events (irAEs) in a subject comprising administering components a and b to the subject:
   (a) a photosensitizing agent, and
   (b) Adiponectin and/or Adiponectin Receptor Agonist (ARA),
   and wherein a blood sample of the subject is subjected to ultraviolet A (UVA) irradiation extracorporeally.
36. The method of embodiment 35, wherein the ultraviolet A (UVA) irradiation is extracorporeal photopheresis (ECP) therapy.
37. The method of embodiment 35 or 36, wherein the photosensitizing agent is a psoralen reagent.
38. The method of embodiment 37, wherein the psoralen reagent is 8-methoxypsoralen.
39. The method of any one of embodiments 35-38, wherein a wavelength of the UVA irradiation is from wavelength of 3200 to 4000 angstroms.
40. The method of any one of embodiments 35-39, wherein the subject suffers from cancer.
41. The method of embodiment 40, wherein the cancer is malignant melanoma or breast cancer.
42. The method of any one of embodiments 35-41, wherein the subject is receiving checkpoint blockade therapy.
43. The method of embodiment 42, wherein the checkpoint blockade therapy comprises administration of at least one antibody.
44. The method of embodiment 43, wherein the at least one antibody is selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
45. The method of embodiment 42, wherein the checkpoint blockade therapy comprises administration of two or more antibodies.
46. The method of embodiment 45, wherein at least one of the two or more antibodies is selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
47. The method of embodiment 45 or 46, wherein the two or more antibodies are selected from the group consisting of cytotoxic T-lymphocyte antigen 4 (CTLA-4), programmed cell death-1 (PD-1), programmed cell death ligand-1 (PD-L1), and Lymphocyte-activation gene 3 (LAG-3).
48. The method of any one of embodiments 35-47, wherein the irAEs involve in any organ.
49. The method of embodiment 48, wherein the organ is selected from the group consisting of gastrointestinal tract, lungs, endocrine glands, skin, and liver.
50. The method of any one of embodiments 35-49, wherein the irAEs comprise an ICB-induced colitis.
51. The method of any one of embodiments 35-50, wherein the administration of Adiponectin and/or ARA reduces ICB-induced colitis.
52. The method of any one of embodiments 35-51, wherein the administration of Adiponectin and/or ARA does not interfere with an anti-tumor response induced by the checkpoint blockade therapy due to the tissue specific adiponectin induction.
53. The method of embodiment 51, wherein the checkpoint blockade therapy is an anti-PD1 treatment.
54. The method of any one of embodiments 35-53, wherein the treatment induces an increase of adiponectin, arginase-1 (Arg1) and tolerogenic KLRG1⁺BTLA⁺CD47⁺TOX^{low} T cells in inflamed intestines, but not in cancer tissue in said subject.
55. A method of treating a patient with cancer comprising administering a combination of Adiponectin and/or Adiponectin Receptor Agonist (ARA) with an anti-tumor immunotherapy, wherein the immunotherapy comprises administration of immunoregulatory T cells, and the patient has immune checkpoint blockade (ICB) induced immune related adverse events (irAEs).
56. The method of embodiment 55, wherein the immunoregulatory T cells are prepared by:
   (a) obtaining a sample derived from an isolated blood sample of the patient,
   (b) adding a photosensitizing agent to the sample, and
   (c) subjecting the sample to irradiation.
57. The method of embodiment 56, wherein the photosensitizing agent is 8-methoxypsoralen.
58. The method of embodiment 56 or 57, wherein the irradiation is ultraviolet A (UVA) irradiation.
59. The method of any one of embodiments 55-58, wherein the patient has a cancer that is malignant melanoma or breast cancer.

### FIGURES

The invention is further described by the following figures. These are not intended to limit the scope of the invention but represent preferred embodiments of aspects of the invention provided for greater illustration of the invention described herein.

### Brief description of the figures:

**Figure 1****:** ECP treatment reduces ICB-enhanced colitis in vivo by adiponectin induction.
**Figure 2****:** ECP treatment and ICB-colitis model.
**Figure 3****:** Adiponectin KO in donor or donor and recipient.
**Figure 4****:** ECP induces apoptosis in leukocytes.
**Figure 5****:** Treatment of murine splenocytes with ECP causes cell death in vivo.
**Figure 6****:** Adiponectin is induced by apoptotic leukocytes that accumulate in inflamed colon tissue.
**Figure** 7: ECP treated splenocytes are phagocytosed by antigen-presenting cells in vitro.
**Figure 8****:** scRNA seq based analysis of the colon reveals myeloid cell alterations upon ECP
**Figure 9****:** Adiponectin and ARA monotherapy as well as combination with ECP reduce intestinal inflammation.
**Figure 10****:** ECP leads to Arg1 upregulation and Arg1 inhibition reduces the protective ECP effects.
**Figure 11****:** Arg1 deficiency of the recipient reduces the protective ECP effects.
**Figure 12****:** ECP and adiponectin reduce pathogenic T cell activation.
**Figure 13****:** The effect of steroids and ECP on the anti-tumor immune responses.
**Figure 14****:** The protective ECP effect against irAE colitis allows for anti-tumor immune responses.
**Figure 15****:** Clinical activity of ECP in patients with irAE.

### Detailed description of the figures:

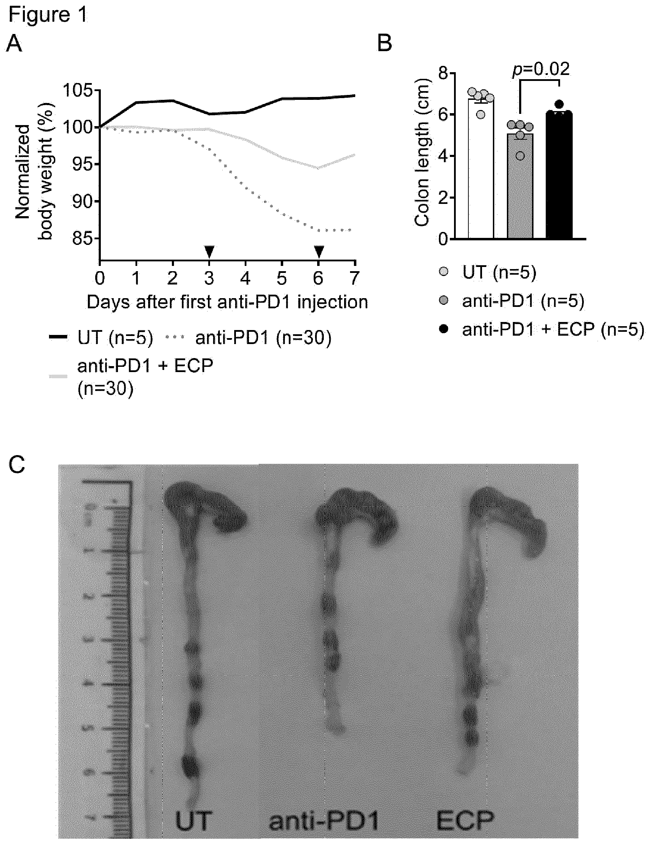

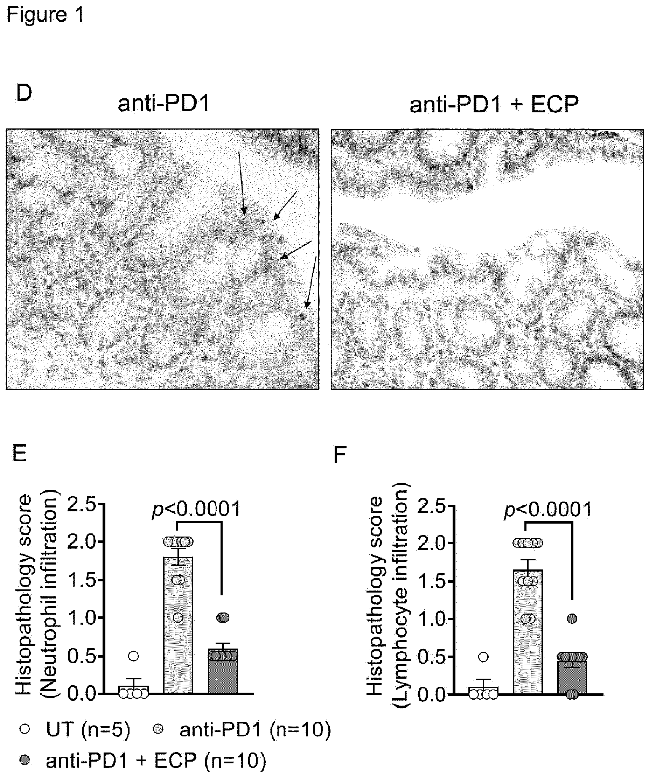

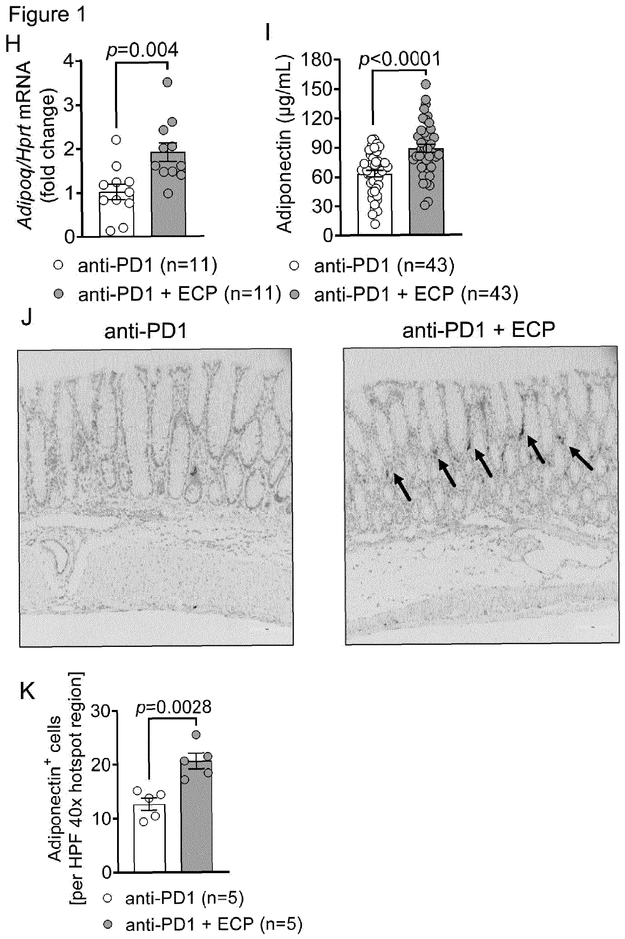

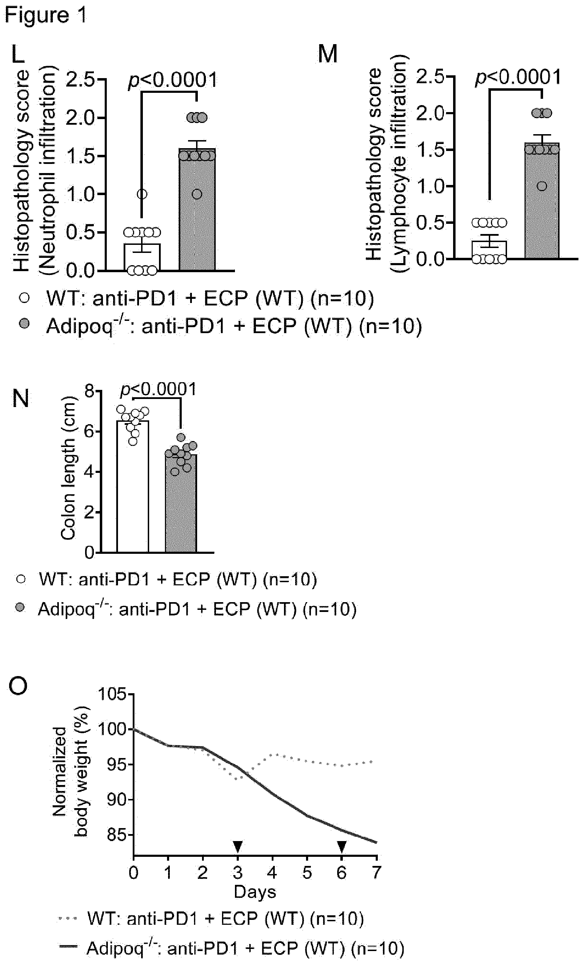

### Figure 1: ECP treatment reduces ICB-enhanced colitis in vivo.

**A)** The graph shows the body weights normalized to the first bodyweight on d0. Follow-up time starts at the first anti-PD1 injection (d0). Groups include untreated mice (UT), animals with DSS-induced colitis treated with anti-PD1 or anti-PD1 plus ECP. Each data point represents the mean of the pooled weight of all animals of the indicated group. The arrows indicate when ECP was performed (d3 and d6). Data were pooled from 2-5 experiments.

**B)** The scatter bar graph shows the median (+/- s.e.m.) of the colon length of untreated animals, mice with DSS/anti-PD1-induced colitis and mice with DSS/anti-PD1-induced colitis treated with ECP as indicated. The pooled values of one experiment are shown. P-values were calculated using an unpaired student t-test.

**C)** Representative colons isolated from mice of the indicated group are shown.

**D)** Representative histology images of colon samples from mice with DSS-induced colitis and anti-PD1 or anti-PD1 plus ECP treatment are shown. The arrows point towards neutrophils in the colon wall.

**E, F)** The scatter bar graphs show the median (+/- s.e.m.) of the pooled values of the histopathological grading of neutrophil **(e)** and lymphocyte **(f)** infiltration into the colon (0 = absent, 1 = mild, 2 = massive) for the indicated groups. Each data point represents the value of an individual mouse. The p-value was calculated using an unpaired student t-test.

**G)** Relative expression, as microarray heatmap ("Z-score intensity"), of RNA isolated from the colon of mice treated with DSS and anti-PD1 (n=4) or DSS, anti-PD1 plus ECP (n=5). Tile display shows the most significantly differentially regulated genes as determined from robust multichip average (RMA) signal values. The scale represents the level of gene expression. The grey arrow points towards the most upregulated gene in the ECP group (adiponectin).

**H)** The scatter bar graph shows median (+/- s.e.m.) of adiponectin RNA expression in the colon of mice that had DSS-induced colitis and were treated with anti-PD1 alone or anti-PD1 plus ECP. Gene expression data was normalized to Hprt expression and is depicted as fold change to anti-PD1 treatment. The experiment was performed 2 times. The p-value was calculated using an unpaired student t-test.

**I)** The scatter bar graph shows median (+/- s.e.m.) of adiponectin in serum samples of mice that had DSS-induced colitis and were treated with anti-PD1 alone or anti-PD1 plus ECP. The experiment was performed 2 times. Each data point represents an individual mouse. The p-value was calculated using an unpaired student t-test.

**J)** The scatter bar graph shows median (+/- s.e.m.) of adiponectin positive cells in the colon of mice that had DSS-induced colitis and were treated with anti-PD1 alone or anti-PD1 plus ECP. The p-value was calculated using an unpaired student t-test.

**K)** Representative adiponectin positive cells in the colon of mice that had DSS-induced colitis and were treated with anti-PD1 alone or anti-PD1 plus ECP. The arrows point towards adiponectin positive cells. The scale bar is represents 10 µm.

**L, M)** The scatter bar graphs show the median (+/- s.e.m.) of the pooled values of the histopathological grading of neutrophil **(L)** and lymphocyte **(M)** infiltration into the colon (0 = absent, 1 = mild, 2 = massive) for mice with DSS/anti-PD1-induced colitis treated with ECP as indicated. WT: wildtype recipient, *Adipoq*^{*-*/*-*}: adiponectin deficient recipient. Each data point represents the value of an individual mouse. The p-value was calculated using an unpaired student t-test.

**N)** The scatter bar graph shows the median (+/- s.e.m.) of the colon length of mice with DSS/anti-PD1-induced colitis treated with ECP as indicated. WT: wildtype recipient, *Adipoq*^{*-*/-}: adiponectin deficient recipient. The pooled values of one experiment are shown. *P*-values were calculated using an unpaired student t-test.

**O)** The graph shows the body weights normalized to the first bodyweight on d0. Time starts at the first anti-PD1 injection (d0). Groups include animals with DSS-induced colitis treated with anti-PD1 plus ECP, either WT: wildtype recipient or *Adipoq*^{*-*/*-*}: adiponectin deficient recipient. Each data points represents the mean of the pooled weight of all animals of the indicated group. The arrows indicate when ECP was performed (d3 and d6). Data were pooled from multiple experiments.

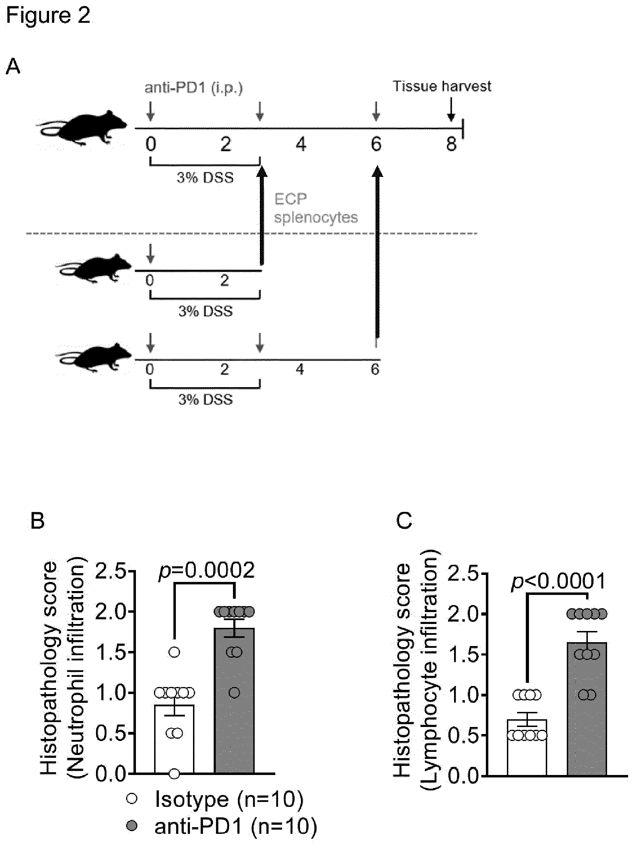

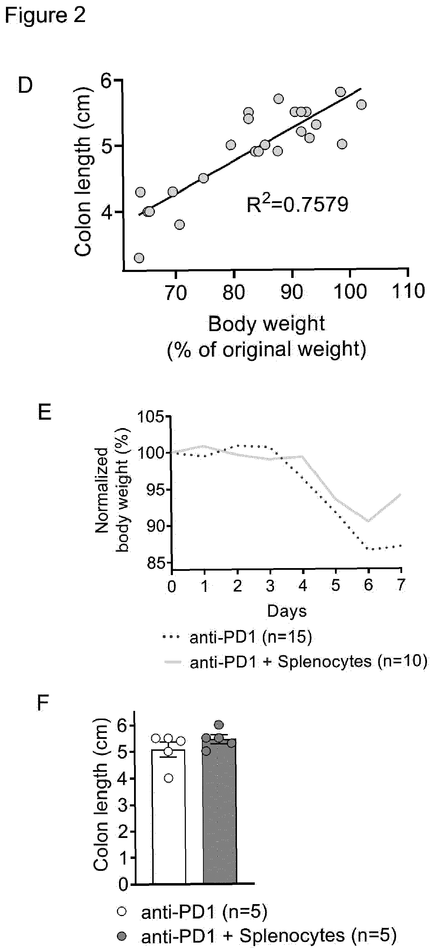

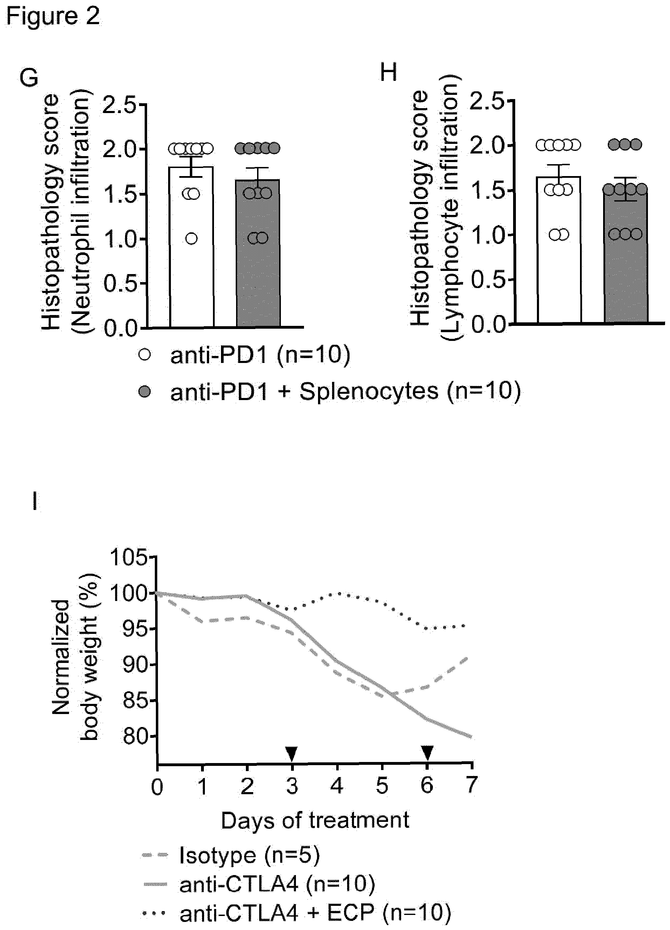

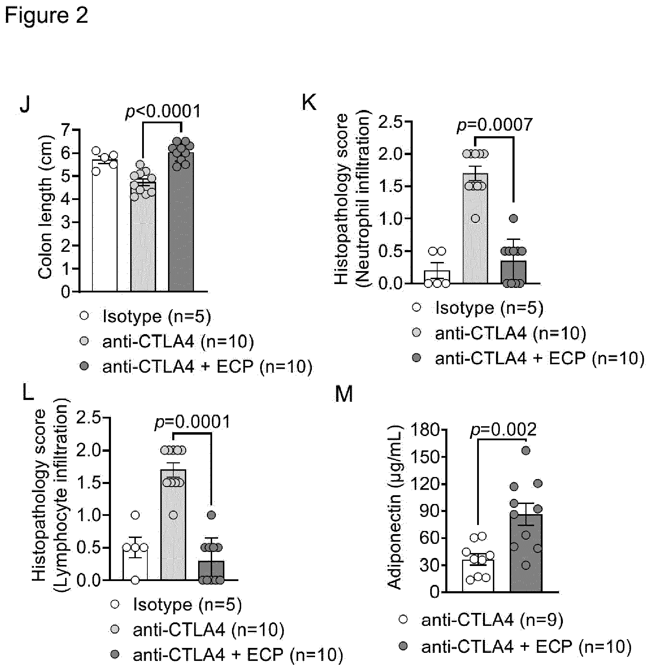

### Figure 2: ECP treatment and ICB-colitis model.

**A)** Experimental setup of DSS/anti-PD1-induced colitis with ECP treatment. Samples were harvested on day 8 after start of DSS treatment.

**B, C)** The scatter bar graphs show the median (+/- s.e.m.) of the pooled values of the histopathological grading of neutrophil **(B)** and lymphocyte **(C)** infiltration into the colon (0 = absent, 1 = mild, 2 = massive) for the indicated groups. Each data point represents the value of an individual mouse. The p-value was calculated using an unpaired student t-test.

**D)** The scatter plot shows the relative colon length (y-axis) and bodyweight in % to original body weight (x-axis). The pooled values of two experiments are shown. P-values were calculated using the D'Agostino-Pearson correlation.

**E)** The graph shows the body weights normalized to the first bodyweight on d0. Follow-up time starts at the first anti-PD1 injection (d0). Groups include animals with DSS-induced colitis treated with anti-PD1 alone or anti-PD1 plus untreated splenocytes. Each data point represents the mean of the pooled weight of all animals of the indicated group. Data were pooled from 2 experiments.

**F)** The scatter bar graph shows the median (+/- s.e.m.) of the colon length of mice with DSS/anti-PD1-induced colitis treated with ECP as indicated. The pooled values of one experiment are shown. P-values were calculated using an unpaired student t-test.

**G, H)** The scatter bar graphs show the median (+/- s.e.m.) of the pooled values of the histopathological grading of neutrophil **(G)** and lymphocyte **(H)** infiltration into the colon (0 = absent, 1 = mild, 2 = massive) for the indicated groups. Each data point represents the value of an individual mouse. The p-value was calculated using an unpaired student t-test.

**I)** The graph shows the body weights normalized to the first bodyweight on d0. Follow-up time starts at the first anti-CTLA-4 injection (d0). Groups include animals with DSS-induced colitis treated with anti- CTLA-4 alone or anti-CTLA-4 plus untreated splenocytes. Each data point represents the mean of the pooled weight of all animals of the indicated group. Data were pooled from 2 experiments.

**J)** The scatter bar graph shows the median (+/- s.e.m.) of the colon length of mice with DSS/anti-CTLA-4-induced colitis treated with ECP as indicated. The pooled values of one experiment are shown. P-values were calculated using an unpaired student t-test.

**K, L)** The scatter bar graphs show the median (+/- s.e.m.) of the pooled values of the histopathological grading of neutrophil **(K)** and lymphocyte **(L)** infiltration into the colon (0 = absent, 1 = mild, 2 = massive) for the indicated groups. Each data point represents the value of an individual mouse. The p-value was calculated using an unpaired student t-test.

**M)** The scatter bar graph shows median (+/- s.e.m.) of adiponectin in serum samples of mice that had DSS-induced colitis and were treated with anti-CTLA-4 alone or anti- CTLA-4 plus ECP. The experiment was performed 2 times. Each data point represents an individual mouse. The p-value was calculated using an unpaired student t-test.

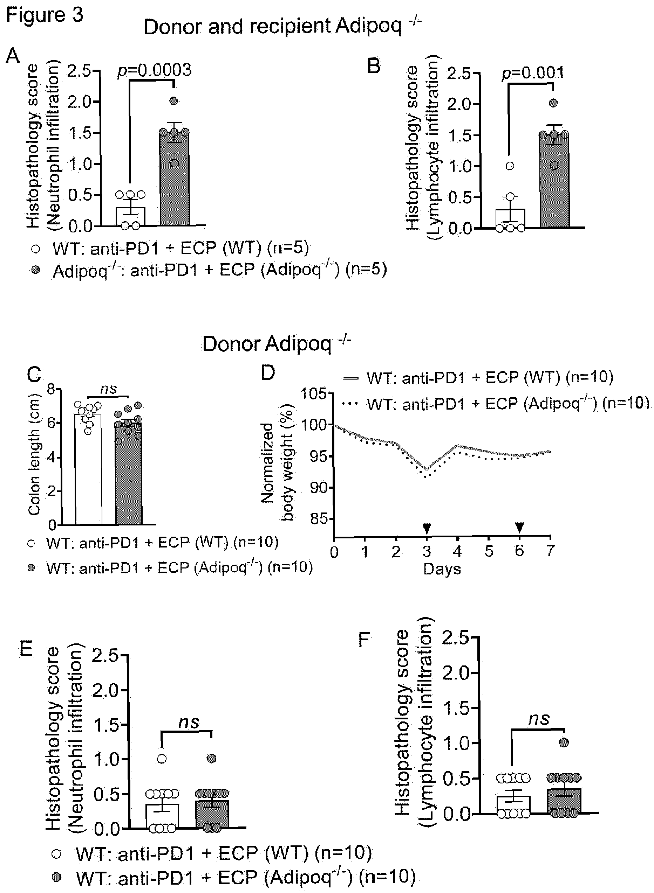

### Figure 3: Adiponectin KO in donor or donor and recipient

**A, B)** The scatter bar graphs show the median (+/- s.e.m.) of the pooled values of the histopathological grading of neutrophil **(A)** and lymphocyte **(B)** infiltration into the colon (0 = absent, 1 = mild, 2 = massive) for mice with DSS/anti-PD1-induced colitis treated with ECP as indicated. WT: wildtype recipient, *Adipoq*^{*-*/*-*}: adiponectin deficient recipient and donor. Each data point represents the value of an individual mouse. The p-value was calculated using an unpaired student t-test.

**C)** The scatter bar graph shows the median (+/- s.e.m.) of the colon length of mice with DSS/anti-PD1-induced colitis treated with ECP as indicated. WT: wildtype donor and recipient, *Adipoq*^{*-*/*-*}: adiponectin deficient donor. The pooled values of one experiment are shown. P-values were calculated using an unpaired student t-test.

**D)** The graph shows the body weights normalized to the first bodyweight on d0. Time starts at the first anti-PD1 injection (d0). Groups include animals with DSS-induced colitis treated with anti-PD1 plus ECP, either WT: wildtype recipient and donor or *Adipoq*^{*-*/*-*}: adiponectin deficient donor. Each data points represents the mean of the pooled weight of all animals of the indicated group. The arrows indicate when ECP was performed (d3 and d6). Data were pooled from multiple experiments.

**E, F)** The scatter bar graphs show the median (+/- s.e.m.) of the pooled values of the histopathological grading of neutrophil **(E)** and lymphocyte **(F)** infiltration into the colon (0 = absent, 1 = mild, 2 = massive) for mice with DSS/anti-PD1-induced colitis treated with ECP as indicated. WT: wildtype recipient, *Adipoq*^{*-*/*-*}: adiponectin deficient recipient. Each data point represents the value of an individual mouse. The p-value was calculated using an unpaired student t-test.

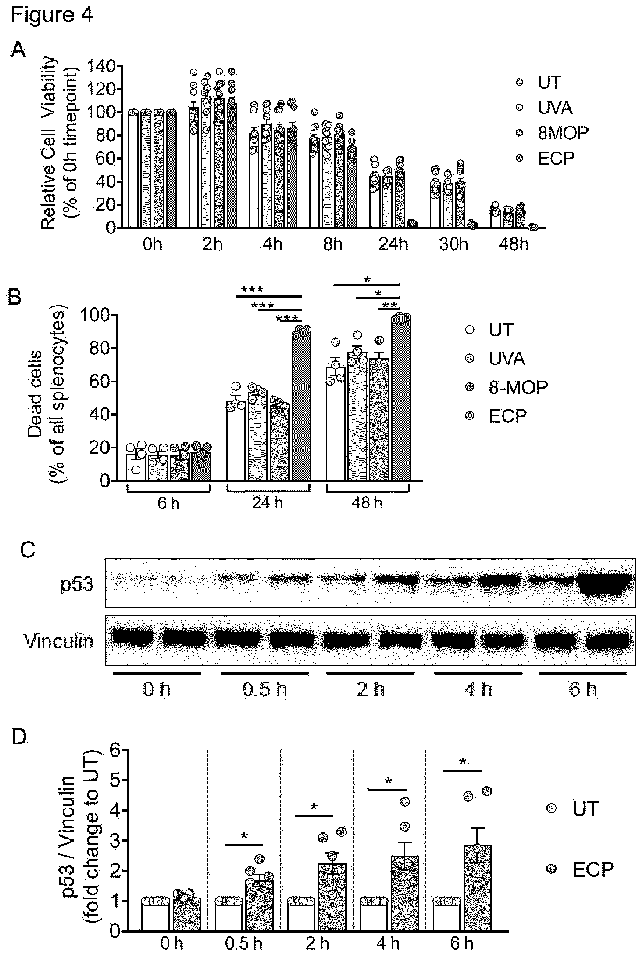

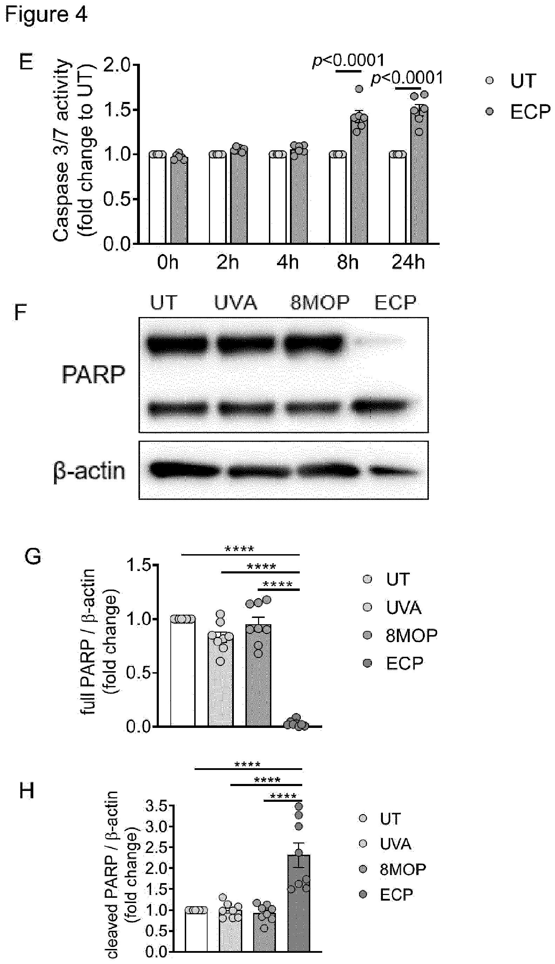

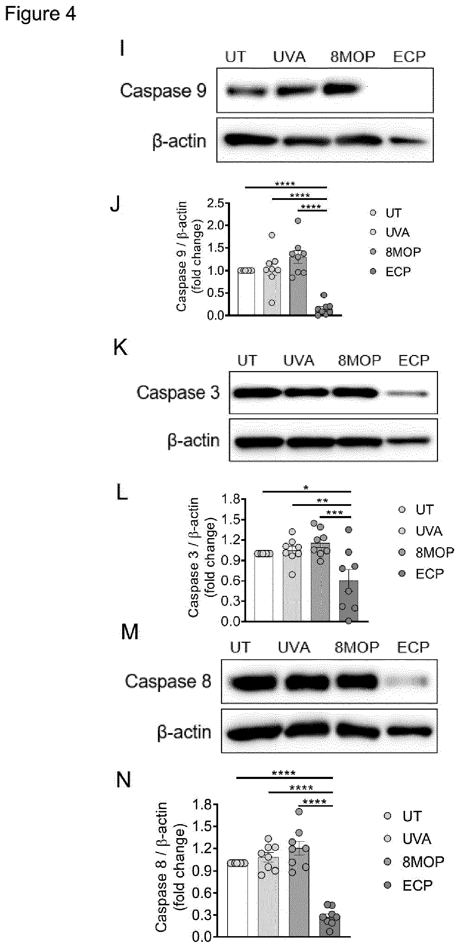

### Figure 4: ECP induces apoptosis in leukocytes.

**A)** The scatter bar graph shows the relative cell viability (% of 0h time point) of murine splenocytes that were left untreated (UT) or treated with UVA or 8-MOP or ECP, as indicated. The viability of the cells was monitored using a luminescence-based viability assay. ECP treatment reduced the viability of splenocytes *in vitro* after 24 h compared to all other conditions. The experiment was performed twice. P-values were calculated using a One-way ANOVA.

**B)** The scatter bar graph shows the percentage of dead cells (% of all splenocytes) within murine splenocytes that were left untreated (UT) or treated with UVA or 8-MOP or ECP, as indicated. Cell death was investigated by FACS analysis. P-values were calculated using a One-way ANOVA.

**C)** Representative western blot image for p53 and vinculin protein in murine splenocytes that were untreated or underwent ECP treatment.

**D)** The scatter bar graph shows the p53 / Vinculin ration (fold change to UT) based on western blot for p53 and vinculin derived from murine splenocytes that were untreated or underwent ECP treatment. Each data point represents the value of an individual mouse. The p-value was calculated using an unpaired student t-test

**E)** The scatter bar graph shows Caspase 3/7 activity (fold change to UT) in murine splenocytes that were treated with ECP or left untreated. The activity of caspase 3 and 7 was measured using a luminescence-based assay. Each data point represents the value of an individual mouse. The p-value was calculated using an unpaired student t-test.

**F-N)** Murine splenocytes were left untreated, treated with UVA or 8MOP alone or with the combination (ECP). Complete protein was isolated 24 h after treatment application. Protein analysis confirmed increased cell death after ECP treatment compared to other treatment through enhanced PARP cleavage, and increased caspase 9, caspase 3 and caspase 8 cleavage. Representative western blot images for PARP, caspase-9, -3, -8 and β-actin protein derived from murine splenocytes that were untreated or underwent ECP treatment as indicated. The scatter bar graphs show the ratio of PARP, caspase-9, -3, -8 relative to β-actin protein derived from murine splenocytes that were untreated or underwent ECP treatment as indicated. P-values were calculated using a One-way ANOVA.

Each data point represents the value of an individual mouse. P-values were calculated using a One-way ANOVA.

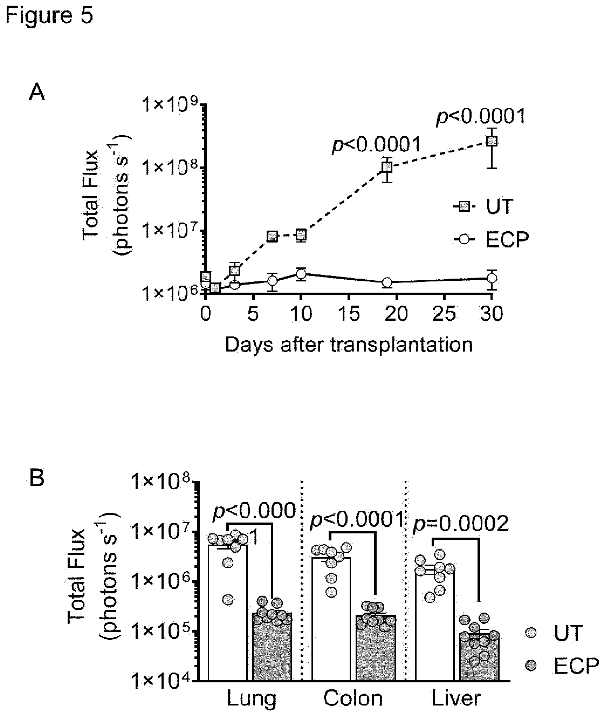

### Figure 5: Treatment of murine splenocytes with ECP causes cell death in vivo.

**A)** The diagram shows the photon flux quantification at multiple time points after transfer of luc transgenic splenocytes (C57BL/6 luc donor mice) in Rag2^{-/-}γc^{-/-} recipients. The splenocytes were either untreated or ECP treated as indicated. Before the transfer the splenocyte donor C57BL/6 luc mice were transplanted with B16.F10 melanoma and treated with anti-PD1 or isotype control. On day 9, splenocytes were harvested and either treated with ECP or left untreated and transplanted in Rag2^{-/-}γc^{-/-} recipients.
**B)** The scatter bar graph shows the ex vivo BLI signal of different organs on day 30 after the transplantation of ECP treated or untreated luc-transgenic splenocytes. The organs were incubated with luciferin solution and imaged ex vivo to analyze the infiltration of transplanted splenocytes in target organs. BLI analysis shows that the signal is significantly higher in organs isolated from mice transplanted with untreated splenocytes compared to those transplanted with ECP-treated splenocytes. Each data point represents the value of an individual mouse. P-values were calculated using a One-way ANOVA.

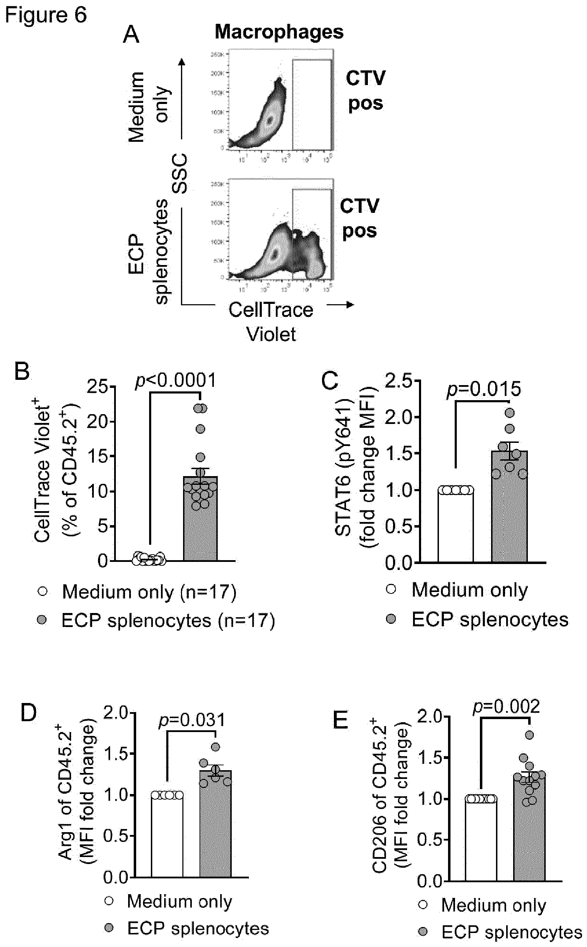

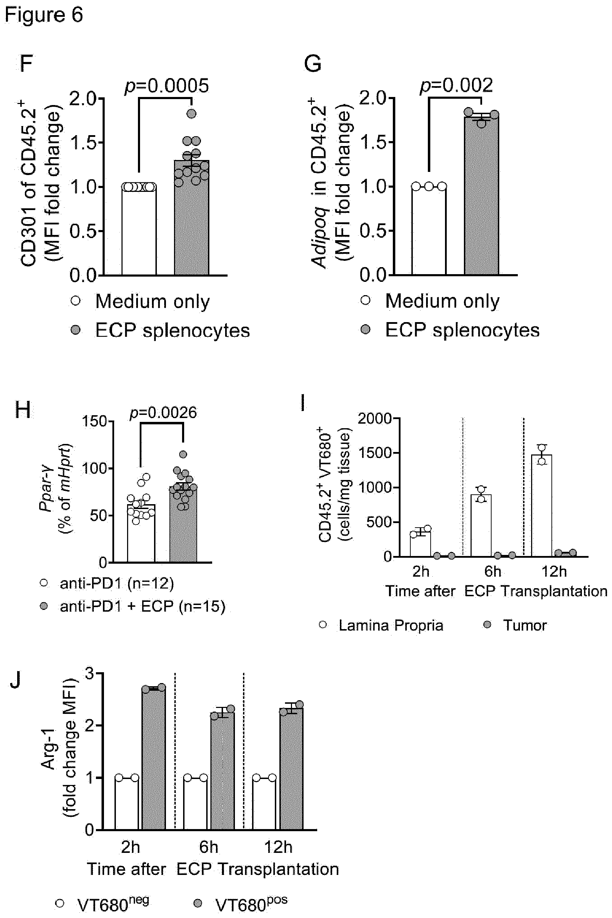

### Figure 6: Adiponectin is induced by apoptotic leukocytes that accumulate in inflamed colon tissue

**A)** The representative flow-cytometry plots show cell trace violet in CD45.2⁺ macrophages. ECP treated cell Trace Violet (CTV) stained CD45.1⁺ splenocytes were exposed to CD45.2⁺ bone marrow-derived macrophages for 24h of co-culture.
**B)** Quantification of cell trace violet positive CD45.2⁺ macrophages. ECP treated cell Trace Violet (CTV) stained CD45.1⁺ splenocytes were exposed to CD45.2⁺ bone marrow-derived macrophages for 24h of co-culture. The p-value was calculated using an unpaired student t-test.
**C)** The scatter bar graph shows phosphorylated STAT6 (fold change of MFI) in CD45.2⁺ macrophages. ECP treated CD45.1⁺ splenocytes were exposed to CD45.2⁺ bone marrow-derived macrophages for 24h of co-culture. The p-value was calculated using an unpaired student t-test.
**D)** The scatter bar graph shows arginase-1 (fold change of MFI) in CD45.2⁺ macrophages. ECP treated CD45.1⁺ splenocytes were exposed to CD45.2⁺ bone marrow-derived macrophages for24h of co-culture. The p-value was calculated using an unpaired student t-test.
**E)** The scatter bar graph shows CD206 (fold change of MFI) in CD45.2⁺ macrophages. ECP treated CD45.1⁺ splenocytes were exposed to CD45.2⁺ bone marrow-derived macrophages for 24h of co-culture. The p-value was calculated using an unpaired student t-test.
**F)** The scatter bar graph shows CD301 (fold change of MFI) in CD45.2⁺ macrophages. ECP treated CD45.1⁺ splenocytes were exposed to CD45.2⁺ bone marrow-derived macrophages for 24h of co-culture. The p-value was calculated using an unpaired student t-test.
**G)** The scatter bar graph shows adiponectin (fold change of MFI) in CD45.2⁺ macrophages. ECP treated CD45.1⁺ splenocytes were exposed to CD45.2⁺ bone marrow-derived macrophages for24h of co-culture. The p-value was calculated using an unpaired student t-test.
**H)** The scatter bar graph shows Ppar-γ RNA (% of mHprt) RNA expression in the colon of mice that had DSS-induced colitis and were treated with anti-PD1 alone or anti-PD1 plus ECP. Gene expression data was normalized to Hprt expression and is depicted as fold change to anti-PD1 treatment. The experiment was performed 2 times. The p-value was calculated using an unpaired student t-test.
**I)** The scatter bar graph shows CD45.2⁺VT680⁺ cells in the lamina propria of the colon or in the subcutaneous melanoma tissue isolated at the indicated time points after injection from mice with anti-PD1 induced colitis. At time point 0 hours the mice had received CD45.1⁺VT680⁺ splenocytes that were ECP treated. The experiment was performed 2 times. The p-value was calculated using an unpaired student t-test.
**J)** The scatter bar graph shows arginase-1 (fold change of MFI) in VT680⁺ cells in the lamina propria of the colon isolated at the indicated time points after injection from mice with anti-PD1 induced colitis. At time point 0 hours the mice had received CD45.1⁺VT680⁺ splenocytes that were ECP treated. The experiment was performed 2 times. The p-value was calculated using an unpaired student t-test.

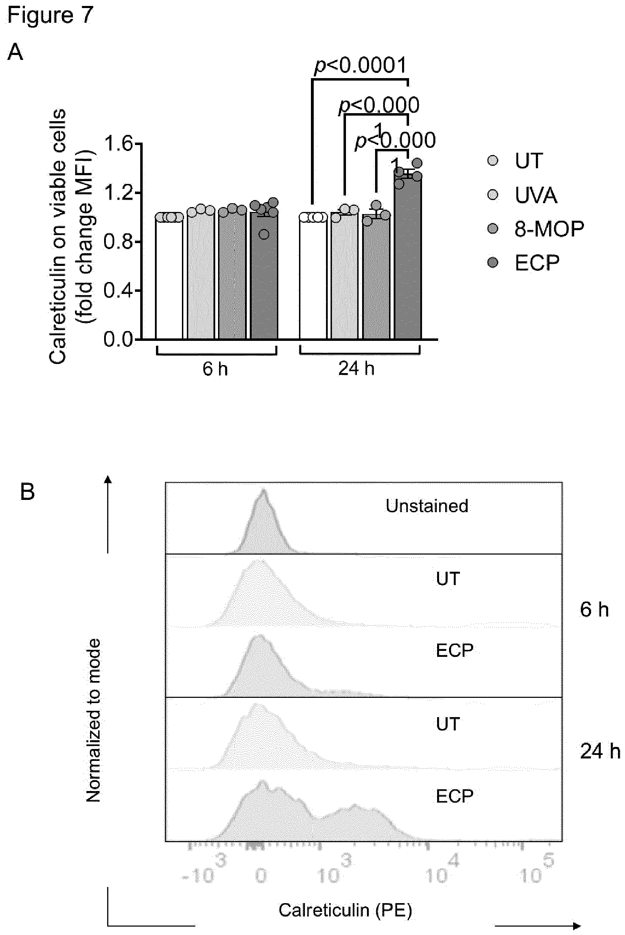

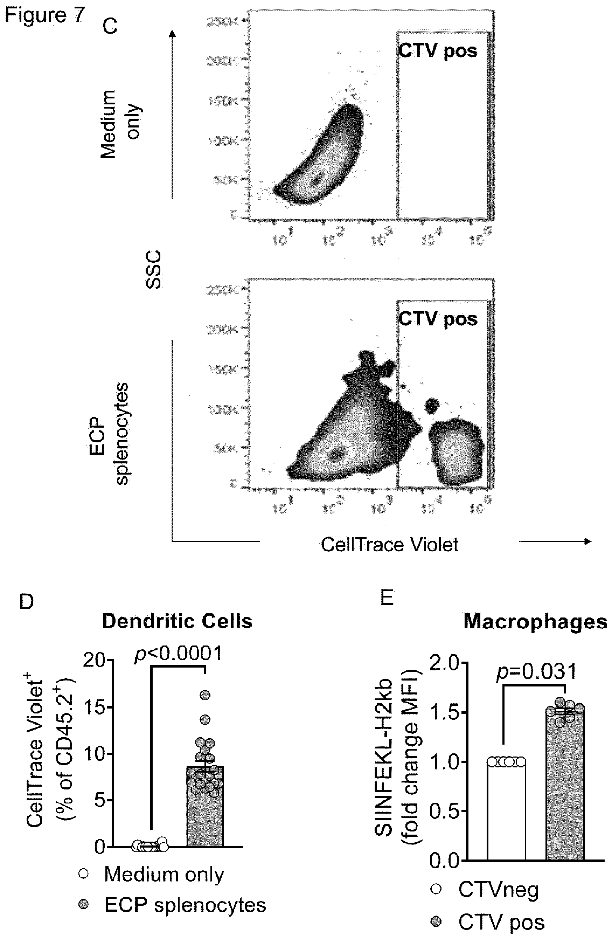

### Figure 7: ECP treated splenocytes are phagocytosed by antigen-presenting cells in vitro.

**A)** The scatter bar graph shows calreticulin protein expression on viable cells (fold change MFI) of splenocytes that were untreated (UT), or exposed to UVA, 8-MOP or ECP. Each data point represents the value of an individual mouse. P-values were calculated using a One-way ANOVA. **B)** Representative histogram for calreticulin on the surface of splenocytes that were untreated (UT), or exposed to UVA, 8-MOP or ECP.

**C)** Representative histogram for CTV in in CD45.2⁺ CD11c⁺ cells. CD45.1⁺ splenocytes were loaded with the SIINFEKL ovalbumin peptide, treated with ECP, stained with cell trace violet (CTV) and co-cultured with CD45.2⁺ CD11c⁺ BM derived dendritic cells (BMDCs) (C) or CD45.2⁺ F4/80⁺ BM derived macrophages (BMDMs)

**D, E)** The scatter bar graph shows SIINFEKL MFI fold change in CD45.2⁺ CD11c⁺ cells. CD45.1⁺ splenocytes were loaded with the SIINFEKL ovalbumin peptide, treated with ECP, stained with cell trace violet (CTV) and co-cultured with CD45.2⁺ CD11c⁺ BM derived dendritic cells (BMDCs) (D) or CD45.2⁺ F4/80⁺ BM derived macrophages (BMDMs) (E) for 24h. The BMDCs and BMDMs that did phagocytose the ECP treated splenocytes and became CTV positive presented the SIINFEKL ovalbumin peptide on their surface. The presentation of the SIINFEKL ovalbumin peptide is detected on CD45.2⁺ BMDCs and BMDMs after 24h. Each data point represents the value of an individual mouse. P-values were calculated using a One-way ANOVA.

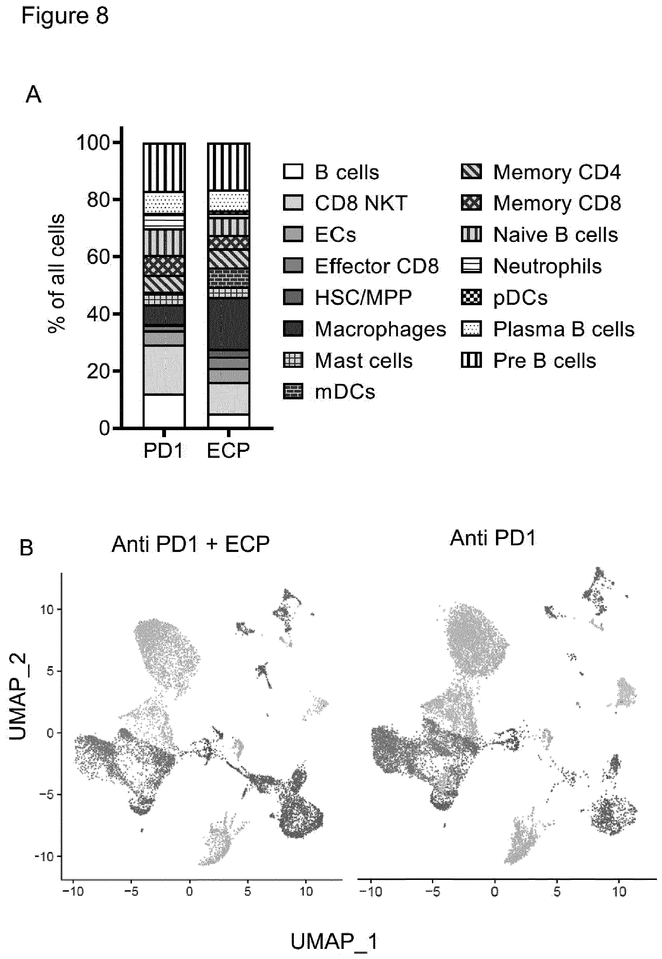

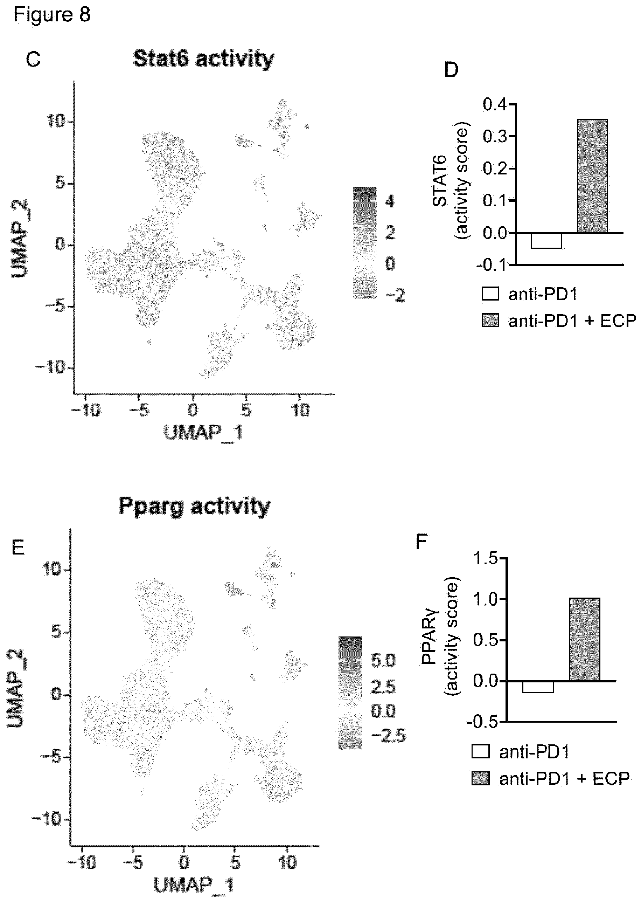

### Figure 8: scRNA seq based analysis of the colon reveals myeloid cell alterations upon ECP

**A)** The bar diagram represents the frequency of intestinal immune cells based on their expression profile. Color-coding is based on cluster assignment by the Seurat v4 algorithm. Cells were isolated from the colon of mice that had received DSS/anti-PD1 treatment alone or combined with ECP.
**B)** UMAP visualization of cells isolated from the colon of mice that had received DSS/anti-PD1 treatment alone or combined with ECP. Color-coding is based on cluster assignment by the Seurat v4 algorithm. The different clusters were determined by marker gene expression.
**C)** UMAP visualization of STAT6 activity in cells isolated from the colon of mice that had received DSS/anti-PD1 treatment alone or combined with ECP. Color-coding is based on STAT6 pathway activity. The different clusters were determined by marker gene expression.
**D)** The bar diagram indicates STAT6 activity score in cells isolated from the colon of mice that had received DSS/anti-PD1 treatment alone or combined with ECP.
**E)** UMAP visualization of PPARγ activity in cells isolated from the colon of mice that had received DSS/anti-PD1 treatment alone or combined with ECP. Color-coding is based on PPARγ pathway activity. The different clusters were determined by marker gene expression.
**F)** The bar diagram indicates PPARγ activity score in cells isolated from the colon of mice that had received DSS/anti-PD1 treatment alone or combined with ECP.

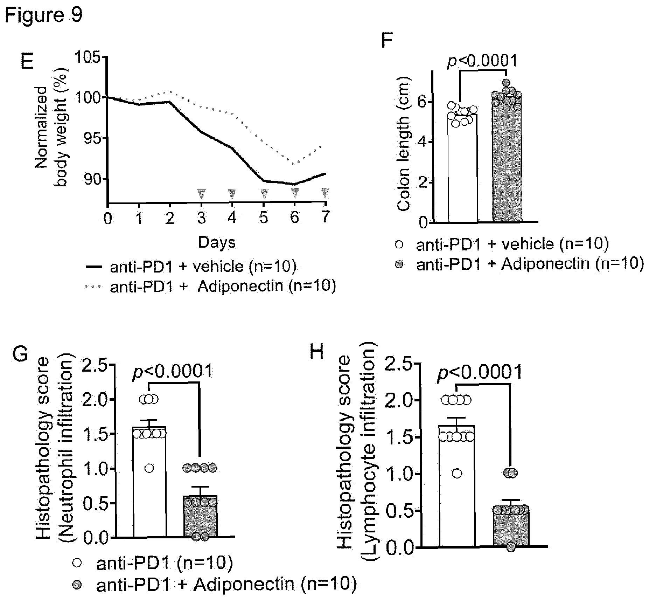

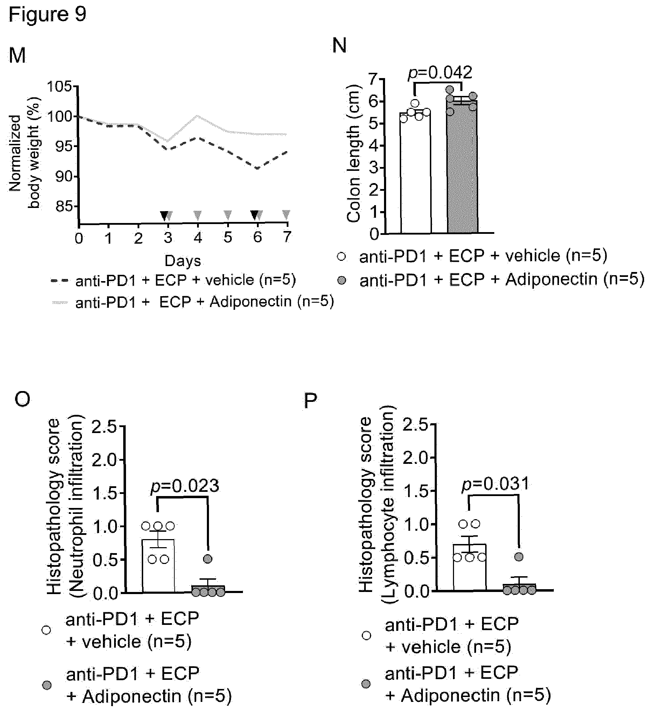

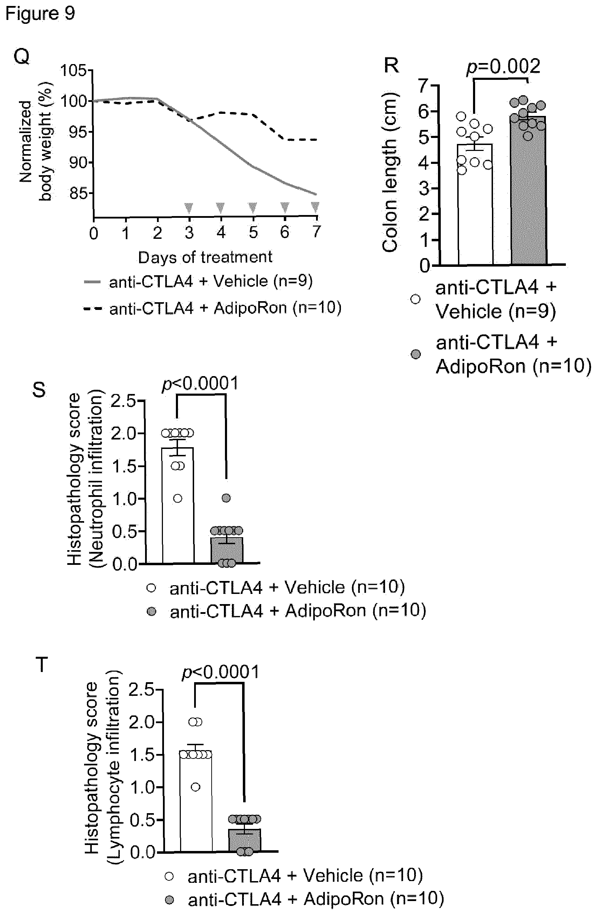

### Figure 9: Adiponectin and ARA monotherapy as well as combination with ECP reduce intestinal inflammation

**A)** The graph shows the body weights normalized to the first bodyweight on d0. Follow-up time starts at the first anti-PD1 injection (d0). Groups include animals with DSS-induced colitis treated with anti-PD1 plus vehicle or anti-PD1 plus Adiponectin receptor agonist (Adipo R Agon). Each data point represents the mean of the pooled weight of all animals of the indicated group. The arrows indicate when treatment with vehicle or Adipo R Agon was performed. Data were pooled from multiple experiments.

**B)** The scatter bar graph shows the median (+/- s.e.m.) of the colon length of mice with DSS/anti-PD1-induced colitis treated with vehicle or Adipo R Agon as indicated. The pooled values of two experiments are shown. P-values were calculated using an unpaired student t-test.

**C, D)** The scatter bar graphs show the median (+/- s.e.m.) of the pooled values of the histopathological grading of neutrophil (C) and lymphocyte (D) infiltration into the colon (0 = absent, 1 = mild, 2 = massive) of mice with DSS/anti-PD1-induced colitis treated with vehicle or Adipo R Agon as indicated. Each data point represents the value of an individual mouse. The p-value was calculated using an unpaired student t-test.

**E)** The graph shows the body weights normalized to the first bodyweight on d0. Follow-up time starts at the first anti-PD1 injection (d0). Groups include animals with DSS-induced colitis treated with anti-PD1 plus vehicle or anti-PD1 plus Adiponectin. Each data point represents the mean of the pooled weight of all animals of the indicated group. The arrows indicate when treatment with vehicle or Adiponectin was performed. Data were pooled from multiple experiments.

**F)** The scatter bar graph shows the median (+/- s.e.m.) of the colon length of mice with DSS/anti-PD1-induced colitis treated with vehicle or Adiponectin as indicated. The pooled values of two experiments are shown. P-values were calculated using an unpaired student t-test.

**G, H)** The scatter bar graphs show the median (+/- s.e.m.) of the pooled values of the histopathological grading of neutrophil **(G)** and lymphocyte **(H)** infiltration into the colon (0 = absent, 1 = mild, 2 = massive) of mice with DSS/anti-PD1-induced colitis treated with vehicle or Adiponectin as indicated. Each data point represents the value of an individual mouse. The p-value was calculated using an unpaired student t-test.

**I)** The graph shows the body weights normalized to the first bodyweight on d0. Follow-up time starts at the first anti-PD1 injection (d0). Groups include animals with DSS-induced colitis treated with anti-PD1 plus ECP and vehicle or anti-PD1 plus ECP and Adipo R Agon. Each data point represents the mean of the pooled weight of all animals of the indicated group. The arrows indicate when treatment with vehicle or Adipo R Agon (grey arrow) or ECP (black arrow) was performed. Data were pooled from 2 experiments.

**J)** The scatter bar graph shows the median (+/- s.e.m.) of the colon length of mice. Groups include animals with DSS-induced colitis treated with anti-PD1 plus ECP and vehicle or anti-PD1 plus ECP and Adipo R Agon as indicated. The pooled values of two experiments are shown. P-values were calculated using an unpaired student t-test.

**K, L)** The scatter bar graphs show the median (+/- s.e.m.) of the pooled values of the histopathological grading of neutrophil **(K)** and lymphocyte **(L)** infiltration into the colon (0 = absent, 1 = mild, 2 = massive) of mice. Groups include animals with DSS-induced colitis treated with anti-PD1 plus ECP and vehicle or anti-PD1 plus ECP and Adipo R Agon. Each data point represents the value of an individual mouse. The p-value was calculated using an unpaired student t-test.

**M)** The graph shows the body weights normalized to the first bodyweight on d0. Follow-up time starts at the first anti-PD1 injection (d0). Groups include animals with DSS-induced colitis treated with anti-PD1 plus ECP and vehicle or anti-PD1 plus ECP and adiponectin. Each data point represents the mean of the pooled weight of all animals of the indicated group. The arrows indicate when treatment with vehicle or adiponectin (grey arrow) or ECP (black arrow) was performed. Data were pooled from 2 experiments.

**N)** The scatter bar graph shows the median (+/- s.e.m.) of the colon length of mice. Groups include animals with DSS-induced colitis treated with anti-PD1 plus ECP and vehicle or anti-PD1 plus ECP and adiponectin as indicated. The pooled values of two experiments are shown. P-values were calculated using an unpaired student t-test.

**O, P)** The scatter bar graphs show the median (+/- s.e.m.) of the pooled values of the histopathological grading of neutrophil **(O)** and lymphocyte **(P)** infiltration into the colon (0 = absent, 1 = mild, 2 = massive) of mice. Groups include animals with DSS-induced colitis treated with anti-PD1 plus ECP and vehicle or anti-PD1 plus ECP and adiponectin. Each data point represents the value of an individual mouse. The p-value was calculated using an unpaired student t-test.

**Q)** The graph shows the body weights normalized to the first bodyweight on d0. Follow-up time starts at the first anti-CTLA-4 injection (d0). Groups include untreated mice (UT), animals with DSS-induced colitis treated with anti-CTLA-4 or anti-CTLA-4 plus AdipoRon. Each data point represents the mean of the pooled weight of all animals of the indicated group. The arrows indicate when AdipoRon treatment was performed. The values of one experiment are shown. **R)** The scatter bar graph shows the median (+/- s.e.m.) of the colon length of untreated animals, mice with DSS/anti-CTLA-4-induced colitis and mice with DSS/anti-CTLA-4-induced colitis treated with AdipoRon as indicated. The values of one experiment are shown. P-values were calculated using an unpaired student t-test.

**S, T)** The scatter bar graphs show the median (+/- s.e.m.) of the pooled values of the histopathological grading of neutrophil **(S)** and lymphocyte **(T)** infiltration into the colon (0 = absent, 1 = mild, 2 = massive) for the indicated groups. Each data point represents the value of an individual mouse. The p-value was calculated using an unpaired student t-test.

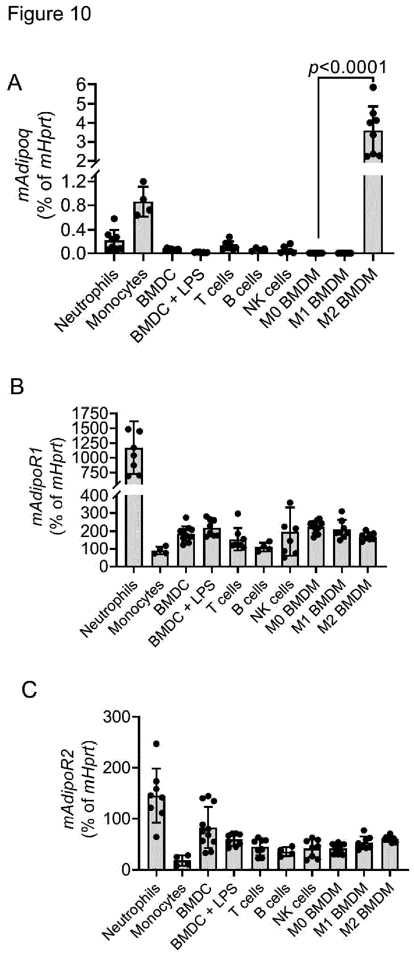

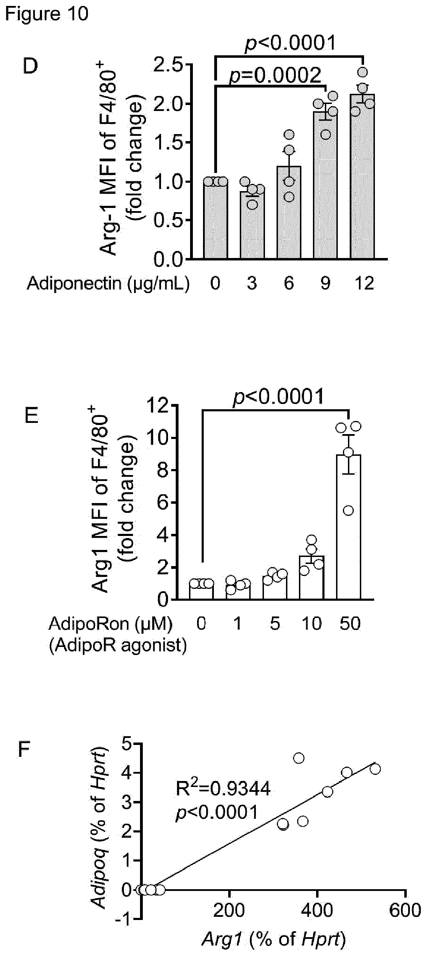

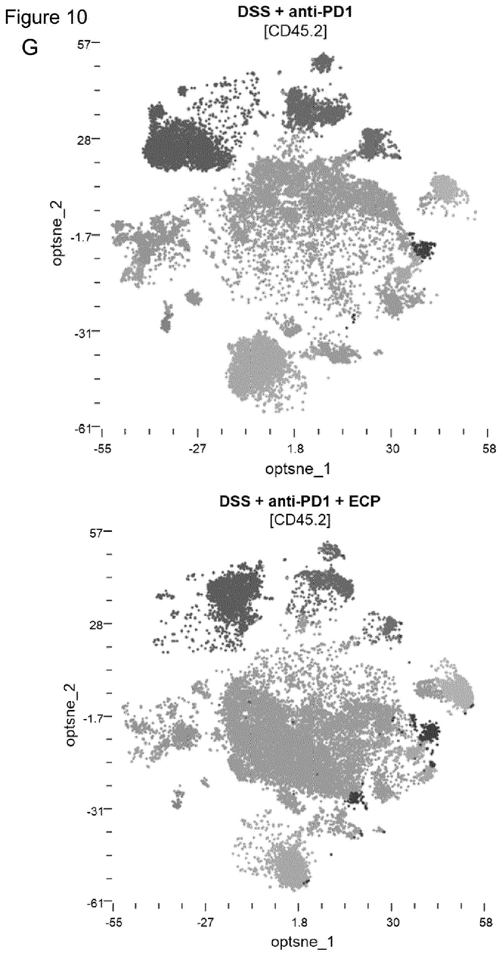

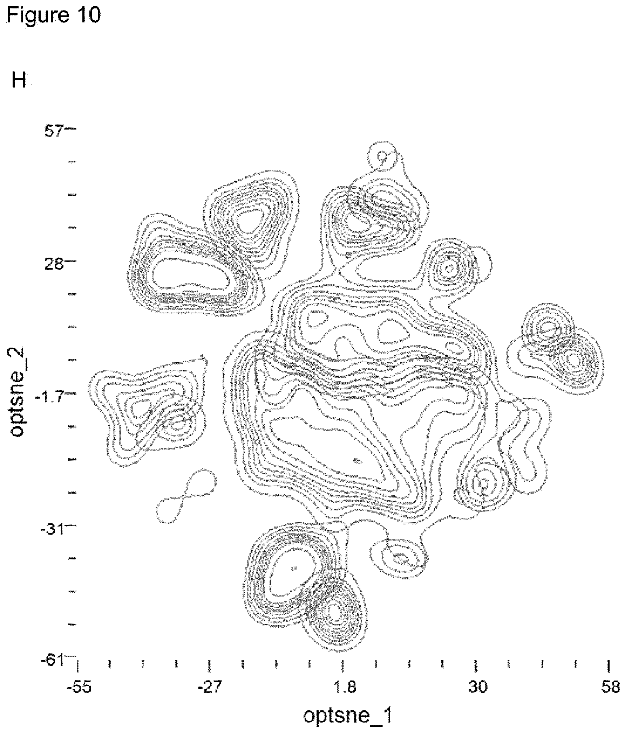

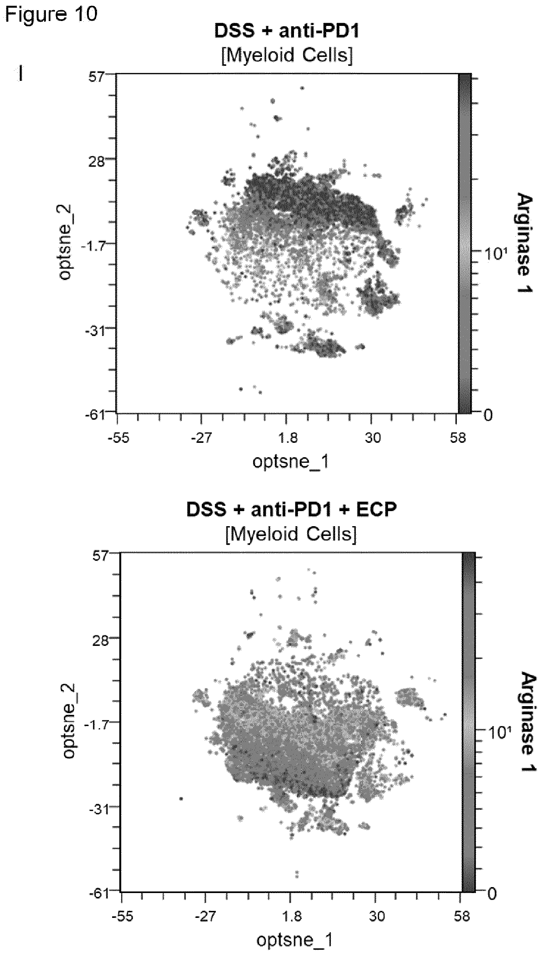

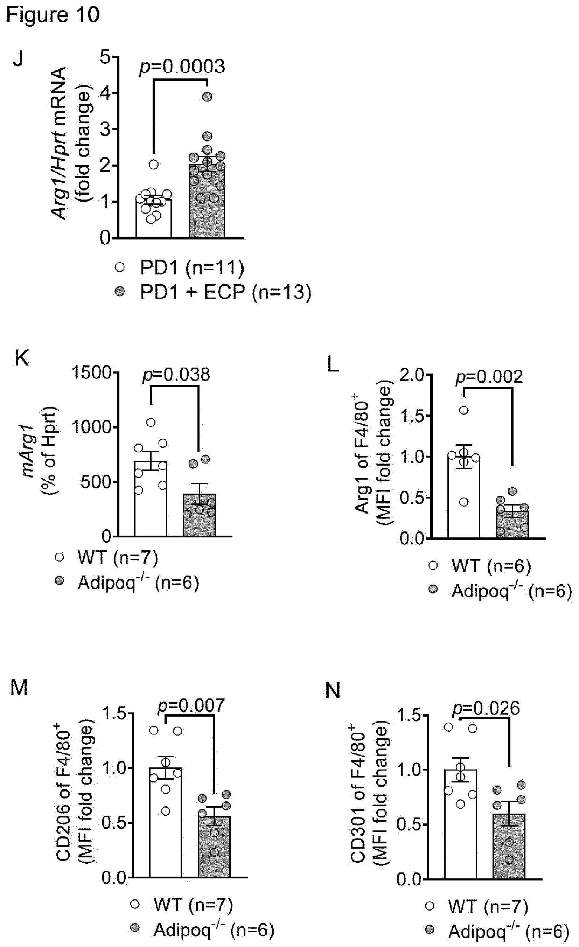

### Figure 10: ECP leads to Arg1 upregulation and Arg1 inhibition reduces the protective ECP effects

**A)** The scatter bar graph shows the median (+/- s.e.m.) of adiponectin RNA expression in different cell types. The pooled values of two experiments are shown. P-values were calculated using using an unpaired student t-test.

**B, C)** The scatter bar graph shows the median (+/- s.e.m.) of adiponectin receptor 1 and 2 RNA expression in different cell types. The pooled values of two experiments are shown. P-values were calculated using an unpaired student t-test.

**D)** The scatter bar graph shows the MFI for Arg1 in macrophages upon exposure to medium only or different concentrations of adiponectin. The median (+/- s.e.m.) is shown and each data point represents a technical replicate. The pooled values of two experiments are shown. P-values were calculated using an unpaired student t-test.

**E)** The scatter bar graph shows the MFI for Arg1 in macrophages upon exposure to medium only or different concentrations of adiponectin receptor agonist. The median (+/- s.e.m.) is shown and each data point represents a technical replicate. The pooled values of two experiments are shown. P-values were calculated using an unpaired student t-test.

**F)** The scatter plot shows the relative *adiponectin* expression normalized to *Hprt* (y-axis) and *Arg1* expression normalized to *Hprt* (x-axis) in macrophages. The pooled values of two experiments are shown. P-values were calculated using the D'Agostino-Pearson correlation.

**G)** OPTSNE map displaying CD45.2⁺ cells (recipient: CD45.2, ECP donor: CD45.1) from the colon of mice that had DSS-induced colitis and were treated with anti-PD1 alone or anti-PD1 plus ECP analyzed by CYTOFF-based flow cytometry. Intensity of the grey scale corresponds to FlowSOM-guided clustering of cell populations.

**H)** OPTSNE overlay map displaying CD45.2⁺ cells (recipient: CD45.2, ECP donor: CD45.1) from the colon of mice that had DSS-induced colitis and were treated with anti-PD1 alone or anti-PD1 plus ECP analyzed by CYTOFF-based flow cytometry. The overlay shows how different the groups are.

**I)** OPTSNE map displaying arginase-1 expression in the myeloid cluster of CD45⁺ cells (recipient: CD45.2, ECP donor: CD45.1) from the colon of mice that had DSS-induced colitis and were treated with anti-PD1 alone or anti-PD1 plus ECP. Intensity of the grey scale corresponds to protein expression intensity.

**J)** The scatter bar graph shows median (+/- s.e.m.) of Arg1 RNA expression in the colon of mice that had DSS-induced colitis and were treated with anti-PD1 alone or anti-PD1 plus ECP. The p-value was calculated using an unpaired student t-test.

**K)** The scatter bar graph shows median (+/- s.e.m.) of mArg1 in % of Hprt expression in F4/80⁺ cells derived from WT or *Adipoq*^{*-*/*-*} mice. The p-value was calculated using an unpaired student t-test.

**L)** The scatter bar graph shows Arg1 (MFI fold change) in F4/80⁺ cells derived from WT or *Adipoq*^{-/-} mice. The p-value was calculated using an unpaired student t-test.

**M)** The scatter bar graph shows CD206 (MFI fold change) in F4/80⁺ cells derived from WT or *Adipoq*^{*-*/*-*} mice. The p-value was calculated using an unpaired student t-test.

N) The scatter bar graph shows CD301 (MFI fold change) in F4/80⁺ cells derived from WT or *Adipoq*^{*-*/*-*} mice. The p-value was calculated using an unpaired student t-test.

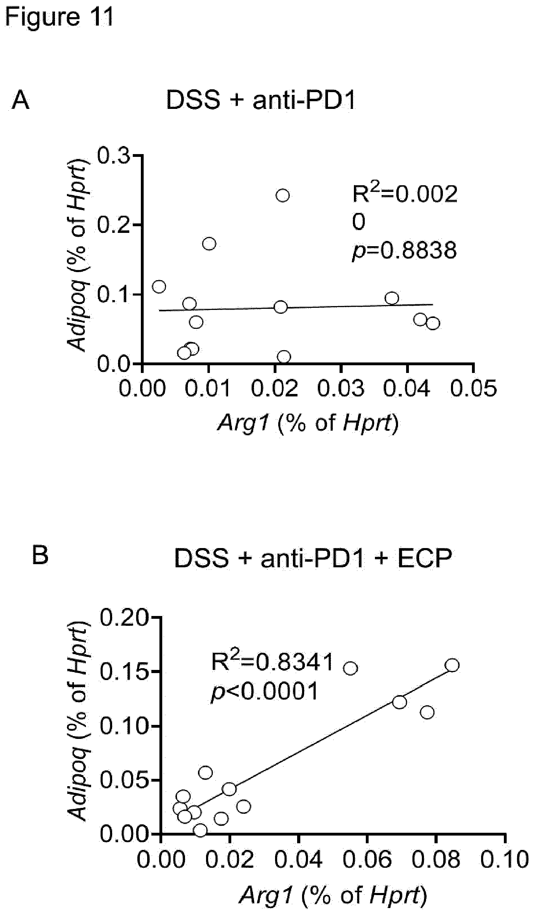

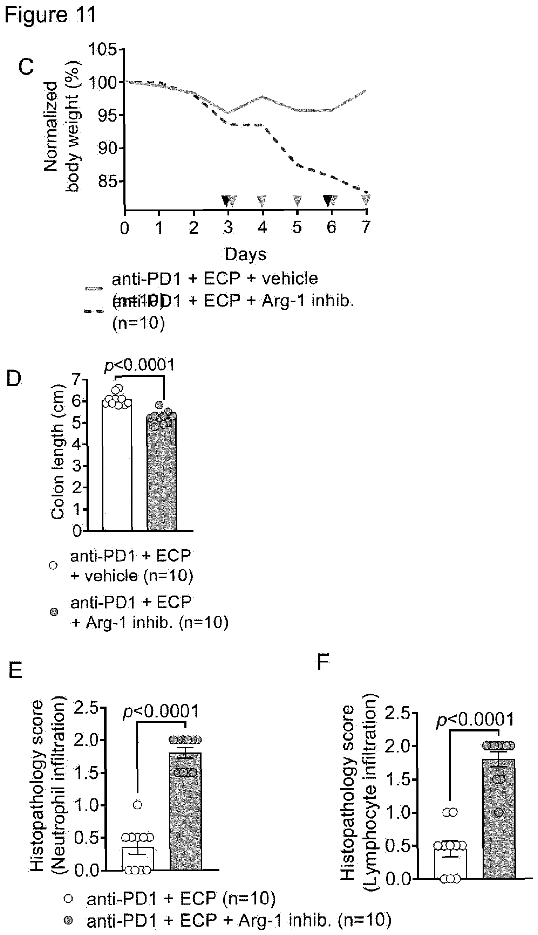

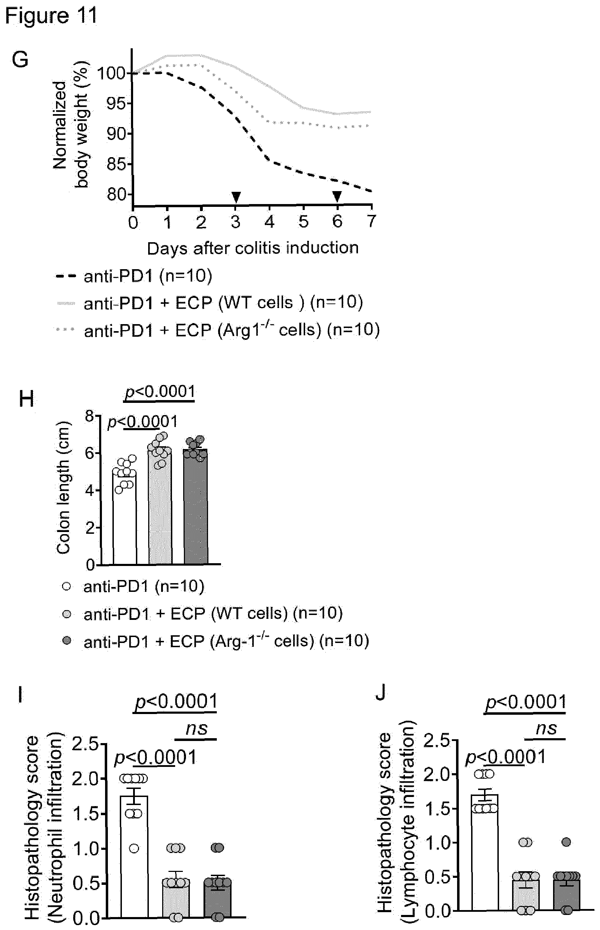

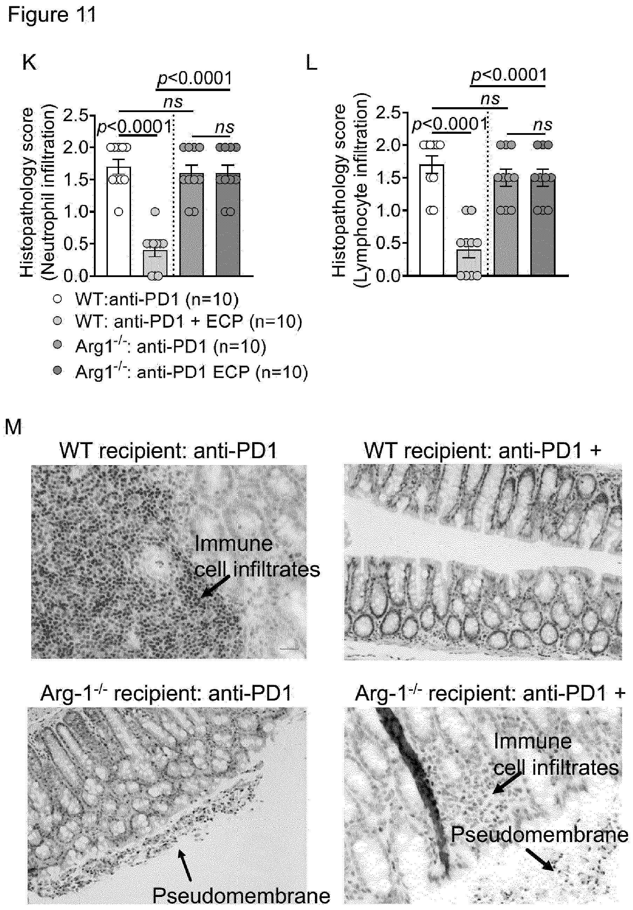

### Figure 11: Arg1 deficiency of the recipient reduces the protective ECP effects

**A)** The scatter plot shows the relative *adiponectin* expression normalized to *Hprt* (y-axis) and *Arg1* expression normalized to *Hprt* (x-axis) in macrophages isolated from mice treated with DSS and anti-PD1. The pooled values of two experiments are shown. P-values were calculated using the D'Agostino-Pearson correlation. No correlation was found.

**B)** The scatter plot shows the relative *adiponectin* expression normalized to *Hprt* (y-axis) and *Arg1* expression normalized to *Hprt* (x-axis) in macrophages isolated from mice treated with DSS, anti-PD1 and ECP. The pooled values of two experiments are shown. P-values were calculated using the D'Agostino-Pearson correlation.

**C)** The graph shows the body weights normalized to the first bodyweight on d0. Follow-up time starts at the first anti-PD1 injection (d0). Groups include animals with DSS-induced colitis treated with anti-PD1, ECP and vehicle or anti-PD1, ECP and Arg1-inhibitor. Each data point represents the mean of the pooled weight of all animals of the indicated group. The black arrows indicate when ECP was performed (d3 and d6), the grey arrows indicate when the vehicle or Arg1-inhibitor treatment was performed. Data were pooled from 2-5 experiments.

**D)** The scatter bar graph shows the median (+/- s.e.m.) of the colon length of mice with DSS/anti-PD1-induced colitis treated with ECP plus vehicle or plus Arg1-inhibitor as indicated. The pooled values of one experiment are shown. P-values were calculated using an unpaired student t-test.

**E, F)** The scatter bar graphs show the median (+/- s.e.m.) of the pooled values of the histopathological grading of neutrophil **(E)** and lymphocyte **(F)** infiltration into the colon (0 = absent, 1 = mild, 2 = massive) for the indicated groups. Each data point represents the value of an individual mouse. The p-value was calculated using an unpaired student t-test.

**G)** The graph shows the body weights normalized to the first bodyweight on d0. Follow-up time starts at the first anti-PD1 injection (d0). Groups include animals with DSS-induced colitis treated with anti-PD1 alone or anti-PD1 and ECP. When indicated the ECP treated splenocytes were derived from WT or *Arg1*^{*-*/*-*} mice. Each data point represents the mean of the pooled weight of all animals of the indicated group. The black arrows indicate when ECP was performed (d3 and d6). Data were pooled from 2-5 experiments.

**H)** The scatter bar graph shows the median (+/- s.e.m.) of the colon length of mice with DSS and anti-PD1 or DSS, anti-PD1 and ECP as indicated. When indicated the ECP treated splenocytes were derived from WT or *Arg1*^{*-*/*-*} mice. The pooled values of one experiment are shown. P-values were calculated using a One-way ANOVA.

**I, J)** The scatter bar graphs show the median (+/- s.e.m.) of the pooled values of the histopathological grading of neutrophil **(I)** and lymphocyte **(J)** infiltration into the colon (0 = absent, 1 = mild, 2 = massive) for the indicated groups. When indicated the ECP treated splenocytes were derived from WT or *Arg1*^{*-*/*-*} mice. Each data point represents the value of an individual mouse. The p-value was calculated using an unpaired student t-test.

**K, L)** The scatter bar graphs show the median (+/- s.e.m.) of the pooled values of the histopathological grading of neutrophil **(K)** and lymphocyte **(L)** infiltration into the colon (0 = absent, 1 = mild, 2 = massive) for the indicated groups. When indicated the recipient mice were WT or *Arg1*^{*-*/}*⁻.* Groups include animals with DSS-induced colitis treated with anti-PD1 alone or anti-PD1 and ECP. Each data point represents the value of an individual mouse. The p-value was calculated using an unpaired student t-test.

**M)** Representative histology images of colon samples from mice with DSS-induced colitis and anti-PD1 or anti-PD1 plus ECP treatment are shown. When indicated the recipient mice were WT or *Arg1*^{*-*/}*⁻.* The red arrows point towards immune cell infiltrates in the colon wall. The blue arrows point towards a pseudomembrane on the colon mucosa.

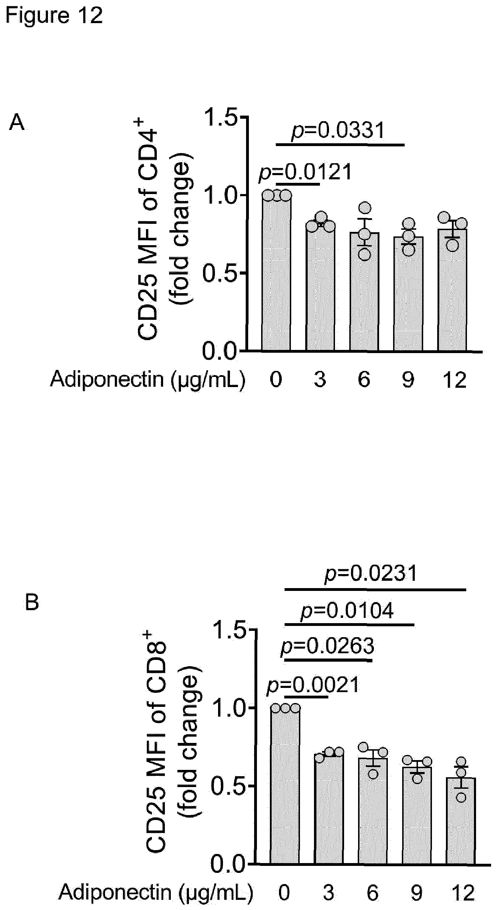

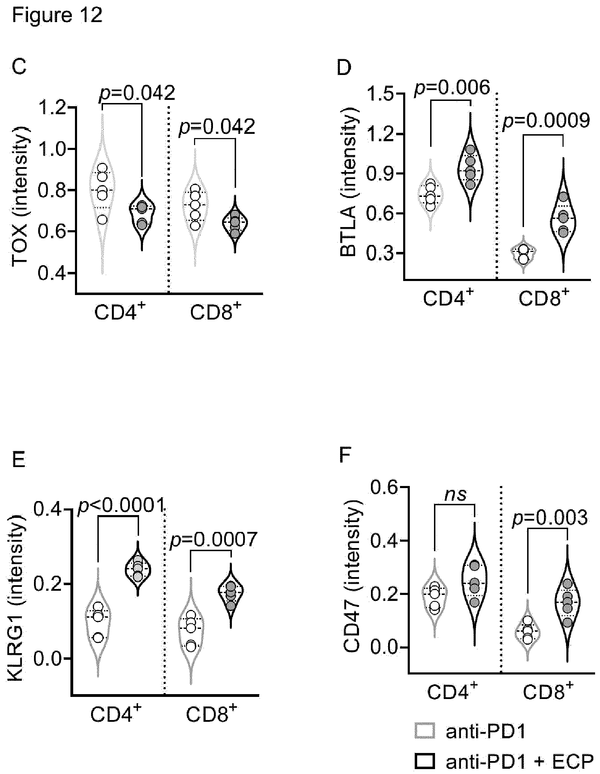

### Figure 12: ECP and adiponectin reduce pathogenic T cell activation

**A, B)** The scatter bar graphs show the MFI for CD25 on CD4⁺ **(A)** and CD8⁺ **(B)** T cells upon exposure to medium only or different concentrations of adiponectin. The median (+/- s.e.m.) is shown and each data point represents a technical replicate. The pooled values of two experiments are shown. P-values were calculated using a paired t-test.

**C-F)** Violin plots displaying protein expression of TOX, BLTA, KLRG1 and CD47 in CD4⁺ or CD8⁺ T cells as indicated. Gating on CD45⁺ cells (recipient: CD45.2, ECP donor: CD45.1) from the colon of mice that had DSS-induced colitis and were treated with anti-PD1 alone or anti-PD1 plus ECP. The p-value was calculated using an unpaired student t-test.

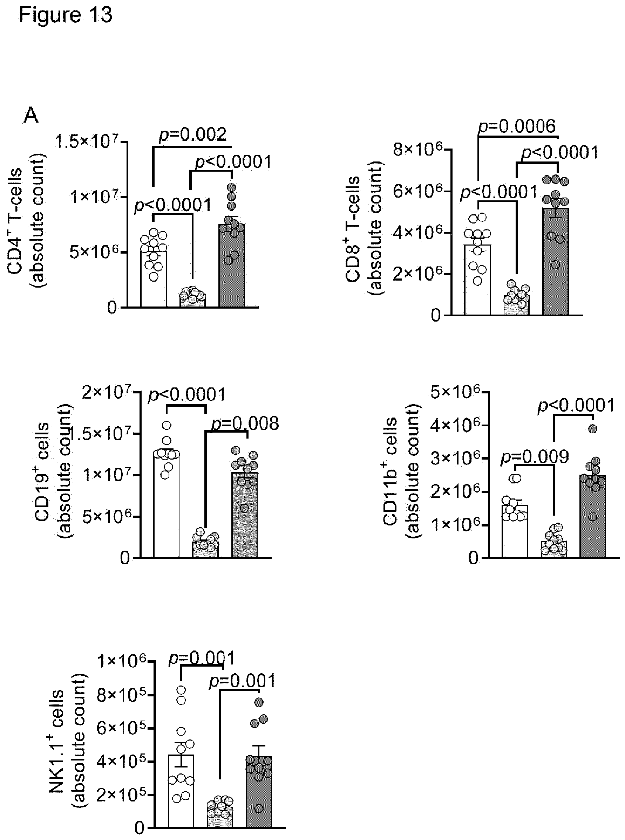

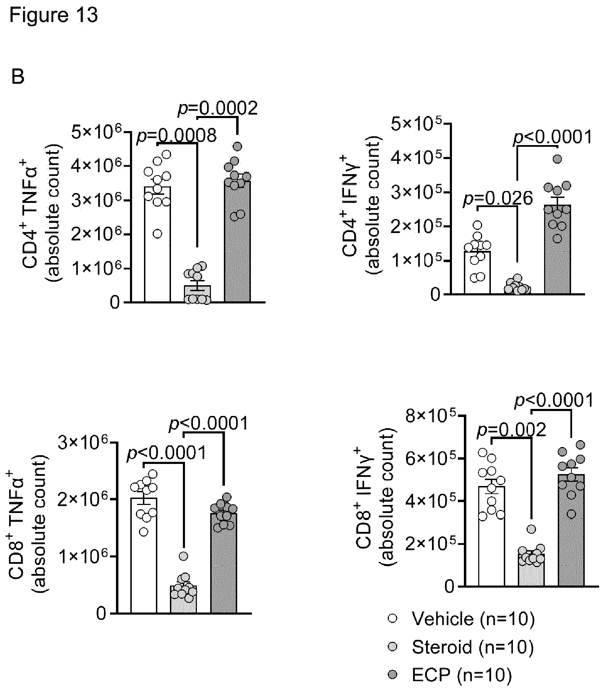

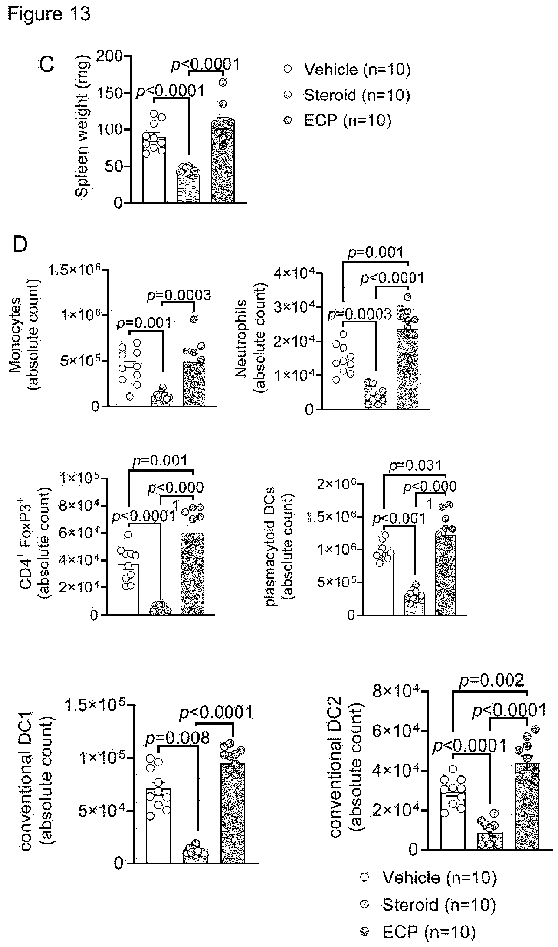

### Figure 13: The effect of steroids and ECP on the anti-tumor immune responses

**A)** The scatter bar graphs show the median (+/- s.e.m.) of the absolute counts of CD4⁺, CD8⁺, CD19⁺, CD11b⁺ and NK1.1⁺ cells isolated from the spleens of mice treated with vehicle, prednisolone or ECP as indicated. Each data point represents the value of an individual mouse. The p-value was calculated using an unpaired student t-test.
**B)** The scatter bar graphs show the median (+/- s.e.m.) of the absolute counts of CD4⁺TNF⁺, CD4⁺INFγ⁺, CD8⁺TNF⁺, CD8⁺INFγ⁺ cells isolated from the spleens of mice treated with vehicle, prednisolone or ECP as indicated. Each data point represents the value of an individual mouse. The p-value was calculated using an unpaired student t-test.
**C)** The scatter bar graphs show the median (+/- s.e.m.) of the spleen weight of mice treated with vehicle, prednisolone or ECP as indicated. Each data point represents the value of an individual mouse. P-values were calculated using a One-way ANOVA.
**D)** The scatter bar graphs show the median (+/- s.e.m.) of the absolute counts of monocytes, neutrophils, Treg, plasmacytoid DCs, DC1 and DC2 isolated from the spleens of mice treated with vehicle, prednisolone or ECP as indicated. Each data point represents the value of an individual mouse. P-values were calculated using a One-way ANOVA.

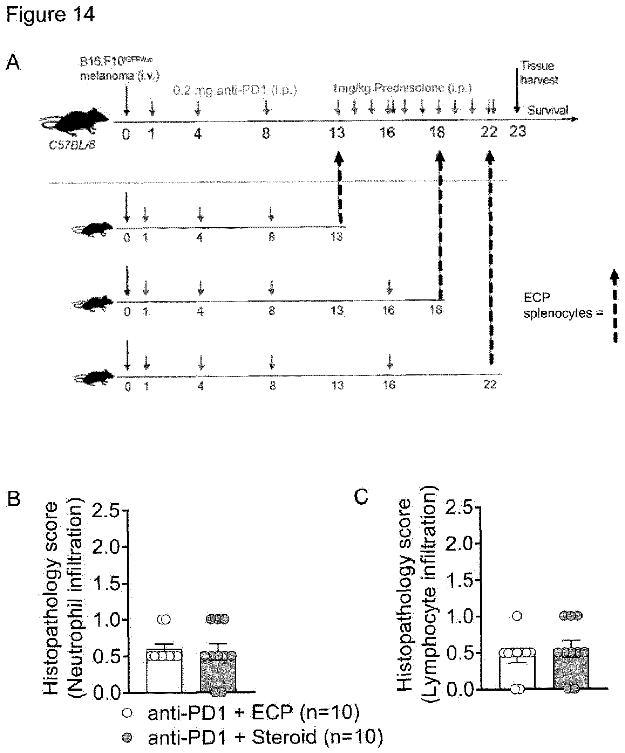

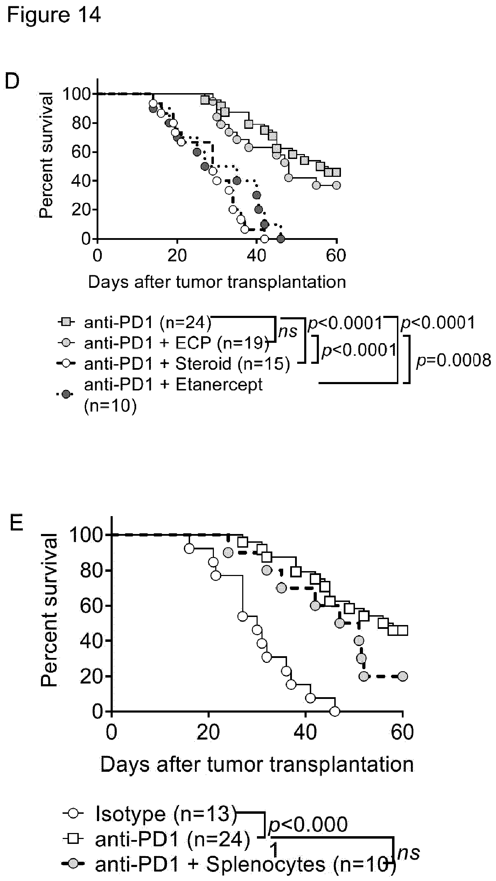

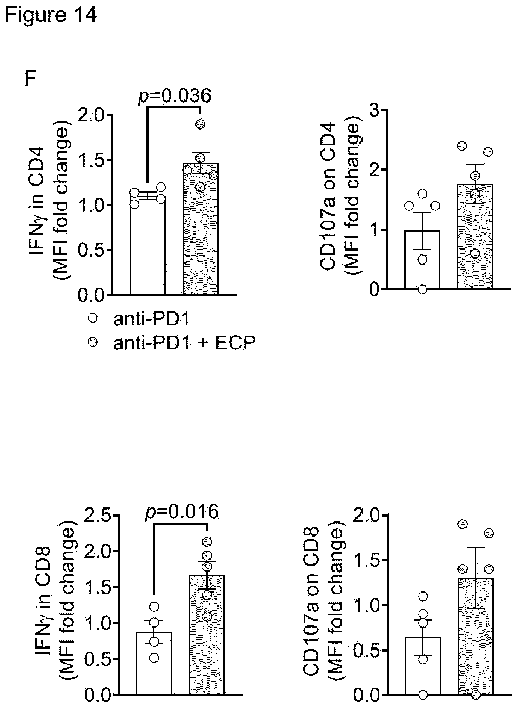

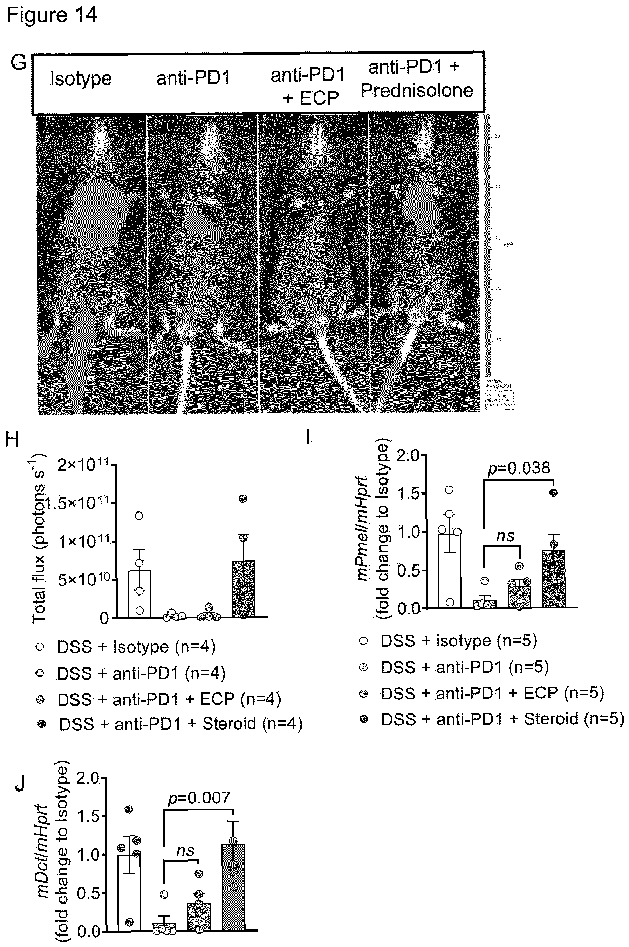

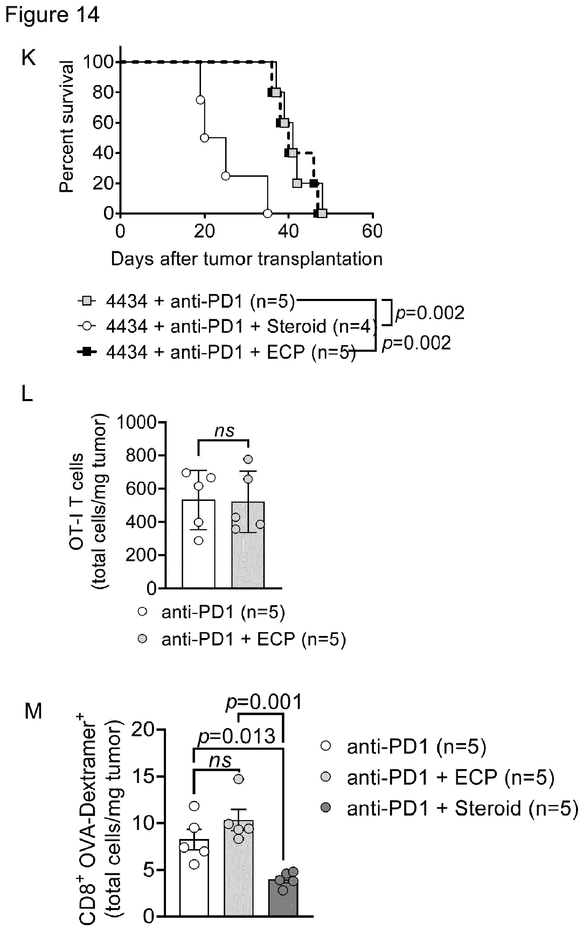

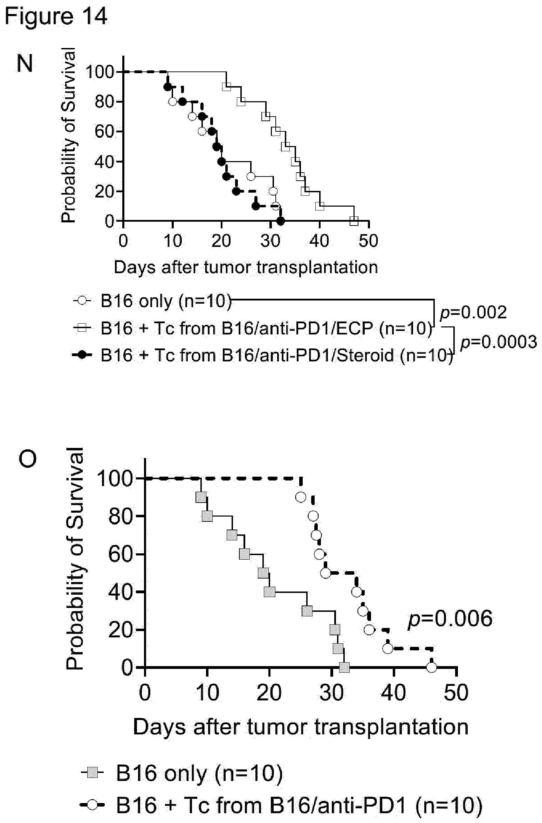

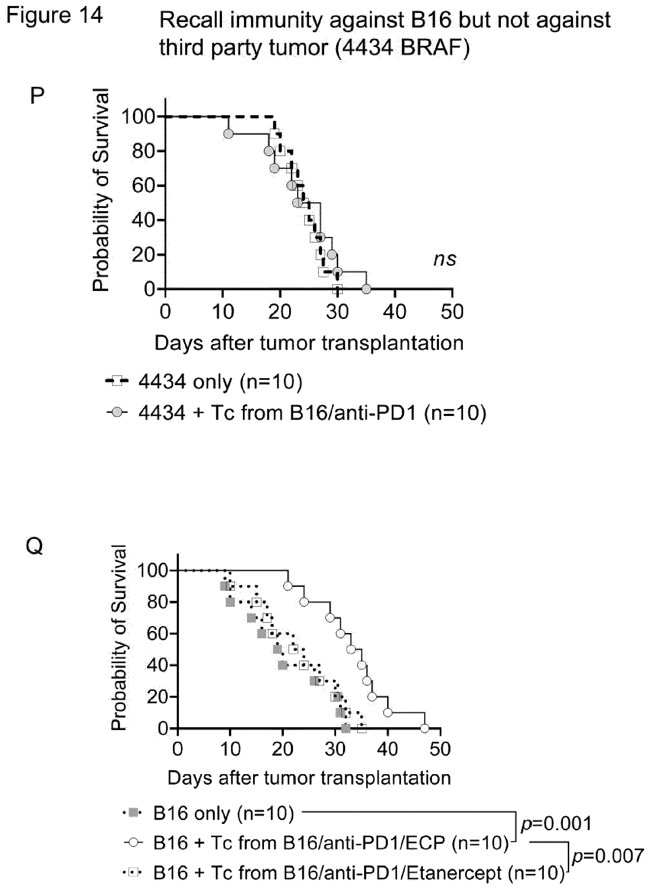

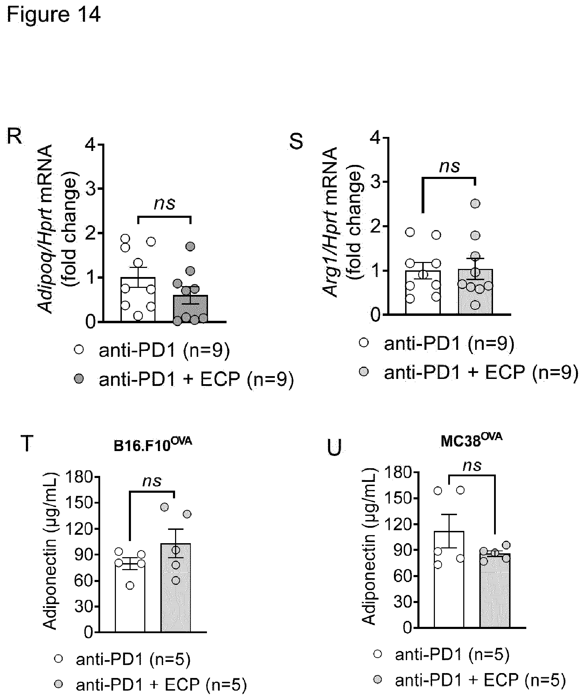

### Figure 14: The protective ECP effect against irAE colitis allows for anti-tumor immune responses

**A)** Experimental setup of DSS/anti-PD1-induced colitis with ECP treatment of B16F10 melanoma bearing mice. Samples were harvested on day 23 after start of DSS treatment. The light grey arrows indicate ant-PD1 treatment. The dark grey arrows indicate prednisolone (1 mg/kg bodyweight) treatment.

**B, C)** The scatter bar graphs show the median (+/- s.e.m.) of the pooled values of the histopathological grading of neutrophil **(B)** and lymphocyte **(C)** infiltration into the colon (0 = absent, 1 = mild, 2 = massive) for the indicated groups. Each data point represents the value of an individual mouse. The p-value was calculated using an unpaired student t-test.

**D)** Percentage survival of C57BL/6 mice after transfer of B16.F10 melanoma cells (C57BL/6 background) is shown. Mice received anti-PD-1 treatment and when indicated additional prednisolone, etanercept (anti-TNF) or ECP treatment. The number of animals per group are shown and p-values were calculated using the two-sided Mantel-Cox test.

**E)** Percentage survival of C57BL/6 mice after transfer of B16.F10 melanoma cells (C57BL/6 background) is shown. Mice received isotype antibody, anti-PD-1 treatment or and anti-PD-1 treatment and splenocytes when indicated. The number of animals per group are shown and p-values were calculated using the two-sided Mantel-Cox test.

**F)** The scatter bar graphs show the median (+/- s.e.m.) of the MFI for INFγ and CD107a in CD4⁺ or CD8⁺, T cells isolated from the spleens of mice treated with anti-PD-1 treatment or and anti-PD-1 treatment and ECP when indicated. Each data point represents the value of an individual mouse. The p-value was calculated using an unpaired student t-test.

**G)** Representative bioluminescence images of mice described in **(E)** taken 14 days after tumor injection are shown.

**H)** The graph shows the photon flux (p/s/mouse) of C57BL/6 mice after i.v. transfer of B16.F10-luc⁺ melanoma cells and treatment with either Isotype control, anti-PD-1, anti-PD-1 and ECP or anti-PD-1 and prednisolone. For the bioluminescence imaging measurements the following parameters were kept constant for all groups: amount of luciferin injected, type of luciferin, exposure time and distance to camera. The means +/- s.e.m. are shown. n=4 animals per group are shown and P-values were calculated using a two-sided Student's unpaired *t-test.*

**I)** The scatter bar graph shows the median (+/- s.e.m.) of the *mPmel*/*mHprt* ratio in fold change relative to mice treated with isotype. The lungs isolated from B16.F10 melanoma bearing mice (C57BL/6 background) were studied. Mice received anti-PD-1 treatment and when indicated additional prednisolone, etanercept (anti-TNF) or ECP treatment. P-values were calculated using a two-sided Student's unpaired *t-test.*

**J)** The scatter bar graph shows the median (+/- s.e.m.) of the *mDct*/*mHprt* ratio in fold change relative to mice treated with isotype. The lungs isolated from B16.F10 melanoma bearing mice (C57BL/6 background) were studied. Mice received anti-PD-1 treatment and when indicated additional prednisolone, etanercept (anti-TNF) or ECP treatment. P-values were calculated using a two-sided Student's unpaired *t-test.*

**K)** Percentage survival of C57BL/6 mice after transfer of 4344-BRAF melanoma cells (C57BL/6 background) is shown. Mice received isotype antibody, anti-PD-1 treatment or and anti-PD-1 treatment and prednisolone or ECP when indicated. The number of animals per group are shown and p-values were calculated using the two-sided Mantel-Cox test.

**L)** The scatter bar graph shows the median (+/- s.e.m.) of the *OT*-*I T* cells in total cells/mg tumor for the indicated groups. The lungs isolated from B16.F10 melanoma bearing mice (C57BL/6 background) were studied. Mice received anti-PD-1 treatment and when indicated additional ECP treatment. P-values were calculated using a two-sided Student's unpaired *t-test.*

**M)** The scatter bar graph shows the median (+/- s.e.m.) of the CD8⁺ OVA-Dextramer⁺ T cells in total cells/mg tumor for the indicated groups. The lungs isolated from B16.F10 melanoma bearing mice (C57BL/6 background) were studied. Mice received anti-PD-1 treatment and when indicated additional prednisolone or ECP treatment. P-values were calculated using a two-sided Student's unpaired *t-test.*

**N)** Percentage survival of C57BL/6 mice after transfer of B16.F10 melanoma cells (C57BL/6 background) is shown. Mice received B16.F10 melanoma cells only or additional T cells isolated from B16.F10 bearing mice that had received anti-PD-1 treatment and when indicated additional prednisolone or ECP treatment. The number of animals per group are shown and *p*-values were calculated using the two-sided Mantel-Cox test.

**O)** Percentage survival of C57BL/6 mice after transfer of B16.F10 melanoma cells (C57BL/6 background) is shown. Mice received B16.F10 melanoma cells only or additional T cells isolated from mice that had received anti-PD-1 treatment when indicated. The number of animals per group are shown and *p*-values were calculated using the two-sided Mantel-Cox test.

**P)** Percentage survival of C57BL/6 mice after transfer of 4434-BRAF melanoma cells (C57BL/6 background) is shown. Mice received 4434-BRAF melanoma cells only or additional T cells isolated from B16.F10 bearing mice that had received anti-PD-1 treatment when indicated. The T cells isolated from the B16.F10 bearing mice are expected to not recognize the 4434-BRAF melanoma cells. The number of animals per group are shown and *p*-values were calculated using the two-sided Mantel-Cox test.

**Q)** Percentage survival of C57BL/6 mice after transfer of B16.F10 melanoma cells (C57BL/6 background) is shown. Mice received B16.F10 melanoma cells only or additional T cells isolated from B16.F10 bearing mice that had received anti-PD-1 treatment and when indicated additional etanercept or ECP treatment. The number of animals per group are shown and *p*-values were calculated using the two-sided Mantel-Cox test.

**R)** The scatter bar graph shows median (+/- s.e.m.) of adiponectin RNA expression in the s.c. grown B16.F10 melanoma of mice that had DSS-induced colitis and were treated with anti-PD1 alone or anti-PD1 plus ECP. Gene expression data was normalized to Hprt expression and is depicted as fold change to anti-PD1 treatment. The experiment was performed 2 times. The p-value was calculated using an unpaired student t-test.

**S)** The scatter bar graph shows median (+/- s.e.m.) of arginase-1 RNA expression in the s.c. grown B16.F10 melanoma of mice that had DSS-induced colitis and were treated with anti-PD1 alone or anti-PD1 plus ECP. Gene expression data was normalized to Hprt expression and is depicted as fold change to anti-PD1 treatment. The experiment was performed 2 times. The p-value was calculated using an unpaired student t-test.

**T)** The scatter bar graph shows median (+/- s.e.m.) of adiponectin in homogenized s.c. grown B16.F10 melanoma tissue samples of mice that had DSS-induced colitis and were treated with anti-PD1 alone or anti-PD1 plus ECP. The experiment was performed multiple times. Each data point represents an individual mouse. The p-value was calculated using an unpaired student t-test.

**U)** The scatter bar graph shows median (+/- s.e.m.) of adiponectin in homogenized s.c. grown MC-38 colorectal cancer tissue samples of mice that had DSS-induced colitis and were treated with anti-PD1 alone or anti-PD1 plus ECP. The experiment was performed multiple times. Each data point represents an individual mouse. The p-value was calculated using an unpaired student t-test.

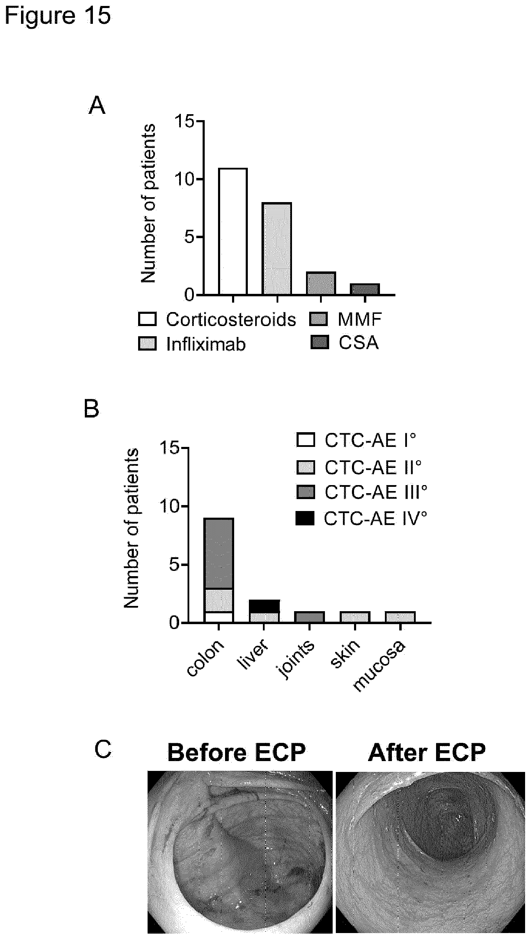

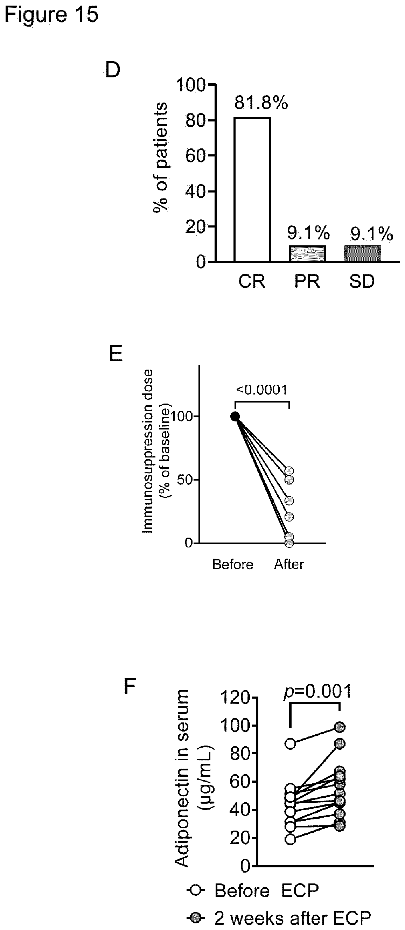

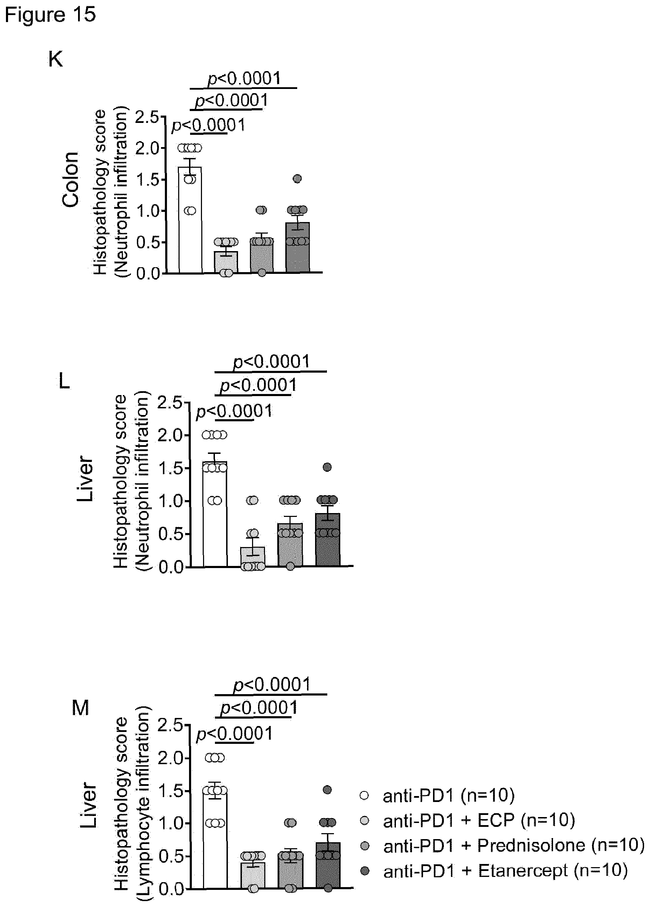

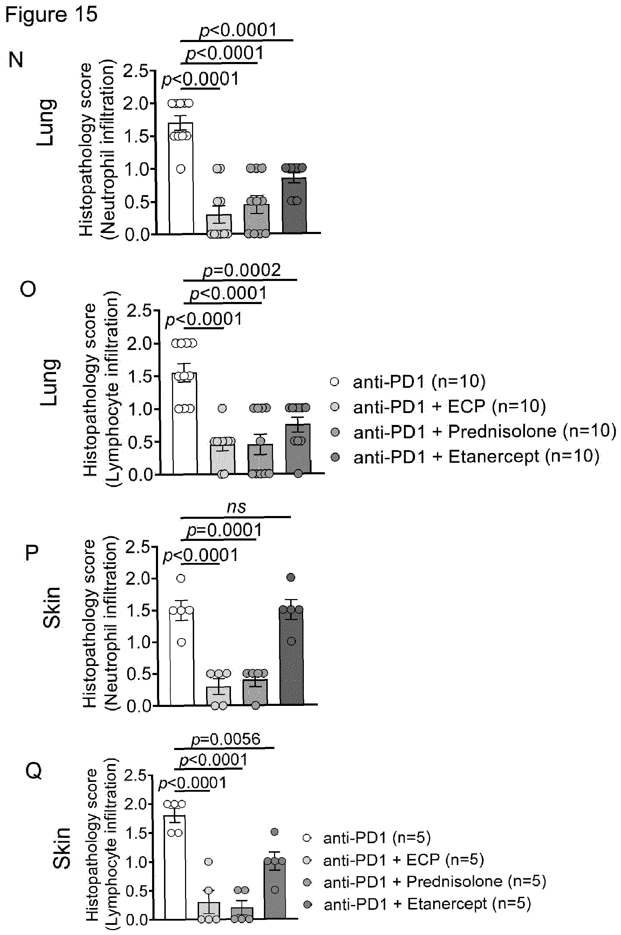

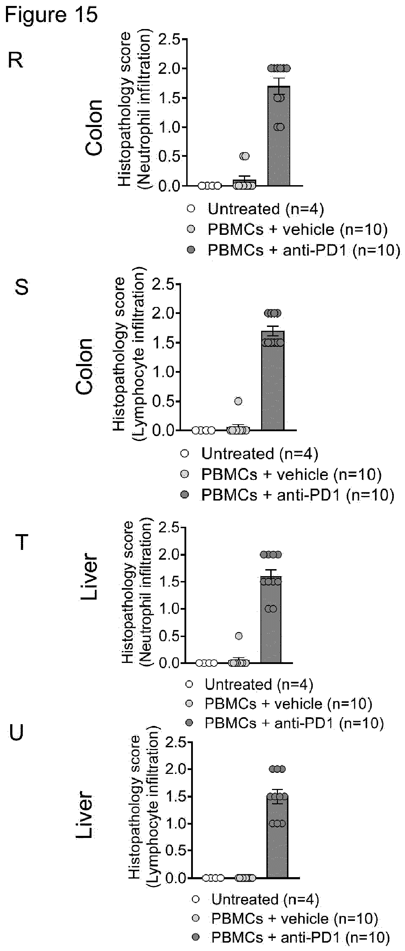

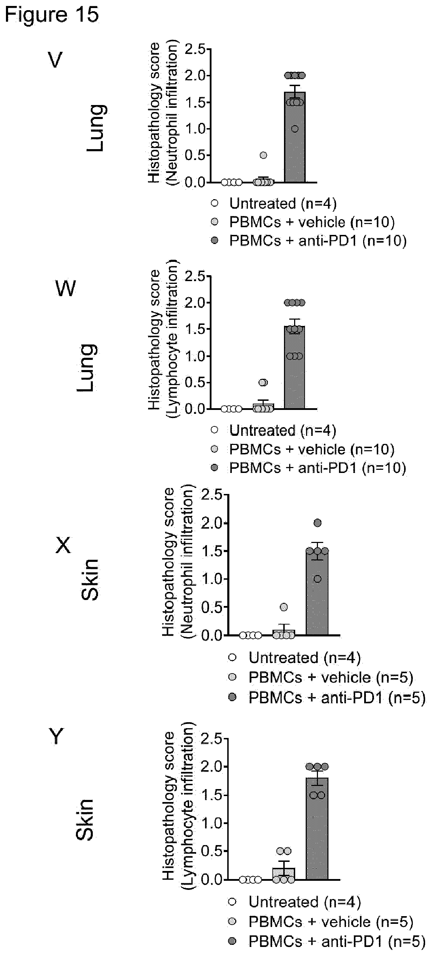

### Figure 15: Clinical activity of ECP in patients with irAE

**A)** The bar graph shows the number of patients that had received the indicated immunosuppressive medication for irAE before ECP, including corticosteroids, infliximab (anti-TNF), mycophenolate mofetile (MMF) and cyclosporine A.

**B**) The bar diagram shows the organ manifestation and the irAE severity grade of the patients included into the study cohort.

**C**) The endoscopic images show the colon mucosa before and after 6 weeks of ECP treatment in a representative patient.

**D**) The bar diagram shows the best response of the irAE to ECP treatment. CR: complete remission, PR: partial remission, SD: stable disease.

**E**) The scatter plot shows the dosage of immunosuppression at start of ECP and at last ECP treatment. The initial dosage was set as 100% (baseline) and the dosage at last ECP treatment was adjusted as the relative dosage. Each data point represents an individual patient. *P*-values were calculated using a two-sided Student's paired *t-test.*

**F**) The scatter plot shows the adiponectin concentration in the serum of patients before and 2 weeks after ECP treatment. Each data point represents an individual patient. *P*-values were calculated using a two-sided Student's paired *t-test.*

**G**) Representative adiponectin staining of the colon of a patient with ICB-induced colitis before and after ECP treatment. Adiponectin staining is indicated by the arrow. The scale bar represents 10 µm.

**H**) Cumulative adiponectin positive cells / all cells in the colon of patients with ICB-induced colitis before and after ECP treatment. Each data point represents an individual patient. *P*-values were calculated using a two-sided Student's paired *t-test.*

**I)** Experimental setup of the humanized irAE model with Rag2^{-/-}γc^{-/-} recipient mice. To induce irAEs 10 million human PBMCs were injected ip followed by treatment with human anti-PD1 antibodies. Samples were harvested on day 15 after PBMC injection. The light grey arrows indicate ant-PD1 treatment, dark grey arrows: prednisolone (1 mg/kg bodyweight) treatment, red arrows: ECP, black arrows: eternacept.

**J-Q)** The scatter bar graphs show the median (+/- s.e.m.) of the pooled values of the histopathological grading of neutrophil and lymphocyte infiltration into the colon, skin, lung and liver (0 = absent, 1 = mild, 2 = massive) of immunodeficient mice treated with human PBMCs plus anti-PD1 (human) and vehicle, prednisolone, ECP or etanercept, as indicated. Each data point represents the value of an individual mouse. *P*-values were calculated using a One-way ANOVA.

**R-Y)** The scatter bar graphs show the median (+/- s.e.m.) of the pooled values of the histopathological grading of neutrophil and lymphocyte infiltration into the colon, skin, lung and liver (0 = absent, 1 = mild, 2 = massive) of immunodeficient mice treated with human PBMCs alone or PBMCs plus anti-PD1 (human), as indicated. Each data point represents the value of an individual mouse. *P*-values were calculated using a One-way ANOVA.

### EXAMPLES

The invention is further described by the following examples. These are not intended to limit the scope of the invention but represent preferred embodiments of aspects of the invention provided for greater illustration of the invention described herein.

### Methods:

### Clinical ECP treatment of patients

At all four centers, ECP was performed on a Therakos CellEx photopheresis system. Methoxsalen (Uvadex^{©}) was administered as a photo-sensitizing agent and 1500 ml blood were processed during each procedure. At the Medical Center - University of Freiburg, the University of Essen and the University of Hamburg, two procedures were commonly performed on consecutive days. The frequency and total number of ECP procedures were determined on individual basis for each patient. At the University of Regensburg, one ECP procedure was performed every 2-3 days for a total of 14 days.

### Evaluation of irAE response of patients

The severity of irAE was evaluated by a clinical assessment of the symptoms, and carried out before the initiation of ECP and at every patient visit after initiation of the ECP therapy. Grading of irAE was performed according to the Common Terminology Criteria for Adverse Events (CTC-AE v5). Hypothyroidism that was hormone-substituted was excluded from the criteria for response evaluation, if TSH was normal as an indication that thyroiditis has resolved. A complete response (CR) was defined as the complete resolution of all irAE-related symptoms. A partial response (PR) was defined as the reduction of at least 1 grade according to CTC-AE in at least 1 irAE organ manifestation. A stable disease (SD) was defined as no change in the CTC-AE grades of all irAE. A progressive disease (PD) was defined as the increase of at least 1 grade according to CTC-AE in at least 1 irAE organ manifestation. CR, PR and PD were diagnosed only if the symptom severity remained stable for at least 5 consecutive days.

### Cell lines

The B16.F10 murine melanoma cell line (C57BL/6 background) was provided by Hanspeter Pircher (Freiburg University, Germany). Luciferase transgenic B16.F10^{luc/GFP} cells were established as described previously (Mastroianni 2019). The BRAF-mutant 4434 murine melanoma cell line was established C57BL/6 LSL-Braf^{V600E};Tyr::CreERT^{+/o} mice (Dhomen 2009) and kindly provided by Richard Marais (Cancer Research UK Manchester Institute, Manchester, UK). The MC38 and MC38^{OVA} cell lines were kindly provided by Karen Dixon and Vijay Kuchroo (Harvard Medical School, US). All cell lines were cultured in DMEM high glucose medium (Gibco), supplemented with 10 % FCS (Sigma) and 1 % Penicillin/streptomycin (Gibco) at 37 °C and 5 % CO₂ in a humidified atmosphere. Medium of MC38 lines was additionally supplemented with 1 % sodium pyruvate (Gibco). B16.F10^{luc/GFP} cells were cultured with 2 mg/mL of the neomycin analogue Geneticin (G-418 sulfate; Genaxxon) after thawing.

### In vivo Bioluminescence imaging (BLI)

Luciferin (D-luciferin, potassium salt (S)-4,5-Dihydro-2-(6-hydroxy-2-benzothiazolyl)-4-thiazolecarboxylic acid potassium salt; Biosynth) was dissolved in H₂O and injected i.p. at a concentration of 150 µg/g body weight. After 10 minutes, the mice were imaged using an IVIS Lumina III *in vivo* imaging system (PerkinElmer) with an exposure time of 2-5 minutes. The luciferase signal was quantified in photons er second per mouse. Acquisition, analysis, and visualization of BLI were performed using Living Image Software (PerkinElmer).

### ECP treatment, mouse model

For modeling ECP transplantation *in vivo,* splenocytes of donor mice were used. Donor animals were treated identically to the recipient groups. Spleens were harvested, followed by splenocyte isolation and erythrocyte lysis. Splenocytes were resuspended to 3-5 ×10⁶ cells/mL in RPMI, supplemented with 10 %FCS and 1 %P/S. Cells were treated with 200 ng/mL 8-methoxypsoralen (8-MOP; Uvadex^{©}, Therakos) at 37 °C and 5 % CO₂ for 30 min. Following incubation, the cells were exposed to UVA light (2 J/cm²) in a calibrated BS-02 irradiation chamber, operated in dose-controlled mode (Opsytec Dr. Gröbel). ECP treated cells were resuspended in PBS in injected intravenously to recipient mice.

### DSS colitis model

For the induction of colitis, C57BL/6 WT, Adipoq^{-/-} or Arg1-deficient chimera mice received 3 % Dextran Sodium Sulfate (DSS colitis grade, 36-50 kDa; MP Biomedicals) in drinking water from day 0 to day 3, followed by normal drinking water. Mice were injected (i.p.) with 0.2 mg immune checkpoint blockade (ICB) or the respective isotype controls on day 0, day 3 and day 6 of the experiment. Recipients were transplanted (i.v.) with 10⁷ ECP treated donor splenocytes on day 3 and day 6. In some experiments, steroid, Adiponectin and AdipoRon were applied daily from day 3-7. Arginase-1 inhibitor was applied twice per day from day 4-7. Serum was harvested on day 8 by retroorbital bleeding under ketamine/xylazine anesthesia, followed by harvest of large intestine and spleen.

### Histopathological analysis of irAE target organs

Colon, liver, skin and lung were harvested as indicated, formalin-fixed and paraffin embedded. Sections were stained with H&E and scored by an experienced pathologist, based on histopathological characterization of human irAEs, including lymphocyte and neutrophil infiltration, crypt abscesses, and apoptotic cells (Beck 2006, Johncilla 2015, Marschner 2020). Scoring was done blinded to the experimental group.

### Intestinal leukocyte isolation

Intestinal leukocytes were isolation using the lamina propria dissociation kit (Miltenyi) according to manufacturer's instructions. In brief, colon samples were harvested, and Peyer's patches removed. Samples were flushed with PBS to remove feces, opened longitudinally and cut into 5-10 mm segments. Epithelial cells were removed using pre-digestion solution. Colon tissue was transferred into gentleMACS C Tubes (Miltenyi) containing the enzyme mix and digested on a gentleMACS Octo Dissociator with Heater (Miltenyi) using the appropriate program. Cells were washed with PBS and applied on a 100 µm strainer before further downstream analysis.

### Immunohistochemistry and evaluation of adiponectin staining

For immunohistochemistry (IHC), 2 µm sections of FFPE-tissue were generated, de-paraffinized, rehydrated and underwent established heat-induced antigen retrieval (HIAR - steam cooker, 20 minutes, pH9). HIAR was followed by peroxidase blocking for 15 minutes using 1 % H₂O₂. Blocking of sections was performed in 5 % NGS in PBS for 30 minutes, followed by primary antibody incubation in blocking solution for 1 hour (primary antibody adiponectin, ThermoFisher, #710179, Lot:2052429, 1:400 dilution). HRP-linked secondary antibodies (Dako) were applied for 30 minutes in blocking solution. Visualization and staining was performed using the DAB+ Substrate Chromogen System (Dako) including counterstaining with Haematoxylin. After dehydration slides were mounted using Entellan.

### Microscopy and scoring of adiponectin staining

For analysis of immunohistochemistry (IHC) and histology, an inverted Zeiss Axio Imager microscope (Zeiss Imager.M1) equipped with an Axiocam 506 color camera and equipped with 10x, 20x, 40x objective was used. Quantifications of adiponectin positive cells within the lamina propria were performed on digitalized images (randomly captured at 40x magnification). Positive and negative cells in hotspot regions were assigned using Fiji imaged v1.52. Adiponectin positive cells were expressed as either densities (in patient samples) or positive cells per hotspot region (in murine samples).

### B16.F10 intravenous tumor model

WT C57BL/6 mice were intravenously injected with 10 000 B16.F10^{luc/GFP} melanoma cells in the tail vein and the survival was monitored. The mice were treated with 0.2 mg ICB or the respective isotype control on day 1, 4, 8, 16 and 22. ECP treated splenocytes were transplanted (10⁷; i.v.) on day 13, 18, and 22, daily steroid was given (i.p.) from day 13-22, Etanercept was given (i.p.) on day 12, 14, 16, 18, 20 and 22.

### 4434 intravenous tumor model

For a second melanoma model, WT C57BL/6 recipients were transplanted intravenously with 2×10⁶ 4434 melanoma cells in the tail vein and survival was monitored. The treatment schedule was comparable as described for the B16.F10 (i.v.) model.

### MC38^{OVA} subcutaneous tumor model

WT C57BL/6 recipients were transplanted subcutaneously with 1×10⁶ MC38^{OVA} or 0.5×10⁶ MC38 (control only) cells into the shorn right flank. Recipients were treated with 0.2 mg ICB on day 1, 4, 8, 11, 15. ECP treated splenocytes were transplanted (10⁷; i.v.) on day 6, 11 and15, daily steroid was given (i.p.) from day 6-15. On day 16, serum samples were harvested by retroorbital bleeding, the mice were euthanized and tumors and spleen were resected for further studies.

### B16.F10^{OVA} subcutaneous model with adoptive T cell transfer

CD45.1 recipients were transplanted subcutaneously with 1×10⁶ B16.F10^{OVA} cells into the shorn right flank. Recipients were treated with 0.2 mg ICB on day 1, 4, 8, 11, 15. ECP treated splenocytes were transplanted (10⁷; i.v.) on day 6, 11 and 15, daily steroid was given (i.p.) from day 6-15. OT-I splenocytes (CD45.2) were activated on day 2 at a density of 2×10⁶ cells/mL in the presence of 100 U/mL rmlL-2 (Peprotech) and 0.1 µg/mL OVA²⁵⁷⁻²⁶⁴ peptide (Sigma) in RPMI supplemented with 10 %FCS, 1 %P/S, 55 µM) beta-Mercaptoethanol (Gibco) and 4 mM glutamine (Gibco). Splenocytes were washed on day 4 and replated with 100 U/mL rmlL-2 at 2×10⁶ cells/mL. On day 5, OT-I splenocytes were washed and 5×10⁶ cells were injected intravenously into the tail vein of tumor bearing mice. On day 16, serum samples were harvested by retroorbital bleeding, the mice were euthanized and tumors and spleen were resected for further studies.

### Combined Colitis and B16.F10 tumor model

WT C57BL/6 mice were intravenously injected with 10 000 B16.F10^{luc/GFP} melanoma cells in the tail vein and received 3 % Dextran Sodium Sulfate (DSS colitis grade, 36-50 kDa; MP Biomedicals) in drinking water from day 9-12, followed by normal drinking water. Mice were treated with 0.2 mg ICB or the respective isotype control on day 1, 4, 8, 16 and 22. ECP treated splenocytes were transplanted (10⁷; i.v.) on day 13, 18 and 22, daily steroid was given (i.p.) from day 13-22. Mice were imaged with BLI on day 23, organs and serum were harvested on day 24.

### Xenograft-induced irAE model

Xenograft-induced irAE was induced as described previously (Perez-Ruiz 2019). In brief, Rag2^{-/-}γc^{-/-} were injected intraperitoneally with 10⁷ human PBMCs in 200 µL PBS. Healthy donor PBMCs were isolated by density-gradient separation (Lymphocyte separation medium, Anprotec). On days 4, 7, 10, and 13, mice were treated with 200 µg Pembrolizumab (i.p.), transplanted with 10⁷ ECP-treated human PBMCs (days 4, 7, 10, 13; i.v. tail vein), treated with prednisolone (days 4-13; i.p.) or Etanercept (days 4, 7, 10, 13; i.p.). The mice were sacrificed on day 15 with serum and organ harvest.

### ALT and AST activity assays

The ALT and AST activity assays on serum harvested from mice with xenograft-induced irAE were performed according to manufacturer's instructions (Sigma).

### Hematopoietic chimera generation

To generate mice lacking Arginase-1 in the hematopoietic compartment, WT C57BL/6 recipients were transplanted on day -30 with 5×10⁶ Arg1^{-/-} or WT bone marrow (BM) cells i.v. in the tail vein after 11 Gy TBI (2x 5.5 Gy). Mice were used as ECP recipients or donors in DSS-induced colitis experiments.

### PrimeFlow^{™} RNA Assay

The PrimeFlow^{™} RNA Assay (Invitrogen) was performed according to manufacturer's instructions to detect gene expression on single cell level by FACS. An *Adipoq*-specific AlexaFluor 647 labeled probe was used for hybridization (assay ID: VB1-17726-PF, type1; Invitrogen).

### In vivo migration analysis

To analyze tissue-specific migration and phagocytosis of ECP treated splenocytes, mice were transplanted subcutaneously with 1×10⁶ B16.F10 melanoma cells into the shorn right flank. Mice were treated with 0.2 mg anti-PD1 on day 1, 5, 8 and 11, and received 3 % DSS (colitis grade, 36-50 kDa; MP Biomedicals) in drinking water from day 5-8. Donor splenocytes were isolated on day 12, subjected to ECP treatment as described before and stained with VivoTrack 680 (PerkinElmer) according to manufacturer's instructions. Briefly, VivoTrack 680 was reconstituted with 1.3 mL PBS, cells were diluted to 250×10⁶ cells/mL in PBS. Cells and VivoTrack 680 were mixed in equal parts, briefly vortexed and incubated at RT for 15 min. Stained cells were washed twice with complete RPMI medium and twice with PBS. Cells were resuspended in PBS and 10⁷ ECP treated VivoTrack 680 stained cells were transplanted intravenously. Recipients were euthanized at 2, 6 and 12 h post-transplantation, and tumors, colitis tissue and spleen were digested as described and stained for FACS.

### Tumor digestion

Tumor tissue was digested to prepare single cell suspensions. B16.F10 tumors were digested with 1 mg/mL collagenase IA (Sigma) and 50 µg/mL DNase I (Sigma) in DMEM medium at 37 °C and 1,400 rpm for 1 h. Hematopoietic cells were enriched by density gradient centrifugation with 70 % (3 mL), 40 % (4 mL) and 30 % (3 mL) Percoll (Cytiva), where the latter contained the digested tumor. Enriched cells were collected after centrifugation (680 g, acc 3, no brake, RT) above the bottom phase.

MC38 tumors were digested with 20 µg/mL Liberase TM (Roche) and 50 µg/mL DNase I (Sigma) in DMEM medium at 37 °C and 1,000 rpm for 30 min. Immune cells were enriched by density gradient centrifugation with 30 % Percoll (Cytiva) for 20 min at 800 g (no brake).

### Generation and culture of bone marrow derived dendritic cells (BMDCs)

BMDCs were generated and cultured as described previously (Stickel 2017). Cells were used on day 7 with a purity >90 %, confirmed by CD11c surface expression. LPS (Sigma) stimulation was done with 100 ng/mL for 16 h.

### Generation and culture of bone marrow derived macrophages (BMDMs)

BMDMs were generated from total bone marrow cells after erythrocyte lysis. Cells were cultured in RPMI supplemented with 10 % FCS, 1 % P/S and 20 ng/mL rmM-CSF (Peprotech). Plates were washed with PBS on day 5 and 6 and culture medium was replaced completely. Cells were used on day 7 with a purity >95 %, confirmed by surface CD11b and F4/80 expression. BMDMs (M0) were polarized for 16 h towards M1 with 50 ng/mL rmIFNγ (Peprotech) and 20 ng/mL LPS (Sigma), and towards M2 with 20 ng/mL rmlL-4 (Peprotech).

### Microarray analysis

Colon tissue was isolated from mice with DSS-induced colitis, flushed and washed in ice-cold PBS and homogenized for total RNA using the Qiagen miRNeasy Micro Kit according to the manufacturer's instructions. RNA quality was assessed using an Agilent 2200 TapeStation (Agilent Technologies) and RIN was above 8.8 for all samples. 250 ng of total RNA were processed to fragmented and labeled ds-cDNA using the GeneChip^{™} WT PLUS Kit according to manufacturer's instructions (Applied Biosystems), hybridized on Clariom S mouse microarrays (Applied Biosystems) using the GeneChip^{™} Hybridization, Wash, and Stain Kit (Applied Biosystems) and scanned according to manufacturer's instructions.

The arrays were normalized via robust multichip averaging as implemented in the R/Bioconductor oligo package. Gene set enrichment was calculated using the R/Bioconductor package `gage'48 using the pathways from the ConsensusPathDB 49 as gene sets and a significance cutoff p<0.05. Microarray analysis was performed as previously described (Hamarsheh 2020).

### Isolation of immune cell populations

Various immune cells were isolated for RNA analysis from the bone marrow using Neutrophil Isolation Kit and Monocyte Isolation Kit, or from the spleen using NK cell Isolation Kit, Pan B cell Isolation Kit and Pan T cell Isolation Kit II (all for mouse; Miltenyi).

### RNA isolation, reverse transcription and qPCR

Total RNA from tissues was isolated using QlAzol lysis reagent (QIAGEN) according to manufacturer's instructions. Depending on cell numbers, RNA from cells was isolated using RNeasy Mini or Micro Kit (QIAGEN) according to manufacturer's instructions. 200 ng-1 µg of total RNA was reverse transcribed into complementary DNA (cDNA) using the High-Capacity cDNA Reverse Transcription Kit (ThermoScientific). Following, quantitative PCR (qPCR) was done using the LightCycler 480 SYBR Green I Master mix (Roche) according to manufacturer's instructions on a LightCycler 480 machine (Roche). 5-40 ng of cDNA and 5 µM forward and reverse primer were used per reaction. Primer sequences were synthesized by Eurofins Genomics. *Hprt* was used as a reference gene. Gene expression was calculated relative to *Hprt* or relative to a control group using the 2^{-ΔΔCT} method.

### In vitro phagocytosis analysis

WT C57BL/6 (CD45.2) bone marrow cells were generated into BMDMs (M0) and BMDCs (no LPS) for 7 days and seeded in 6-well plates at 10⁶ cells/well. The next day, splenocytes from CD45.1 mice were isolated and ECP treated as described, followed by staining with 1 µM CellTrace Violet (Thermo Scientific) according to manufacturer's instructions. 2×10⁶

CellTrace Violet stained splenocytes were added into the BMDM and BMDC cultures. Only gender-matched co-cultures were used. Plates were centrifuged at 300 x g for 2 min and cultures for 24 h before further analysis. Phagocytosis was analyzed by FACS using congenic markers and CellTrace Violet signal.

### Recall immunity experiment

For the recall immunity experiment, T cell donor mice challenged with 10⁴ B16.F10 melanoma cells (i.v.) and treated with anti-PD1 (day 1, 3, 6, 9, 12), ECP (day 3, 7, 12), steroid (day 3-12) or Etanercept (day 3, 5, 7, 9, 11, 12). Untreated melanoma mice served as control. Secondary recipient mice were sub-lethally irradiated with 6 Gy and injected i.v. with 10⁴ B16.F10 or 2×10⁶ 4434 melanoma cells (d0). Splenocytes were harvested from T cells donors on day 3 after tumor injection. T cells were isolated using the Pan T Cell Isolation Kit II (mouse, Miltenyi) and 1×10⁵ T cells were transplanted i.v. into secondary recipients on day 3 following tumor injection.

### In vivo treatments

Treatment timepoints are highlighted in the figures and methods. ICB treatment consisted of either 0.2 mg anti-PD1 (mouse; Ichorbio, clone RMP1-14) or 0.2 mg anti-CTLA4 (mouse; Ichorbio, clone 9D9) per injection intraperitoneally. Controls were Rat IgG2a Isotype Control (Ichorbio, clone 1-1) and Mouse IgG2b Isotype Control (Ichorbio, clone MPC-11), respectively. Pembrolizumab (Keytruda, anti-human PD1; MSD) was injected intraperitoneally at 0.2 mg. Steroids (Prednisolone, Solu-Decortin H; Merck) were dissolved in PBS applied at 1 mg/kg bodyweight and Etanercept (Enbrel; Pfizer) was given at 40 µg per treatment intraperitoneally. Adiponectin/Acrp30 (GenScript) was reconstituted in H2O, diluted in PBS and applied intraperitoneally at 4 µg per injection. AdipoRon (MedChemExpress) was prepared in DMSO/corn oil (v/v, 5%/95%) and given via oral gavage at 50 mg/kg bodyweight. The Arginase-1 inhibitor CB-1158 (Numidargistat) dihydrochloride (MedChemExpress) was given orally in H2O twice per day at 100 mg/kg bodyweight.

### Single cell RNA Sequencing (scRNA Seq)

WT C57BL/6 mice were treated with DSS and ICB according to the schedule described before to induce colitis, whereas one group received ECP splenocytes (n=2 per group). On day 8, the colon was harvested and digested as described to isolate lamina propria cells. Dead cells were removed using the Dead Cell Removal Kit (Miltenyi), followed by immune cell enrichment using CD45 MicroBeads (mouse; Miltenyi) according to manufacturer's instructions. Cells were washed and counted in 0.04 % BSA/PBS according to the 10X Genomics cell preparation protocol. 10 000 CD45⁺ cells were encapsulated into droplets with barcoded Gel Beads using the Chromium Controller Single-Cell Instrument (10X Genomics). Libraries were prepared using Chromium Next GEM Single Cell 3' Reagent Kits v3.1 (10X Genomics) according to the manufacturer's instructions. Generated scRNA Seq libraries were pooled and sequenced on a NovaSeq 6000 (Illumina) at 44658 mean reads per cell.

### Western Blot

Cells and tissues were lysed with RIPA lysis buffer (Santa Cruz Biotechnology) supplemented with Phosphatase Inhibitor Cocktail 2 (Sigma). Protein concentrations were determined using the Pierce BCA protein assay (ThermoScientific). 10-15 µg total protein were separated on SDS-PAGE gradient gels (Bis-Tris, 4-12 %; ThermoScientific), then transferred onto Nitrocellulose membranes (Merck). Membranes were blotted for proteins with specific antibodies, and imaged using the INTAS ECL Chemocam Imager after incubation with a chemiluminescent substrate (Advansta). Western blots were quantified using Lablmage 1D software.

### Viability and Caspase 3/7 activity assays

For analysis of cell viability and caspase 3/7 activity following ECP treatment, WT C57BL/6 splenocytes were either treated with 8-MOP or UVA light only, or the combination (ECP), and 10⁵ cells were seeded in 96-well plates. Viability was analyzed using the CellTiter-Glo 2.0 Cell Viability assay (Promega) according to manufacturer's instructions, where viability correlated with luminescence signal. Caspase activity was measured by increased luminescence signal using the Caspase-Glo 3/7 assay system (Promega) according to manufacturer's instructions.

### Long-term steroid and ECP treatment

To better depict the patient situation where steroids and ECP are given for a prolonged time, WT C57BL/6 (CD45.2) mice were injected daily with steroids (1 mg/kg bodyweight) or vehicle for 49 days, or received 8 weekly ECP transplantations (CD45.1). Mice were sacrificed and spleens were isolated on day 50, and processed for FACS analysis.

### In vivo apoptosis analysis

To better understand if ECP treated splenocytes also undergo apoptosis in vivo, splenocytes from C57BL/6^{luc} donor mice were either ECP treated or left untreated and 10⁷ splenocytes were injected intravenously into Rag2^{-/-}γc^{-/-} mice. Expansion of cells was monitored by regular BLI. Recipients were sacrificed on day 30, colon, lung, liver and spleen were imaged by BLI ex vivo and embedded for histopathology scoring.

### Flow cytometry (FACS)

Spleens were isolated, meshed and erythrocytes were lysed. Single cell suspensions of tissues were prepared as described. Viability was analyzed by either Zombie NIR fixable dye (BioLegend) or LIVE/DEAD Fixable Aqua (Invitrogen) staining. Fc receptors were blocked with anti-mouse CD16/CD32 (BioLegend) for 10 min at 4 °C, followed by surface staining with fluorochrome-conjugated antibodies for 30 min at 4 °C. Cells were fixed and permeabilized for 40-60 min at 4 °C using Fixation/Permeabilization Kit (BD Biosciences) or Transcription Factor Staining Buffer Set (eBioscience) for staining of cytoplasmatic proteins and cytokines or intranuclear proteins and transcription factors, respectively. For intracellular cytokine staining, cells were stimulated in the presence of Cell Stimulation Cocktail with protein transport inhibitor (eBioscience) for 4 h before surface and intracellular cytokine staining. Antibody against CD107α was added during stimulation. Intracellular cytokines were stained after fixation and permeabilization for 30 min at 4°C. Antigen-specific CD8 T cells were determined after H-2Kb/OVA²⁵⁷⁻²⁶⁴ dextramer staining according to manufacturer's instructions (Immudex). To determine protein phosphorylation, cells were surface stained, followed by incubation Lyse/Fix (BD Biosciences) for 10 min at 37 °C. Cells were permeabilized with ice-cold Phosflow Perm Buffer III (BD Biosciences) for 30 min at 4 °C. Phosphorylated STAT6 (pY641) was stained for 30 min at 4 °C. If total cell counts were needed,
123count eBeads Counting Beads (Invitrogen) were added directly before acquisition. All data were acquired on a BD LSR Fortessa (BD Biosciences) and analyzed with FlowJo v10 (TreeStar).

### Mass Cytometry (CyTOF)

For multidimensional protein analysis, WT C57BL/6 (CD45.2) mice were treated with DSS and ICB to induce colitis, whereas one group received ECP splenocytes (CD45.1). Organs were harvested on day 8 for analysis. Spleens were meshed and underwent erythrocyte lysis; colon tissue was digested as described. 3×10⁶ cells were used for staining. Dead cells were stained with 2.5 µM MM-DOTA 139La for 5 min at RT, followed by surface protein staining for 30 min at RT. Cells were fixed in 1.6 % PFA in PBS for 5 min at RT and permeabilized using the Transcription Factor Staining Buffer Set (eBioscience) for 30 min at 4 °C. Intracellular proteins were labelled for 60 min at RT, followed by secondary antibodies for 30 min at RT. Cells were stained with 125 nM Cell-ID Intercalator-Ir (Fluidigm) in 4 % PFA in PBS o/n at 4 °C. Data were acquired on a Helios CyTOF (Fluidigm) and processed using FlowJo v10 (TreeStar) and OMIQ (Dotmatics) softwares. Files were cleaned manually by exclusion of doublets and dead cells. Cells were scaled using Arcsinh from -5 to 12000 and pre-gated on CD45.2 (recipient cells), followed by subsampling on 23000 (colon) or 8000 (spleen) cells (per file, max equal). Dimension reduction was run to create opt-SNE plots. Different populations were generated by FlowSOM clustering with Elbow and Consensus metaclustering. Identified clusters were merged and split manually based on surface marker expression and verified based on signature markers. Violin plots show median marker intensity in specified clusters. Heatmaps were generated on medians with Euclidean row distance.

### Measurement of adiponectin in patient serum

Adiponectin concentrations in patient serum samples were measured using the Human Adiponectin ProQuantum Immunoassay Kit (ThermoFisher) according to manufacturer's instructions in 384-well format.

### Measurement of adiponectin in murine serum

For quantification of Adiponectin concentrations in murine serum specimen, blood was harvested by retroorbital bleeding and serum was isolated by centrifugation. Adiponectin concentrations were measured using Adiponectin Mouse ELISA Kit (ThermoFisher) according to manufacturer's instructions after 20,000-fold dilution.

### RESULTS

### ECP reduces immune related colitis via adiponectin expression

To test ECP in a controlled in vivo model for irAEs leading to colitis, mice were treated with 3% DSS and anti-PD1 according to previous reports (Perez-Ruiz 2019). DSS and anti-PD1 treatment reduced the body weight of the mice consistent with the development of colitis (**Figure 1A**)**.** ECP-treatment improved weight loss (**Figure 1A**) and led to increased colon length compared to the group treated with anti-PD1 antibody alone (Figure 1B-C)**.** In agreement with reduced colitis severity, the inventors observed reduced infiltration of neutrophil granulocytes (neutrophils) and lymphocytes in the colon wall of mice treated with ECP compared to the group treated with anti-PD1 only (**Figure 1D-F**). The inventors studied these parameters because in patients with ICB-induced colitis, an increase in intraepithelial lymphocytes, and neutrophils had been described (Beck 2006).

To identify the mechanism by which ECP reduced intestinal inflammation the inventors studied gene expression of cells residing in the intestinal wall. Adiponectin was the most upregulated gene in intestinal cells of colitis mice treated with anti-PD1/ECP compared to anti-PD1 alone **(****Figure 1G****).** Adiponectin RNA upregulation in the intestinal tract upon ECP was confirmed by qPCR (**Figure 1H**). Additionally, adiponectin was increased in the serum of colitis mice treated with ECP compared to anti-PD1 alone (**Figure 11**). Adiponectin protein increased in situ in the intestinal tract of mice treated with ECP compared to control mice (**Figure 1J-K**). To understand if adiponectin was functionally relevant for the ECP-mediated effects, the inventors used *Adipoq*^{*-*/-}mice. The inventors observed that ECP-treatment did not protect *Adipoq*^{*-*/*-*} mice (ECP recipient is KO) from colitis based on colon length, body weight and histological colitis severity (**Figure 1L-****O**).

The treatment schedule is shown in **Figure 2A****.** Anti-PD1-treatment increased colitis severity compared to isotype control (**Figure 2B****, C**) indicating ICB-induced colitis. Colon length was reported to be a surrogate parameter for the severity of anti-PD1 immunotherapy-induced colitis(Perez-Ruiz 2019) and the inventors found colon length and body weight to correlate (**Figure 2D**). The transfer of spenocytes that were not exposed to ECP was not protective (**Figure 2E-H**). The therapeutic effect of ECP against immune related colitis was not restricted to anti-PD1-treatment, but also improved colitis severity induced by anti-CTLA4 (**Figure 2I-L**), which is frequently applied in combination with anti-PD1 treatment in patients with metastatic melanoma. In anti-CTLA4 induced colitis ECP led to less weight loss, higher colon length and reduced the infiltration of inflammatory cells into the colon wall of these mice (**Figure 2I-L**). Adiponectin was increased in the serum of colitis mice treated with anti-CTLA-4 and ECP compared to anti- CTLA-4 alone (**Figure 2M**).

When donor and recipient were *Adipoq*^{*-*/*-*} mice ECP was not protective (**Figure 3A****, B**). Lack of adiponectin in the donor splenocytes that were exposed to ECP was not relevant as here ECP was still protective (**Figure 3C-F**). These findings indicate that ECP reduces ICB-induced colitis in mice by causing production of adiponectin in recipient cells.

### Adiponectin is induced by apoptotic leukocytes that accumulate in inflamed colon tissue

To understand how ECP-treated leukocytes induce adiponectin expression the inventors next analyzed the impact of individual components of ECP, including UV light and 8-Methoxyproralen (8-MOP) on leukocytes in detail. ECP-treated leukocytes underwent rapid apoptosis in vitro compared to untreated (UT) cells (**Figure 4A****, B**). Treatment with UVA light or 8-MOP alone did not affect viability of leukocytes compared to untreated cells, only the combination of both induced cell death (**Figure 4A****, B**). P53, as an indicator of apoptosis induction, was rapidly stabilized after ECP treatment (**Figure 4C****, D**). Pro-apoptotic caspase 3/7 activity was enhanced and increased cleavage of PARP and the caspases-3, -8 and -9 was detected in leukocytes exposed to ECP therapy (**Figure 4E****-Q**). To determine the viability of ECP-treated leukocytes in vivo the inventors used bioluminescence imaging. Only viable luciferase-transgenic cells will convert luciferin and emit light (Schwab 2014). Transfer of ECP-treated leukocytes into Rag2^{-/-}γc^{-/-} mice showed lower overall bioluminescence signal and organ-specific bioluminescence signal compared to control leukocytes, indicating the cell death of transferred ECP-treated leukocytes **(****Figure 5A**, **B**).

To understand how ECP mediates the adiponectin induction, the inventors exposed macrophages, which were shown to regulate intestinal inflammation (Zeiser 2022), to ECP-treated apoptotic leukocytes. The inventors observed that the apoptotic ECP-treated leukocytes were internalized by bone marrow derived macrophages (BMDM) during co-culture (**Figure 6A****, B**). Mechanistically, apoptotic ECP-treated splenocytes induced STAT6 activation in macrophages (**Figure 6C**). STAT6 is known to drive alternatively activated (also called M2) macrophage polarization (Yu 2019), which could explain the protective effect of ECP treated leukocytes on the intestinal immune system. In agreement with this hypothesis ECP-treated splenocytes induced Arg1, CD206 and CD301 (**Figure 6D-F**) which are markers of M2 macrophages in vitro (Orecchioni 2019). Also apoptotic ECP-treated splenocytes induced adiponectin production by BMDM (**Figure 6G**). In vivo transfer of ECP-treated splenocytes induced expression of Peroxisome proliferator-activated receptor γ (PPARγ) transcription in the intestinal tract (**Figure 6H**). PPARγ polarizes macrophages into an anti-inflammatory M2 state (Bouhlel 2007).

To understand if the apoptotic ECP-treated leukocytes reached the intestinal tract the inventors used the congenic marker CD45.1 for the donor cells that were ECP treated and stained these with VT680. The inventors could detect CD45.2 positive cells that were positive for VT680 in the colon of mice with anti-PD1 induced colitis that received CD45.1 ECP-treated VT680+ leukocytes but not in melanoma tissue (**Figure 6I**). This indicated that VT680 labelled CD45.1 positive donor cells were taken up by CD45.2 positive recipient cells in the intestines after transfer. The recipient cells that were VT680⁺ were also positive for arginase-1 indicating their M2 polarization (**Figure 6J**). These findings indicate that ECP-induced apoptotic leukocytes cause adiponectin production and M2 polarization in BMDM after their phagocytic uptake.

Calreticulin acts as an engulfment signal stimulating the recognition of dying cells by phagocytes (Gardai 2005). Consistent with high phagocytic uptake, calreticulin increased on ECP-treated leukocytes compared to all other groups (**Figure 7A****, B**). Additionally, when SIINFEKL loaded leukocytes were treated with ECP and then co-cultured with DCs or BMDM, the peptide SIINFEKL was found on DCs and BMDM, indicating phagocytosis of the ECP-treated cells (**Figure 7C-E**).

To decipher the impact of ECP on multiple immune cell subsets in vivo the inventors used single cell RNA sequencing-based analysis of the intestinal immune cells. The inventors observed that ECP treatment induced a major change of the myeloid immune cell cluster in the colon (**Figure 8A****, B**) of mice that had received DSS/anti-PD1 treatment. The macrophage population increased while neutrophil frequency decreased in mice treated with ECP (**Figure 8A****, B**). This is consistent with findings by others showing a contribution of neutrophils to colitis (Schwab 2014). The analysis of gene expression related to signaling pathways revealed that STAT6 and PPARγ revealed an increased activity score (**Figure 8C-F**). STAT6 activation and PPARγ polarize macrophages into an anti-inflammatory M2 state (Bouhlel 2007).

### Adiponectin monotherapy or combination with ECP reduces intestinal inflammation

To understand the potential therapeutic value of the discovery that ECP treatment leads to adiponectin induction the inventors treated mice that had received DSS and anti-PD1 treatment with adiponectin receptor agonist (ARA) or adiponectin. The inventors observed that ARA- or adiponectin-treatment improved weight loss, led to increased colon length and reduced infiltration of neutrophils and lymphocytes in the colon wall compared to the group treated with anti-PD1 antibody and vehicle (**Figure 9A-H**). Consistent with a key functional role of adiponectin, the inventors observed that ARA-treatment combined with ECP improved weight loss, led to increased colon length and reduced infiltration of neutrophils and lymphocytes in the colon wall compared to the group treated with anti-PD1 antibody and ECP alone (**Figure 9I-L**). Additionally adiponectin-treatment combined with ECP improved weight loss, led to increased colon length and reduced infiltration of neutrophils and lymphocytes in the colon wall compared to the grouptreated with anti-PD1 antibody and ECP alone (**Figure 9N-P**).

To test this strategy in mice with anti-CTLA-4 induced colitis the inventors treated such mice with ARA. The inventors observed that ARA reduced weight loss and led to increased colon length compared to the group treated with anti-CTLA4 only (**Figure 9Q****, R**). ARA treatment reduced infiltration of neutrophils and lymphocytes in the colon wall of mice treated with anti-CTLA4 compared to the group treated with anti-CTLA4/vehicle (**Figure 9S****, T**).

Adiponectin and ARA-treatment reduce colitis severity induced by anti-PD-1 or anti-CTLA-4 ICB-therapy and enhance the activity of ECP.

### Adiponectin and ECP increase Arg1 expression in myeloid cells

To understand which cells contribute most to Adiponectin secretion, the inventors analyzed gene expression in various immune cells derived from naive mice. The inventors found particularly high levels in monocytes and M2 macrophages (**Figure 10A**). The adiponectin receptors AdipoR1 and AdipoR2 were expressed by multiple adaptive and innate immune cells (**Figure 10B****, C**). A particularly high expression of AdipoR1 and AdipoR2 was detected in neutrophils (**Figure 10B****, C**). To analyze the impact of adiponectin in a controlled system the inventors studied its impact on BMDM in vitro. Upon exposure to adiponectin or ARA, BMDM expressed increasing levels of Arg1 (**Figure 10D****, E**), which was previously shown to be immunosuppressive(Highfill 2010). The levels of adiponectin and arginase-1 were highly correlated in M2 BMDM (**Figure 10F**).

To decipher the impact of ECP on multiple immune cell subsets in vivo the inventors used CyTOF-based analysis. The inventors observed that ECP treatment induced a major change of the myeloid immune cell cluster in the colon (**Figure 10G-H**) of mice that had received DSS/anti-PD1 treatment. Arg1 expression in the myeloid immune cell cluster increased significantly in the group treated with anti-PD1/ECP compared to anti-PD1 treatment alone in the colon (**Figure 10I**). Consistently, the inventors observed increased Arg1 RNA in the unselected cells of the intestinal tract of colitis mice treated with anti-PD1/ECP compared to anti-PD1 alone (**Figure 10J**).

Based on the strong upregulation of Arg1 in myeloid cells of ECP treated mice and the induction of Arg1 by adiponectin in vitro, the inventors aimed to understand the functional connection between adiponectin and Arg1. The inventors analyzed Arg1 levels in myeloid cells derived from WT and *Adipoq*^{*-*/*-*} mice and found reduced Arg1 RNA and protein levels in F4/80⁺ macrophages derived from *Adipoq*^{*-*/*-*} mice (**Figure 10K****, L**)**.** CD206 and CD301 are normally expressed on the M2 but not M1 macrophage subtype (Gordon 2003) and therefore serve as markers to identify the M2 phenotype. The inventors observed reduced CD206 and CD301 protein levels in F4/80⁺ macrophages derived from *Adipoq*^{*-*/*-*} mice (**Figure 10M****, N**)**.** These findings indicate a direct link of adiponectin with higher Arg1 expression and M2 polarization in mice with ICI-induced colitis.

### Arg1 inhibition or genetic Arg1 deficiency reduce the protective ECP effects

To decipher the role of Arg1 the inventors correlated Arg1 and adiponectin expression in the colon of mice treated with DSS/anti-PD1 compared to DSS/anti-PD1 and ECP. The inventors observed that adiponectin gene expression correlated with Arg1 gene expression in the colon of DSS/anti-PD1/ECP treated mice, whereas this correlation was not detectable in mice treated with DSS/anti-PD1 alone (**Figure 11A****, B**). In agreement with a functional role of Arg1 for the ECP mediated effects the inventors found that Arg1 inhibition reduced the protective effect of ECP on weight loss (**Figure 11C**), intestinal inflammation as measured via colon length (**Figure 11D**) and neutrophil as well as lymphocyte infiltration in the colon wall (**Figure 11** **E, F**)**.** To understand if Arg1 was required in the cells that underwent the ECP procedure the inventors used Arg1^{-/-} mice as donor for the splenocyte isolation. Using Arg1^{-/-} splenocytes, the protective effect of ECP was still intact with respect to body weight loss (**Figure 11G**), colon length (**Figure 11H**) and neutrophil as well as lymphocyte infiltration (**Figure 11I**, **J**). Conversely, the protective effect of ECP was reduced when Arg1^{-/-} mice were used as recipients with respect to neutrophil as well as lymphocyte infiltration (**Figure 11K-M**). These findings indicate that Arg1 is required in the recipient tissues to allow for the full protective ECP effect while Arg1 is not required in the leukocytes that undergo ECP.

### ECP and adiponectin reduce pathogenic T cell activation

The inventors aimed to understand the impact of adiponectin on T cells in a controlled in vitro setting. CD25, the alpha chain of the IL-2 receptor, is the most prominent T cell activation marker (Rubin 1985, Ross 2018), activating polymorphisms in CD25 were connected to autoimmunity such as diabetes (Lowe 2007) and multiple sclerosis (MS)(Hafler 2007) and high levels of soluble CD25 have been found in MS(Maier 2009). CD4⁺ and CD8⁺ T cells activated by CD3/CD28 beads exhibited less CD25 expression upon exposure to adiponectin compared to the vehicle group (**Figure 12A****, B**). To characterize the impact of ECP on T cells in mice with colitis the inventors investigated T cells isolated from the spleen of colitis mice treated with DSS/anti-PD1 or DSS/anti-PD1/ECP by high-resolution CyTOF-based analysis. The inventors found in CD4⁺ and CD8⁺ T cells lower TOX expression and higher KLRG1, BTLA and CD47 expression in the anti-PD1/ECP compared to the anti-PD1 group (**Figure 12C-F**). The low expression of the terminal exhaustion marker TOX (Alfei 2019) indicates that ECP does not lead to T cell exhaustion. Killer cell lectin like receptor G1 (KLRG1) expression identifies a subset of antigen-experienced T cells in humans that lack proliferative capacity (Voehringer 2002). B and T lymphocyte attenuator (BTLA) is an inhibitory receptor, reducing cytokine production in CD4⁺ T cell (Watanabe 2003). BTLA was recently shown to reduce inflammation in a model of LPS-induced endotoxic shock (Kobayashi 2013). CD47 expressed on T cells primarily functions as an inhibitory molecule against Th1 T cell responses (Bouguermouh 2008). The high levels of KLRG1, BTLA and CD47 on T cells after ECP support an anti-inflammatory effect of ECP on T cells.

### The protective ECP effect against irAE colitis allows for anti-tumor immune responses

To understand if the immunomodulatory effect of ECP was connected to immunosuppression, which may reduce anti-tumor activity, the inventors next treated naive mice with vehicle, prednisolone or ECP for 50 days. Prednisolone (1mg/kg bodyweight) or an equivalent glucocorticoid is the standard first-line therapy recommended for patients with severe ICB-induced colitis according to the ASCO Guideline(Schneider 2021). The inventors observed that prednisolone treatment reduced the absolute numbers of CD4⁺, CD8⁺, CD19⁺, CD11b⁺ and NK1.1⁺ cells in the spleens of melanoma-bearing mice, while the numbers of these cells types were not reduced by ECP (**Figure 13A**). Mice treated with prednisolone also exhibited reduced absolute counts of CD4⁺TNF⁺ cells, CD8⁺TNF⁺ cells, CD4⁺IFNγ⁺ cells and CD8⁺IFNγ⁺ cells, while the numbers of these cytokine-producing T cells were not reduced by ECP (**Figure 13B**). Additionally, the spleen weight and multiple other immune cell types were reduced in the prednisolone group compared to the vehicle- or ECP-groups (**Figure 13C****, D**).

To determine if ECP was connected to a loss of anti-tumor activity the inventors next treated melanoma bearing mice with anti-PD1 alone or in combination with prednisolone or ECP for 10 days (**Figure 14A**). Reduction of colitis severity in anti-PD1/ECP-treated mice was comparable to anti-PD1/prednisolone-treated mice in this model (**Figure 14B****, C**).

The inventors observed that prednisolone treatment reduced the survival of melanoma-bearing mice compared to the group treated with anti-PD1 only (**Figure 14D**). In contrast, the group treated with anti-PD1 and ECP had a comparable outcome as the group treated with anti-PD1 only (**Figure 14D**). The transfer of splenocytes itself without ECP had no impact on survival of melanoma bearing mice treated with anti-PD1 immunotherapy (**Figure 14E**). Treatment with the TNF blocker etanercept that is recommended for second line therapy of ICB-induced colitis by the ASCO Guideline (Schneider 2021) was connected to reduced survival compared to the anti-PD1 alone group (**Figure 14D**). Consistent with intact anti-melanoma immunity seen in the survival study, ECP-treatment of melanoma bearing mice, receiving anti-PD1 immunotherapy, exhibited no reduction of cytotoxic CD4⁺IFN-γ⁺CD107a⁺ and CD8⁺ IFN-γ**⁺**CD107a⁺ T cells (**Figure 14F**). The melanoma derived bioluminescence signal was reduced upon anti-PD1 immunotherapy (**Figure 14G****, H**). The therapeutic effect was intact in mice receiving ECP but reduced in mice that received prednisolone treatment (**Figure 14G****, H**). In agreement the expression of the melanoma specific transcripts premelanosome protein (gp100, mPmel) and dopachrome tautomerase (mDct) in the lungs of melanoma bearing mice decreased upon anti-PD1-treatment (**Figure 14I, J**). Using a second melanoma model (4344, BRAF-V600E positive) the inventors could reproduce that ECP did not interfere with anti-tumor immune responses (**Figure 14K**).

Moreover, analysis of tumor-specific T cells in a B16.F10-OVA s.c. melanoma and a MC38-OVA colorectal cancer model revealed that ECP did not reduce the frequency of tumor-specific T cells (**Figure 14L****, M**). Recall immunity was investigated using T cells isolated from anti-PD1, anti-PD1/steroid or anti-PD1/ECP treated melanoma mice transplanted into sublethally irradiated melanoma-bearing recipients. Survival of mice receiving T cells from anti-PD1/ECP treated B16 melanoma bearing mice was improved compared to mice receiving T cells from anti-PD1/prednisolone treated mice (**Figure 14N**). Survival of mice receiving T cells from anti-PD1/ECP treated B16 melanoma bearing mice was improved compared to mice receiving no T cells (**Figure 14O**). Survival of mice receiving T cells from anti-PD1/ECP treated B16 melanoma bearing mice was not improved when a third party melanoma (4434-BRAF) was used (**Figure 14P**). Survival of mice receiving T cells from anti-PD1/ECP treated B16 melanoma bearing mice was improved compared to mice receiving T cells from anti-PD1/etanercept treated mice (**Figure 14Q**).

To understand why the anti-melanoma effect was intact despite ECP induced adiponectin and Arg1 production in the colon that the inventors had observed, the inventors analyzed the expression of both molecules in the melanoma tissue. The inventors observed that adiponectin and Arg1 were not induced by ECP in the melanoma or colorectal cancer tissue (**Figure 14R-U**).

These findings indicate that ECP does not interfere with the anti-melanoma response induced by anti-PD1 treatment. The immune response is intact because of the tissue specific effect of ECP induced apoptotic leukocytes that exert their effect in the inflamed colon but not in the melanoma microenvironment.

### Clinical activity of ECP in patients with irAE

In parallel to the studies in mice the inventors evaluated the clinical efficacy and safety of ECP in a real-world cohort of 11 patients treated at the University Medical Centers in Freiburg, Essen, Regensburg and Hamburg, Germany (NCT0541455). All patients had received 1-3 previous treatments for irAE and were corticosteroid-refractory, defined as an absence of improvement of symptoms after 1 week of 1 mg/kg BW steroid equivalent or rebound of symptoms upon immunosuppression-tapering. The majority of patients had received a combination of corticosteroids and infliximab (**Figure 15A**). At initiation of ECP, 9/11 patients presented with colitis (mainly grade 3), two patients presented with hepatitis (1 grade 2, 1 grade 4), one patient had arthralgia and one patient had skin and mucosal involvement (**Figure 15B**). The patients received a median of 16 ECP procedures until achievement of best response. A representative colonoscopy image of a colitis patient responding to ECP is shown in **Figure 15C****.** Nine of the eleven patients (81.8%) reached a complete response, defined as absence of any symptoms of irAE (**Figure 15D**). One patient achieved a partial response (decrease of at least 1 grade in at least 1 organ manifestation) and one patient had a stable disease (**Figure 15D**). Systemic immunosuppression was reduced after ECP treatment in all patients (**Figure 15E**). Patients were also monitored for adverse events that could be related to the ECP treatment. Two patients developed blurry vision that was attributed to cataract. This condition was pre-existing in one of the patients but aggravated during ECP treatment. The other patients reported no adverse events that could be attributed to the ECP procedure. One patient experienced a bowel perforation after two weeks of ECP treatment, which is a known complication of immune-related colitis and causal relation to ECP treatment is unlikely. In summary, ECP appeared to be safe in this clinical context with no unexpected toxicities being reported.

Serum concentrations of adiponectin increased in patients two weeks after ECP therapy compared to before therapy (**Figure 15F**). Colon biopsies from irAE patients with ICB-induced colitis were analyzed for Adiponectin expression in the lamina propria by IHC before and after ECP. Adiponectin positive cells increased in all patients after ECP treatment (**Figure 15G****, H**).

In a humanized irAE model, Rag2^{-/-}γc^{-/-} mice received healthy donor PBMCs and anti-PD1 alone or in combination with ECP, steroid or etanercept (**Figure 15I**). Histopathological analysis showed reduced colitis severity in mice receiving anti-PD1 plus ECP, prednisolone or etanercept treatment compared to anti-PD1 alone (**Figure 15** **J, K**). Additionally in this model of systemic irAEs the severity of inflammation in liver, lung and skin was reduced by ECP, prednisolone or etanercept (**Figure 15L-Q**). The irAE grade of untreated mice or mice receiving only PBMCs was low to absent (**Figure 15R-Y**).

These findings indicate that ECP has activity in patients with ICB-induced colitis that have failed prednisolone and other immunosuppressive agents and that it ECP reduces ICB-induced colitis severity in a humanized mouse model.

### DISCUSSION

Patients developing severe grade 3 or 4 irAEs after ICB are currently treated with a durable interruption of the immunotherapy and the administration of glucocorticosteroids (Haanen 2017, Schneider 2021). Both interventions may have negative effects on the anti-tumor immune response as suggested by retrospective analyses (Horvat 2015, Marschner 2020, Luo 2021). Additionally, it is desirable to avoid glucocorticosteroids because of the systemic side-effects including hyperglycemia, fluid retention, psychological disorders, and infectious complication (Postow 2018). Currently no prospective randomized trial supports the use of glucocorticosteroids or second line therapies such as mycophenolate mofetile (MMF), TNF antagonists or cyclosporine A. Novel approaches that are active against irAE without strong systemic immunosuppression are lacking. The inventors have reported activity of ECP in patients with severe chronic graft-versus-host-disease (cGVHD) (Maas-Bauer 2021), refractory to ruxolitinib which has proven efficacy against cGVHD (Zeiser 2021). Additionally the inventors have reported a patient with irAE colitis who was successfully treated with ECP (Apostolova 2020).

To decipher the mechanism of action of ECP against irAE-colitis and to understand its impact on the anti-tumor response after anti-PD1 based immunotherapy the inventors used an established model of melanoma that is sensitive to ICB (Mastroianni 2019). The inventors discovered that ECP caused adiponectin expression in the intestinal tract, using an unbiased microarray approach. Adiponectin had never been connected to the mode of action of ECP before.

Adiponectin is the most abundant adipokine in the blood stream in humans. A meta-analysis of 12 studies including 784 patients with rheumatoid arthritis (RA) and 655 healthy controls showed that circulating adiponectin levels are significantly higher in RA patients than in healthy controls (HCs) (Lee 2017). Conversely, adiponectin blood levels were lower in patients with systemic sclerosis compared to HC and correlated negatively with disease activity (Lee 2017, Stochmal 2020). Therefore so far there was no clear pattern indicating that adiponectin is pro- or anti-inflammatory. Adiponectin expression is negatively regulated by multiple inflammatory mediators such as IL-6 and consistently anti-IL-6R inhibition with tocilizumab was associated with increased adiponectin levels in the blood of RA patients (Fioravanti 2019, Choi 2020).

While these correlative studies are suggestive, they cannot clarify the stimulus for adiponectin production or the mechanism of action of adiponectin in colitis. The inventors could show that adiponectin production was induced by apoptotic leukocytes in the inflamed colon after ECP exposure. The inventors could decipher the mechanism of action showing that adiponectin functions via Arg1 production as Arg1^{-/-} mice were less protected from colitis by ECP. Arg1 was shown to deplete arginine, which is essential for activated T cells (Highfill 2010). In addition to the indirect inhibition of T cells via the induction of Arg1 production the inventors observed that adiponectin exerted a direct inhibitory effect on T cell activation, leading to a reduction of CD25⁺ T cells, that were pathogenic in the anti-PD1-induced colitis model. Additionally, ECP and adiponectin led to increased frequencies of tolerogenic KLRG1⁺BTLA⁺CD47⁺TOX^{low} T cells in the inflamed intestinal tract. The increase of KLRG1⁺BTLA⁺CD47⁺TOX^{low} T cells is consistent with the expression of KLRG1 a marker for antigen-experienced T cells that lack proliferative capacity (Voehringer 2002), the inhibitory receptor BTLA, reducing cytokine production in CD4⁺ T cell (Watanabe 2003) and CD47 an inhibitory molecule reducing Th1 T cell responses (Bouguermouh 2008), indicating an anti-inflammatory effect of ECP on T cells.

Anti-tumor immunity in melanoma-bearing mice treated with anti-PD1 antibodies remained intact during ECP-treatment, while corticosteroids reduced anti-melanoma immune effects. Consistent with reduced anti-melanoma activity, prednisolone treatment reduced CD4⁺ and CD8⁺ T cell as well as NK cell numbers. The maintenance of anti-tumor immune responses was connected to the accumulation of anti-inflammatory apoptotic cells in the inflamed colon but not in the melanoma tissue. Consistently the expression of adiponectin and Arg1 increased in the inflamed colon but not in the melanoma tissue upon ECP treatment. ECP did not interfere with melanoma-specific recall immunity induced by adoptively transferred T cells isolated from anti-PD1/ECP treated mice.

In agreement with our findings in the mouse models, the inventors observed that ECP treatment of irAE patients within a multicenter study led to clinical responses in 11/11 patients (9CR/1PR/1SD) and induced adiponectin in blood and intestinal tissues of irAE patients without inducing major toxicities. All patients treated with ECP were able to reduce the immunosuppression. The favorable safety profile of ECP observed in our study patients with irAE is in agreement with the reported safety profile of patients treated with ECP for acute or chronic GVHD (Zeiser 2017, Zeiser 2017, Maas-Bauer 2021). Consistent with the role of adiponectin identified in mice, the inventors observed that serum concentrations of adiponectin increased in patients two weeks after ECP therapy compared to the time before ECP. Additionally, adiponectin positive cells located in the colon increased in all patients after ECP treatment compared to the time of analysis before ECP start.

In summary, the inventors identified ECP as a novel immunomodulatory approach against ICB-induced colitis that induces adiponectin, Arg1 and tolerogenic KLRG1⁺BTLA⁺CD47⁺TOX^{low} T cells in the inflamed intestines but not in melanoma tissue, thereby leaving anti-tumor immunity intact. ECP induced complete remissions in over 80% of patients with irAEs and led to adiponectin induction in colon tissue.

The inventors discovered that ECP caused adiponectin expression in the intestinal tract by using an unbiased microarray approach. Adiponectin had never been connected to the mode of action of ECP before. Previous work had connected adiponectin to anti-inflammatory activity in autoimmune diseases. Furthermore, the inventors found a functional connection between adiponectin and Arg-1 production by myeloid cells. Arg-1 was shown to deplete arginine, which is essential for activated T cells.

In addition to the indirect inhibition of T cells via the induction of Arg-1 production the inventors observed that adiponectin that was in direct contact with T cells, reduced their activation.

Using genetic loss-of-function approaches the inventors could show a functional role of the adiponectin/Arg-1 axis for the protective effects of ECP.

The present results show successful testing of the therapeutic activity of adiponectin and ECP in irAE.

### References

Alfei, F., Kanev K, Hofmann M, Wu M, Ghoneim HE, Roelli P, Utzschneider DT, von Hoesslin M, Cullen JG, Fan Y, Eisenberg V, Wohlleber D, Steiger K, Merkler D, Delorenzi M, Knolle PA, Cohen CJ, Thimme R, Youngblood B, Zehn D. (2019). "TOX reinforces the phenotype and longevity of exhausted T cells in chronic viral infection." Nature 571: 265-269.

Apostolova, P., Unger, S., Meiss, F., von Bubnoff, D., Aumann, K., Becher, B., Zeiser, R. (2020). "Extracorporeal photopheresis for severe immune checkpoint inhibitor-induced autoimmune colitis." N Eng J Med 382: 294-296.

Beck, K. E., Blansfield JA, Tran KQ, Feldman AL, Hughes MS, Royal RE, Kammula US, Topalian SL, Sherry RM, Kleiner D, Quezado M, Lowy I, Yellin M, Rosenberg SA, Yang JC (2006). "Enterocolitis in patients with cancer after antibody blockade of cytotoxic T-lymphocyte-associated antigen 4." J Clin Oncol 24: 2283-2289.

Beck, K. E., Blansfield, J.A., Tran, K.Q., Feldman, A.L., Hughes, M.S., Royal, R.E., et al. (2006). "Enterocolitis in patients with cancer after antibody blockade of cytotoxic T-lymphocyte-associated antigen 4." J Clin Oncol. 24: 2283-2289.

Bouguermouh, S., Van VQ, Martel J, Gautier P, Rubio M, Sarfati M. (2008). "CD47 expression on T cell is a self-control negative regulator of type 1 immune response." J Immunol. 180: 8073-8082.

Bouhlel, M. A., Derudas B, Rigamonti E, Dièvart R, Brozek J, Haulon S, Zawadzki C, Jude B, Torpier G, Marx N, Staels B, Chinetti-Gbaguidi G. (2007). "PPARgamma activation primes human monocytes into alternative M2 macrophages with anti-inflammatory properties." Cell Metab. 2: 137-143.

Choi, I. A., Sagawa A., Lee E.Y., Lee E.B., Song Y.W. (2020). "Tocilizumab Increases body weight and serum adipokine levels in patients with rheumatoid arthritis independently of their treatment response: A retrospective cohort study." J. Korean Med. Sci. 35: e155.

Dhomen, N., Reis-Filho, J.S., da Rocha Dias, S., Hayward, R., Savage, K., Delmas, V., Larue, L., Pritchard, C., Marais, R. (2009). "Oncogenic Braf induces melanocyte senescence and melanoma in mice." Cancer Cell 15: 294-303.

Fioravanti, A., Tenti S., Bacarelli M.R., Damiani A., Li Gobbi F., Bandinelli F., Cheleschi S., Galeazzi M., Benucci M (2019). "Tocilizumab modulates serum levels of adiponectin and chemerin in patients with rheumatoid arthritis: Potential cardiovascular protective role of IL-6 inhibition." Clin. Exp. Rheumatol. 37: 293-300.

Gardai, S. J., McPhillips, K.A., Frasch, S.C., Janssen, W.J. (2005). "Starefeldt A, Murphy-Ullrich JE, et al. Cell-surface calreticulin initiates clearance of viable or apoptotic cells through transactivation of LRP on the phagocyte." Cell 123: 321-334.

Gordon, S. (2003). "Alternative activation of macrophages." Nat Rev Immunol. 1: 23-35.

Haanen, J. B. A. G., Carbonnel F, Robert C, Kerr KM, Peters S, Larkin J, Jordan K; ESMO Guidelines Committee. (2017). "Management of toxicities from immunotherapy: ESMO Clinical Practice Guidelines for diagnosis, treatment and follow-up." Ann Oncol. 28: iv119-iv142.

Hafler, D. A., Compston A, Sawcer S, Lander ES, Daly MJ, et al. (2007). "Risk alleles for multiple sclerosis identified by a genome wide study." N Eng J Med 357: 851-862.

Hamarsheh, S., Osswald, L., Saller, B.S., Unger S, De Feo D, Vinnakota JM, Konantz M, Uhl FM, Becker H, Lübbert M, Shoumariyeh K, Schürch C, Andrieux G, Venhoff N, Schmitt-Graeff A, Duquesne S, Pfeifer D, Cooper MA, Lengerke C, Boerries M, Duyster J, Niemeyer CM, Erlacher M, Blazar BR, Becher B, Groß O, Brummer T, Zeiser R. (2020). "Oncogenic KrasG12D causes myeloproliferation via NLRP3 inflammasome activation." Nat Commun. 11: 1659.

Highfill, S. L., Rodriguez, P.C., Zhou, Q., Goetz, C.A., Koehn, B.H., Veenstra, R., Taylor PA, Panoskaltsis-Mortari A, Serody JS, Munn DH, Tolar J, Ochoa AC, Blazar BR. (2010). "Bone marrow myeloid-derived suppressor cells (MDSCs) inhibit graft-versus-host disease (GVHD) via an arginase-1-dependent mechanism that is up-regulated by interleukin-13." Blood 116: 5738-5747.

Horvat, T. Z., Adel, N.G., Dang TO, Momtaz P, Postow MA, Callahan MK, Carvajal RD, Dickson MA, D'Angelo SP, Woo KM, Panageas KS, Wolchok JD, Chapman PB. (2015). "Immune-related adverse events, need for systematic immunosuppression, and effects on survival and time to treatment failure in patients with melanoma treated with ipilimumab at Memorial Sloan Kettering Cancer Center." J Clin Oncol. 33: 3193-3198.

Johncilla, M., Misdraji, J., Pratt DS, Agoston AT, Lauwers GY, Srivastava A, Doyle LA. (2015). "Ipilimumab-associated Hepatitis: Clinicopathologic Characterization in a Series of 11 Cases." Am J Surg Pathol. 39: 1075-1084.

Kobayashi, Y., Iwata A, Suzuki K, Suto A, Kawashima S, Saito Y, Owada T, Kobayashi M, Watanabe N, Nakajima H. (2013). "B and T lymphocyte attenuator inhibits LPS-induced endotoxic shock by suppressing Toll-like receptor 4 signaling in innate immune cells." PNAS 110: 5121-5126.

Lee, Y. H., Bae, S.C. (2017). "Circulating adiponectin and visfatin levels in rheumatoid arthritis and their correlation with disease activity: A meta-analysis." Int. J. Rheum. Dis. 21: 664-672.

Lee, Y. H., Song, G.G. (2017). "Meta-analysis of circulating adiponectin, leptin, and resistin levels in systemic sclerosis." Z. Rheumatol. 76: 789-797.

Lowe, C. E., Cooper JD, Brusko T, Walker NM, Smyth DJ, et al. (2007). "Large-scale genetic fine mapping and genotype-phenotype associations implicate polymorphism in the IL2RA region in type 1 diabetes." Nat Genet. 39: 1074-1082.

Luo, J., Beattie JA, Fuentes P, Rizvi H, Egger JV, Kern JA, Leung DYM, Lacouture ME, Kris MG, Gambarin M, Santomasso BD, Faleck DM, Hellmann MD. (2021). "Beyond steroids: immunosuppressants in steroid-refractory/resistant immune related adverse events."J Thorac Oncol.: S1556-0864(1521)02291-02297. doi: 02210.01016/j.jtho.02021.02206.02024. Epub ahead of print. PMID: 34265432.

Maas-Bauer, K., Kiote-Schmidt C, Bertz H, Apostolova P, Wäsch R, Ihorst G, Finke J, Zeiser R. (2021). "Ruxolitinib-ECP combination treatment for refractory severe chronic graft-versus-host disease." Bone Marrow Transplant. 56: 909-916.

Maier, L. M., Anderson DE, Severson CA, Baecher-Allan C, Healy B, et al. (2009). "Soluble IL-2RA levels in multiple sclerosis subjects and the effect of soluble IL-2RA on immune responses." J Immunol. 182: 1541-1547.

Marschner, D., Falk M, Javorniczky NR, Hanke-Müller K, Rawluk J, Schmitt-Graeff A, Simonetta F, Haring E, Dicks S, Ku M, Duquesne S, Aumann K, Rafei-Shamsabadi D, Meiss F, Marschner P, Boerries M, Negrin RS, Duyster J, Zeiser R, Köhler N. (2020). "MicroRNA-146a regulates immune-related adverse events caused by immune checkpoint inhibitors." JCI Insight 5: 132334.

Mastroianni, J., Stickel, N., Andrlova, H., Hanke K, Melchinger W, Duquesne S, Schmidt D, Falk M, Andrieux G, Pfeifer D, Dierbach H, Schmitt-Graeff A, Meiss F, Boerries M, Zeiser R. (2019). "miR-146a Controls Immune Response in the Melanoma Microenvironment." Cancer Res 79: 183-195.

Orecchioni, M., Ghosheh, Y., Pramod, A.B., Ley K. (2019). "Macrophage Polarization: Different Gene Signatures in M1(LPS+) vs. Classically and M2(LPS-) vs. Alternatively Activated Macrophages." Front Immunol. 10: 1084.

Perez-Ruiz, E., Minute L, Otano I, Alvarez M, Ochoa MC, Belsue V, de Andrea C, Rodriguez-Ruiz ME, Perez-Gracia JL, Marquez-Rodas I, Llacer C, Alvarez M, de Luque V, Molina C, Teijeira A, Berraondo P, Melero I (2019). "Prophylactic TNF blockade uncouples efficacy and toxicity in dual CTLA-4 and PD-1 immunotherapy." Nature 569: 428-432.

Postow, M. A., Sidlow R, Hellmann MD. (2018). "Immune-Related Adverse Events Associated with Immune Checkpoint Blockade." N Eng J Med 378: 158-168.

Ross, S. H., Cantrell DA. (2018). "Signaling and Function of Interleukin-2 in T Lymphocytes." Annu Rev Immunol. 36: 411-433.

Rubin, L. A., Kurman CC, Fritz ME, Biddison WE, Boutin B, et al. (1985). "Soluble interleukin 2 receptors are released from activated human lymphoid cells in vitro." J Immunol. 135: 3172-3177.

Schneider, B. J., Naidoo J, Santomasso BD, Lacchetti C, Adkins S, Anadkat M, Atkins MB, Brassil KJ, Caterino JM, Chau I, Davies MJ, Ernstoff MS, Fecher L, Ghosh M, Jaiyesimi I, Mammen JS, Naing A, Nastoupil LJ, Phillips T, Porter LD, Reichner CA, Seigel C, Song JM, Spira A, Suarez-Almazor M, Swami U, Thompson JA, Vikas P, Wang Y, Weber JS, Funchain P, Bollin K. (2021). "Management of Immune-Related Adverse Events in Patients Treated With Immune Checkpoint Inhibitor Therapy: ASCO Guideline Update." J Clin Oncol. 39: 4073-4126.

Schwab, L., Goroncy, L., Palaniyandi, S., Gautam, S., Triantafyllopoulou, A., Mocsai, A, Reichardt, W., Karlsson, F.J. Radhakrishnan, S.V., Hanke, K., Schmitt-Graeff, A., Freudenberg, M., von Loewenich, F.D., Wolf, P., Leonhardt, F., Baxan, N., Pfeifer, D., Schmah, O., Schönle, A., Martin, S.F., Mertelsmann, R., Duyster, J, Finke, J., Prinz, M., Henneke, P., Häcker, H., Hildebrandt, G.C., Häcker, G., Zeiser, R. (2014). "Neutrophil granulocytes recruited upon translocation of intestinal bacteria enhance GvHD via tissue damage." Nat Med 20: 648-654.

Stickel, N., Hanke, K., Marschner, D., Prinz, G., Köhler, M., Melchinger, W., Pfeifer, D., Schmitt-Graeff, A., Brummer, T., Heine, A., Brossart, P., Wolf, D., von Bubnoff, N., Finke, J., Duyster, J., Ferrara, J., Salzer, U., Zeiser, R. (2017). "MicroRNA-146a reduces MHC-II expression via targeting JAK/STAT-signaling in dendritic cells after stem cell transplantation." Leukemia 31: 2732-2741.

Stochmal, A., Czuwara, J., Zaremba, M., Rudnicka, L. (2020). "Altered serum level of metabolic and endothelial factors in patients with systemic sclerosis." Arch. Dermatol. Res. 312: 453-458.

Voehringer, D., Koschella, M., Pircher, H. (2002). "Lack of proliferative capacity of human effector and memory T cells expressing killer cell lectinlike receptor G1 (KLRG1)." Blood 100: 3698-3702.

Watanabe, N., Gavrieli, M., Sedy, J.R., Yang, J., Fallarino F, Loftin SK, Hurchla MA, Zimmerman N, Sim J, Zang X, Murphy TL, Russell JH, Allison JP, Murphy KM. (2003). "BTLA is a lymphocyte inhibitory receptor with similarities to CTLA-4 and PD-1." Nat Immunol. 4: 670-679.

Yu, T., Gan S, Zhu Q, Dai D, Li N, Wang H, Chen X, Hou D, Wang Y, Pan Q, Xu J, Zhang X, Liu J, Pei S, Peng C, Wu P, Romano S, Mao C, Huang M, Zhu X, Shen K, Qin J, Xiao Y. (2019). "Modulation of M2 macrophage polarization by the crosstalk between Stat6 and Trim24." Nat Commun. 10: 4353.

Zeiser, R., Blazar, B.R. (2017). "Acute Graft-versus-host disease - Biologic process, prevention, and therapy." N Eng J Med 377: 2167-2179.

Zeiser, R., Blazar, B.R. (2017). "Pathophysiology of Chronic Graft-versus-Host Disease and Therapeutic Targets." N Eng J Med 377: 2565-2579.

Zeiser, R., Polverelli, N., Ram R, Hashmi SK, Chakraverty R, Middeke JM, Musso M, Giebel S, Uzay A, Langmuir P, Hollaender N, Gowda M, Stefanelli T, Lee SJ, Teshima T, Locatelli F, for the REACH3 Investigators. (2021). "Ruxolitinib for Glucocorticoid-Refractory Chronic Graft-versus-Host Disease." N Eng J Med 385: 228-238.

Zeiser, R., Warnatz, K., Rosshart S, Sagar, TanriverY. (2022). "GVHD, IBD, and primary immunodeficiencies: The gut as a target of immunopathology resulting from impaired immunity." Eur J Immunol.: doi: 10.1002/eji.202149530. Epub ahead of print.

## Claims

1. Adiponectin and/or Adiponectin Receptor Agonist (ARA) for use in the treatment or prevention of immune checkpoint blockade (ICB)-induced immune related adverse events (irAEs) in a subject.

2. Adiponectin and/or ARA for use according to claim 1, wherein the subject is receiving checkpoint blockade therapy.

3. Adiponectin and/or ARA for use according to any one of the preceding claims, wherein Adiponectin and/or ARA is administered at least 1 hour before, during and/or at least one hour after administration of checkpoint blockade therapy.

4. Adiponectin and/or ARA for use according to any one of the preceding claims, wherein the checkpoint blockade therapy comprises administration of at least one antibody, preferably at least two antibodies.

5. Adiponectin and/or ARA for use according to any one of the preceding claims, wherein the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

6. Adiponectin and/or ARA for use according to any one of the preceding claims, wherein irAEs can involve any organ, preferably the gastrointestinal tract, lungs, endocrine glands, skin, and liver.

7. Adiponectin and/or ARA for use according to any one of the preceding claims, wherein the irAE comprise an ICB-induced colitis.

8. Adiponectin and/or ARA for use according to any one of the preceding claims, wherein the administration of Adiponectin and/or ARA reduces ICB-induced colitis.

9. Adiponectin and/or ARA for use according to any one of the preceding claims, wherein the administration of Adiponectin and/or ARA does not interfere with the anti-tumor response induced by the checkpoint blockade therapy, which is preferably an anti-PD1 treatment, due to the tissue specific adiponectin induction.

10. Combination medication for use in the treatment of immune checkpoint blockade (ICB)-induced immune related adverse events (irAEs) in a subject, comprising at least two of the following components:
a. a photosensitizing agent, and
b. Adiponectin and/or Adiponectin Receptor Agonist (ARA),
and wherein a blood sample of said subject is subjected to ultraviolet A (UVA) irradiation extracorporeally, preferably to extracorporeal photopheresis (ECP) therapy.

11. The combination medication for use according to claim 10, wherein the photosensitizing agent is a psoralen reagent, preferably 8-methoxypsoralen.

12. The combination medication for use according to claims 10 to 11, wherein the wavelength of the UVA irradiation is from wavelength of 3200 to 4000 angstroms.

13. The combination medication for use according to claims 10 to 12, wherein the subject suffers from cancer.

14. The combination medication for use according to claims 10 to 13, wherein the checkpoint blockade therapy comprises administration of at least one antibody, preferably at least two antibodies.

15. The combination medication for use according to claims 10 to 14, wherein the at least one antibody is selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

16. The combination medication for use according to claims 10 to 15, wherein irAEs can involve any organ, preferably the gastrointestinal tract, lungs, endocrine glands, skin, and liver.

17. The combination medication for use according to claims 10 to 16, wherein the irAE comprises an ICB induced colitis.

18. The combination medication for use according to claims 10 to 17, wherein the combination medication induces an increase of adiponectin, arginase-1 (Arg1) and tolerogenic KLRG1⁺BTLA⁺CD47⁺TOX^{low} T cells in inflamed intestines, but not in cancer tissue in said subject.

19. Adiponectin and/or Adiponectin Receptor Agonist (ARA) for use in the treatment of a tumor patient (or: subject suffering from cancer), wherein said patient has developed immune checkpoint blockade (ICB)-induced immune related adverse events (irAEs), wherein said treatment comprises the combined administration of Adiponectin and/or ARA with an anti-tumor immunotherapy, wherein said immunotherapy comprises the administration of immunoregulatory T cells.

20. Adiponectin and/or ARA for use in the treatment of a tumor patient according to the preceding claim, wherein the immunoregulatory T cells.
a. are obtained from a method comprising the steps of
i. providing a sample derived from an isolated blood sample of said patient,
ii. adding a photosensitizing agent to the sample, and subjecting the sample to irradiation.

21. Adiponectin and/or ARA for use in the treatment of a tumor patient according to claim 19 or 20, wherein the photosensitizing agent is 8-methoxypsoralen and/or the irradiation is UVA irradiation.
